(19) Europäisches Patentamt
European Patent Office
Office européen des brevets

(11) EP 0 773 920 B1

(12) **EUROPÄISCHE PATENTSCHRIFT**

(45) Veröffentlichungstag und Bekanntmachung des Hinweises auf die Patenterteilung:
**26.01.2000 Patentblatt 2000/04**

(21) Anmeldenummer: **95924988.9**

(22) Anmeldetag: **11.07.1995**

(51) Int Cl.[7]: **C07C 69/013**, C07C 69/96, A01N 35/06, A01N 37/02, C07C 49/747, C07C 309/65, C07C 271/32, C07C 59/84, C07C 329/06

(86) Internationale Anmeldenummer:
**PCT/EP95/02684**

(87) Internationale Veröffentlichungsnummer:
**WO 96/03366 (08.02.1996 Gazette 1996/07)**

(54) **2-(2,4,6-TRIMETHYLPHENYL)-CYCLOPENTAN-1,3-DION-DERIVATE**

2-(2,4,6-TRIMETHYL PHENYL)CYCLOPENTANE-1,3-DIONE DERIVATIVES

DERIVES DE 2-(2,4,6-TRIMETHYLPHENYL)CYCLOPENTANE-1,3-DIONE

(84) Benannte Vertragsstaaten:
**BE CH DE ES FR GB IT LI NL**

(30) Priorität: **21.07.1994 DE 4425953**
**31.01.1995 DE 19502945**
**13.06.1995 DE 19521430**

(43) Veröffentlichungstag der Anmeldung:
**21.05.1997 Patentblatt 1997/21**

(73) Patentinhaber: **BAYER AG**
**51368 Leverkusen (DE)**

(72) Erfinder:
- **FISCHER, Reiner**
  **D-40789 Monheim (DE)**
- **DUMAS, Jacques**
  **D-51061 Köln (DE)**
- **BRETSCHNEIDER, Thomas**
  **D-53797 Lohmar (DE)**
- **ERDELEN, Christoph**
  **D-42799 Leichlingen (DE)**
- **WACHENDORFF-NEUMANN, Ulrike**
  **D-53177 Bonn (DE)**
- **SANTEL, Hans-Joachim**
  **D-51371 Leverkusen (DE)**
- **DOLLINGER, Markus**
  **D-51381 Leverkusen (DE)**
- **TURBERG, Andreas**
  **D-40699 Erkrath (DE)**
- **MENCKE, Norbert**
  **D-51381 Leverkusen (DE)**

(56) Entgegenhaltungen:
**US-A- 4 283 348**
**US-A- 4 436 666**
**US-A- 4 551 547**

Bemerkungen:
Die Akte enthält technische Angaben, die nach dem Eingang der Anmeldung eingereicht wurden und die nicht in dieser Patentschrift enthalten sind.

EP 0 773 920 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft neue 2-Aryl-3-hydroxy-$\Delta^2$-cyclopenten-1-on-Derivate, Verfahren zu ihrer Herstellung und ihre Verwendung als Herbizide und Schädlingsbekämpfungsmittel.

Es ist bekannt, daß bestimmte substituierte 2-Arylcyclopentandione, wie z.B. 2-(2',4'-Dimethylphenyl)-4,5,6,7,8,9-hexahydro-1,3-indandion, herbizide und akarizide Eigenschaften besitzen (vgl. z.B. US 4 283 348; 4 338 122; 4 436 666; 4 526 723; 4 551 547 und 4 626 698). Außerdem ist 2-(2,4,6-Trimethylphenyl)-1,3-indandion aus der Publikation J. Economic Entomology, 66, (1973), 584 und der Offenlegungsschrift DE 2 361 084 bekannt, mit Angabe von herbiziden und akariziden Wirkungen.

Die Wirksamkeit dieser bekannten Verbindungen ist jedoch, insbesondere bei niedrigen Aufwandmengen und -konzentrationen, nicht in allen Anwendungsgebieten völlig zufriedenstellend.

Es wurden nun neue substituierte bicyclische 2-Mesityl-cyclopentan-1,3-dion-Derivate der Formel (I)

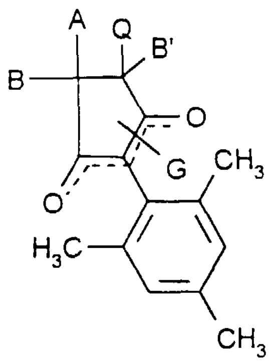

(I)

gefunden,
in welcher

A und Q gemeinsam für jeweils gegebenenfalls durch Halogen, Hydroxy, Mercapto, jeweils gegebenenfalls substituiertes Alkyl, Alkoxy, Alkylthio, Cycloalkyl, Benzyloxy oder Aryl substituiertes Alkandiyl oder Alkendiyl stehen, welches außerdem gegebenenfalls eine der nachstehenden Gruppen

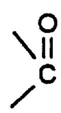 ; 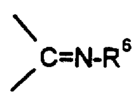 ; 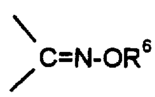 ; 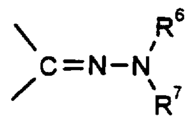 ;

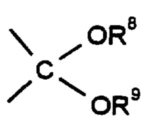 ; 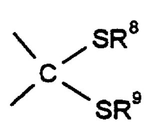 ; 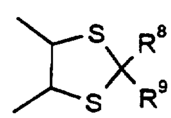 ;

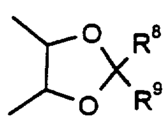 ; 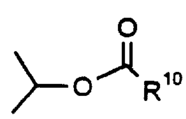 oder 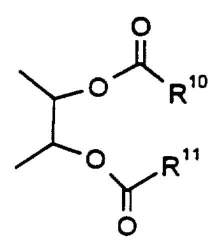

enthält oder durch eine Alkandiylgruppe überbrückt ist,

B und B' unabhängig voneinander für Wasserstoff, Halogen oder Alkyl oder gemeinsam für jeweils gegebenenfalls substituiertes Alkandiyl oder Alkendiyl stehen,

G für Wasserstoff (a) oder für eine der Gruppen

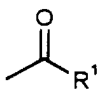 (b), 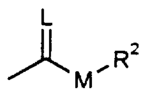 (c), 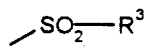 (d), 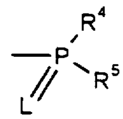 (e),

E (f)

oder

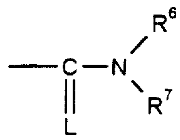 (g),

steht,
in welchen

| | |
|---|---|
| E | für ein Metallionäquivalent oder ein Ammoniumion steht, |
| L | für Sauerstoff oder Schwefel steht und |
| M | für Sauerstoff oder Schwefel steht, |
| $R^1$ | für jeweils gegebenenfalls substituiertes Alkyl, Alkenyl, Alkoxyalkyl, Alkylthioalkyl, Polyalkoxyalkyl oder Cycloalkyl, das mindestens ein Heteroatom enthalten kann, jeweils gegebenenfalls substituiertes Phenyl, Phenylalkyl, Hetaryl, Phenoxyalkyl oder Hetaryloxyalkyl steht, |
| $R^2$ | für jeweils gegebenenfalls substituiertes Alkyl, Cycloalkyl, Alkenyl, Alkoxyalkyl, Polyalkoxyalkyl oder jeweils gegebenenfalls substituiertes Phenyl oder Benzyl steht, |
| $R^3$ | für jeweils gegebenenfalls substituiertes Alkyl, Phenyl oder Phenylalkyl steht, |
| $R^4$ und $R^5$ | unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Phenoxy oder Phenylthio stehen, |
| $R^6$ | für Wasserstoff, jeweils gegebenenfalls durch Halogen substituiertes Alkyl, Alkenyl, Alkoxyalkyl, für gegebenenfalls substituiertes Phenyl oder für jeweils gegebenenfalls substituiertes Cycloalkyl oder Benzyl steht, |
| $R^7$ | für Wasserstoff, jeweils gegebenenfalls durch Halogen substituiertes Alkyl oder Alkenyl steht oder |
| $R^6$ und $R^7$ | gemeinsam mit dem N-Atom, an das sie gebunden sind, für einen gegebenenfalls Sauerstoff oder Schwefel enthaltenden Ring stehen, |

$R^8$ und $R^9$ unabhängig voneinander für Wasserstoff oder jeweils gegebenenfalls substituiertes Alkyl, Phenyl oder Phenylalkyl stehen oder zusammen für einen gegebenenfalls substituierten Alkandiylrest stehen und

$R^{10}$ und $R^{11}$ unabhängig voneinander für jeweils gegebenenfalls durch Halogen substituiertes Alkyl, Alkenyl, Alkoxy, Alkylamino, Dialkylamino, Alkenylamino oder Dialkenylamino oder jeweils gegebenenfalls substituiertes Phenyl oder Benzyl stehen.

Die Verbindungen der Formel (I) können in Abhängigkeit von der Stellung des Substituenten G in den zwei isomeren Formen der Formeln (I-A) bzw. (I-B) vorliegen, was durch die gestrichelte Linie in der Formel (I) zum Ausdruck gebracht werden soll:

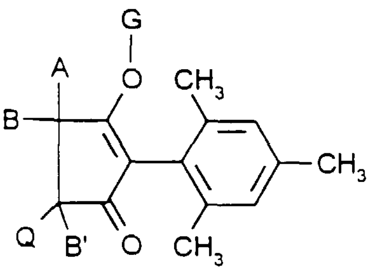

(I-A)

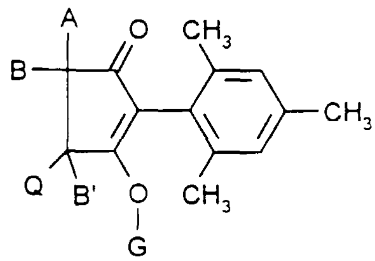

(I-B)

Die Verbindungen der Formeln (I-A) bzw. (I-B) können sowohl als Gemische als auch in Form ihrer reinen Isomeren vorliegen. Gemische der Verbindungen der Formeln (I-A) und (I-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die betreffende Verbindung gegebenenfalls als Isomerengemisch oder in der jeweils anderen isomeren Form vorliegen kann.

Unter Einbeziehung der verschiedenen Bedeutungen (a), (b), (c), (d), (e), (f) und (g) der Gruppe G ergeben sich folgende hauptsächlichen Strukturen (Ia) bis (Ig):

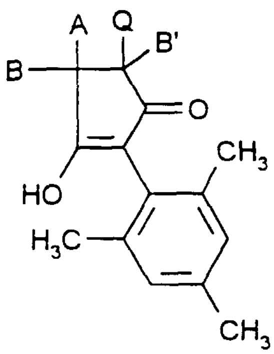

(Ia)

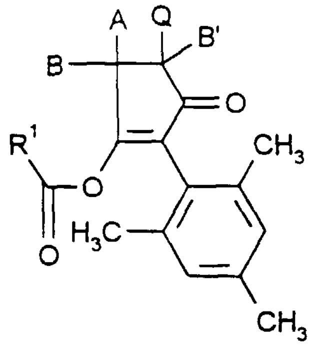

(Ib)

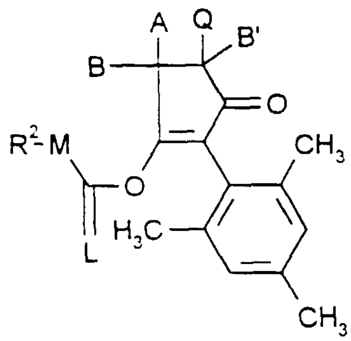

(Ic)

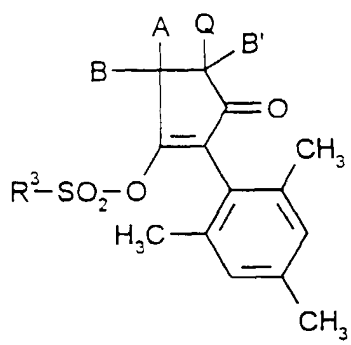

(Id)

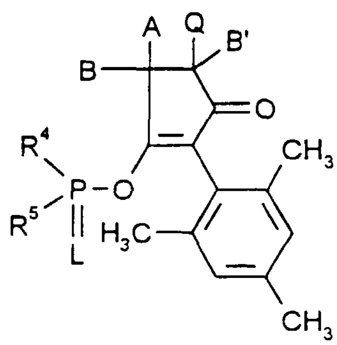

(Ie)

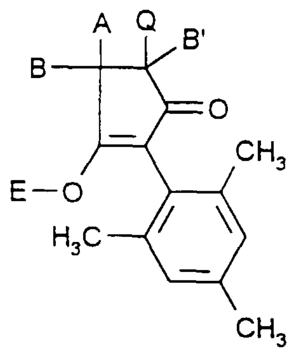

(If)

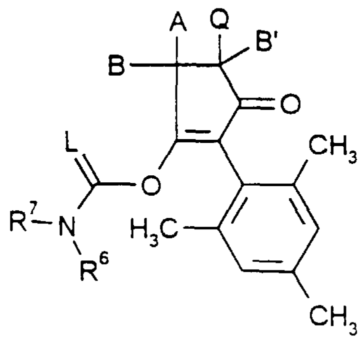

(Ig)

worin
A, B, B', E, L, M, Q, $R^1$, $R^2$, $R^3$, $R^4$, $R^5$, $R^6$ und $R^7$ die oben angegebenen Bedeutungen besitzen.

Aufgrund eines oder mehrerer Chiralitätszentren, fallen die Verbindungen der Formel (Ia) - (Ig) im allgemeinen als Stereoisomerengemisch an. Sie können sowohl in Form ihrer Diastereomerengemische als auch als reine Diastereomere oder Enantiomere vorliegen und verwendet werden.

Weiterhin wurde gefunden, daß man die neuen substituierten 2-Mesityl-cyclopentan-1,3-dion-Derivate der Formel (I) nach einem der im folgenden beschriebenen Verfahren erhält.

(A) Man erhält 2-Mesityl-cyclopentan-1,3-dione bzw. deren Enole der Formel (Ia)

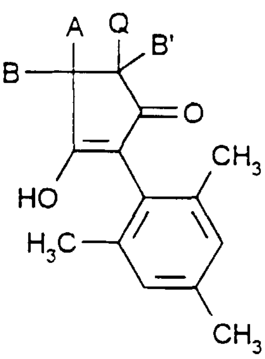

(Ia)

in welcher
A, B, B' und Q die oben angegebene Bedeutung haben,
wenn man
5-Aryl-4-keto-valeriansäureester der Formel (II)

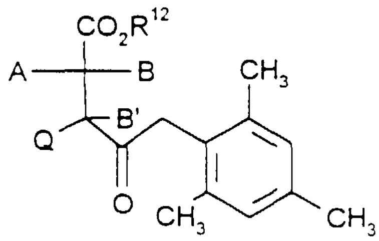

(II)

in welcher
A, B, B' und Q die oben angegebene Bedeutung haben, und
$R^{12}$ für Alkyl (bevorzugt $C_1$-$C_6$-Alkyl) steht,
in Gegenwart eines Verdünnungsmittels und in Gegenwart einer Base intramolekular kondensiert; und
(B) man erhält Verbindungen der Formel (Ib)

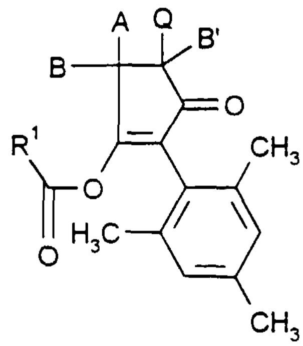

(Ib)

in welcher
A, B, B', Q und $R^1$ die oben angegebene Bedeutung haben,
wenn man Verbindungen der Formel (Ia),

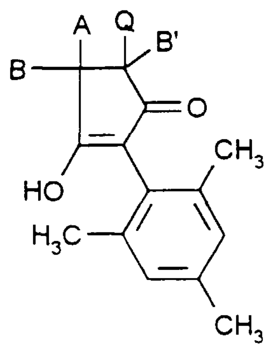

(Ia)

in welcher
A, B, B' und Q die oben angegebene Bedeutung haben,

α) mit Säurehalogeniden der Formel (III)

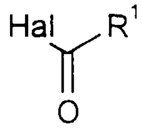

(III)

in welcher

$R^1$ die oben angegebene Bedeutung hat und
Hal für Halogen (insbesondere Chlor und Brom) steht,

gegebenenfalls in Gegenwart eines Verdünnungsmittels und gegebenenfalls in Gegenwart eines Säurebindemittels umsetzt
oder
β) mit Carbonsäureanhydriden der Formel (IV)

$$R^1\text{-CO-O-CO-}R^1 \quad \text{(IV)}$$

in welcher

$R^1$ die oben angegebene Bedeutung hat,

gegebenenfalls in Gegenwart eines Verdünnungsmittels und gegebenenfalls in Gegenwart eines Säurebindemittels umsetzt;

und
(C) man erhält Verbindungen der Formel (Ic-1)

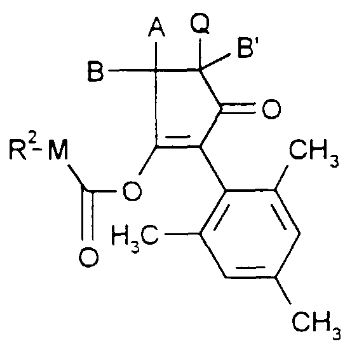

(Ic-1)

in welcher
A, B, B', Q und $R^2$ die oben angegebene Bedeutung haben,
und

M für Sauerstoff oder Schwefel steht,

wenn man Verbindungen der Formel (Ia)

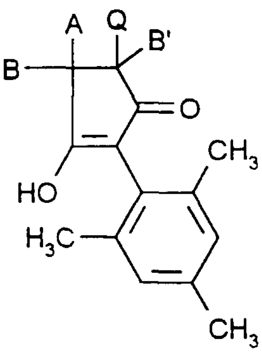

(Ia)

in welcher

A, B, B' und Q die oben angegebene Bedeutung haben,

mit einem Chlorameisensäureester oder Chlorameisensäurethiolester der Formel (V)

$$R^2\text{-M-CO-Cl} \quad (V)$$

in welcher
$R^2$ und M die oben angegebene Bedeutung haben,
gegebenenfalls in Gegenwart eines Verdünnungsmittels und gegebenenfalls in Gegenwart eines Säurebindemittels umsetzt,
und
(D) man erhält Verbindungen der Formel (Ic-2)

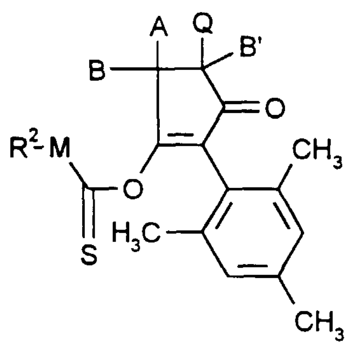

(Ic-2)

in welcher
A, B, B', Q und $R^2$ die oben angegebene Bedeutung haben
und

M für Sauerstoff oder Schwefel steht,

wenn man Verbindungen der Formel (Ia)

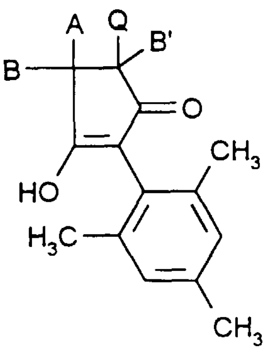

(Ia)

in welcher

A, B, B' und Q die oben angegebene Bedeutung haben,

α) mit einem Chlormonothioameisensäureester oder Chlordithioameisensäureester der Formel (VI)

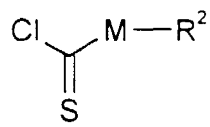

(VI)

in welcher

M und $R^2$ die oben angegebene Bedeutung haben,

gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der allgemeinen Formel (VII)

$$R^2\text{-Hal} \quad \text{(VII)}$$

in welcher

$R^2$ die oben angegebene Bedeutung hat

und

Hal für Chlor, Brom oder Iod steht,

gegebenenfalls in Gegenwart eines Verdünnungsmittels und gegebenenfalls in Gegenwart einer Base umsetzt;

und
(E) man erhält Verbindungen der Formel (Id)

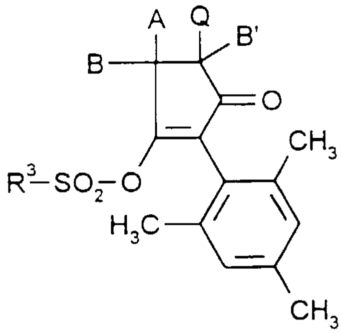

(Id)

in welcher

A, B, B', Q und $R^3$ die oben angegebene Bedeutung haben,

wenn man Verbindungen der Formel (Ia)

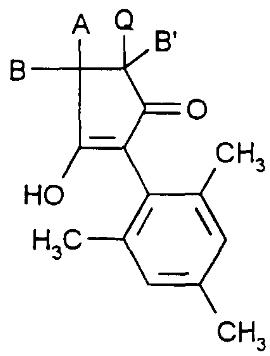

(Ia)

in welcher

A, B, B' und Q die oben angegebene Bedeutung haben,

mit Sulfonsäurechloriden der Formel (VIII)

$$R^3\text{-}SO_2\text{-}Cl \quad \text{(VIII)}$$

in welcher

$R^3$ die oben angegebene Bedeutung hat,

gegebenenfalls in Gegenwart eines Verdünnungsmittels und gegebenenfalls in Gegenwart eines Säurebindemittels umsetzt;
und
(F) man erhält Verbindungen der Formel (Ie)

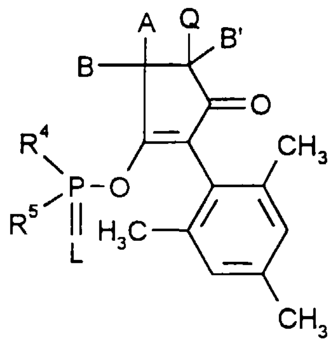

(Ie)

in welcher

A, B, L, B', Q, $R^4$ und $R^5$ die oben angegebene Bedeutung haben,

wenn man
Verbindungen der Formel (Ia) bzw. deren Enole

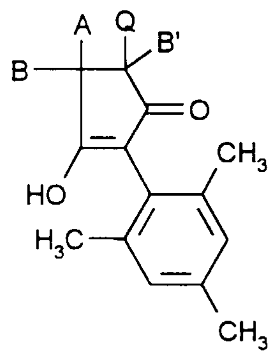

(Ia)

in welcher

A, B, B' und Q die oben angegebene Bedeutung haben,

mit Phosphorverbindungen der Formel (IX)

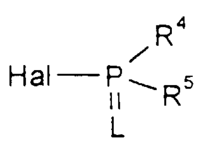

(IX)

in welcher

L, $R^4$ und $R^5$ die oben angegebene Bedeutung haben

und

Hal für Halogen (insbesondere Chlor und Brom) steht,

gegebenenfalls in Gegenwart eines Verdünnungsmittels und gegebenenfalls in Gegenwart eines Säurebindemittels umsetzt;
und

(G) man erhält Verbindungen der Formel (If)

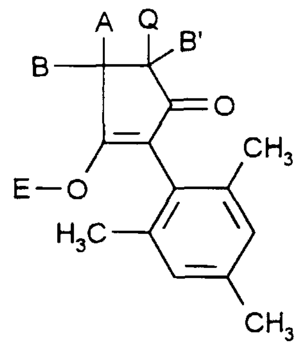

(If)

in welcher

A, B, B' und Q die oben angegebene Bedeutung haben,

und

E für ein Metallionäquivalent oder für ein Ammoniumion steht,

wenn man Verbindungen der Formel (Ia)

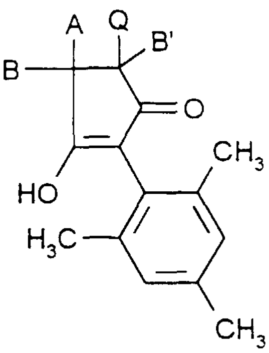

(Ia)

in welcher

A, B, B' und Q die oben angegebene Bedeutung haben,

mit Metall-Verbindungen oder Aminen der Formeln (X) und (XI)

$$Me\ R_t^{16} \quad (X)$$

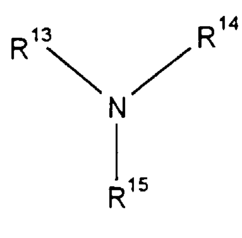

(XI)

in welchen

Me für ein- oder zweiwertige Metallionen (insbesondere Alkali- oder Erdalkaliionen, beispielsweise des Lithiums, Natriums, Kaliums, Magnesiums oder Calciums) steht,

t für die Zahl 1 oder 2 steht,

$R^{13}$, $R^{14}$ und $R^{15}$ unabhängig voneinander für Wasserstoff oder Alkyl (insbesondere $C_1$-$C_8$-Alkyl) stehen und

$R^{16}$ für Wasserstoff, Hydroxy oder $C_1$-$C_4$-Alkoxy steht,

gegebenenfalls in Gegenwart eines Verdünnungsmittels umsetzt.
(H) Ferner wurde gefunden, daß man Verbindungen der Formel (Ig)

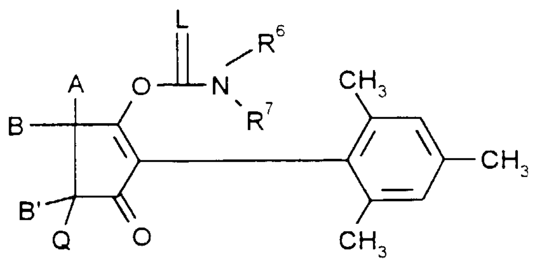

(Ig)

in welcher

A, B, L, B', Q, $R^6$ und $R^7$ die oben angegebene Bedeutung haben,

erhält, wenn man Verbindungen der Formel (Ia)

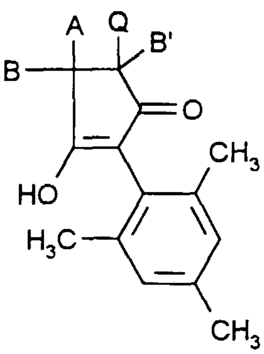

(Ia)

in welcher

A, B, B' und Q die oben angegebene Bedeutung haben,

α) mit Verbindungen der Formel (XII)

$$R^6\text{-N=C=L} \quad (XII)$$

in welcher

L und $R^6$ die oben angegebene Bedeutung haben,

gegebenenfalls in Gegenwart eines Verdünnungsmittels und gegebenenfalls in Gegenwart eines Katalysators oder

β) mit Carbamidsäurechloriden oder Thiocarbamidsäurechloriden der Formel (XIII)

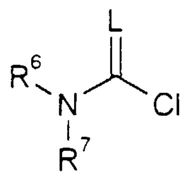

(XIII)

in welcher

L, $R^6$ und $R^7$ die oben angegebene Bedeutung haben

gegebenenfalls in Gegenwart eines Verdünnungsmittels und gegebenenfalls in Gegenwart eines Säurebindemittels umsetzt.

Weiterhin wurde gefunden, daß sich die neuen 2-(2,4,6-Trimethylphenyl)-cyclopentan-1,3-dion-Derivate der Formel (I) durch hervorragende insektizide, akarizide und herbizide Wirkungen auszeichnen.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.

A und Q stehen gemeinsam bevorzugt für jeweils gegebenenfalls einfach bis dreifach, gleich oder verschieden durch Halogen, Hydroxy, Mercapto, jeweils gegebenenfalls einfach bis neunfach, gleich oder verschieden durch Halogen substituiertes $C_1$-$C_{10}$-Alkyl, $C_1$-$C_6$-Alkoxy, $C_1$-$C_6$-Alkylthio, $C_3$-$C_7$-Cycloalkyl oder jeweils gegebenenfalls einfach bis fünffach, gleich oder verschieden durch Halogen, $C_1$-$C_6$-Alkyl oder $C_1$-$C_6$-Alkoxy substituiertes Benzyloxy oder Phenyl substituiertes $C_1$-$C_6$-Alkandiyl oder $C_2$-$C_6$-Alkendiyl, welches außerdem gegebenenfalls eine der nachstehenden Gruppierungen

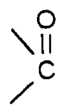 ; 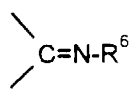 ; 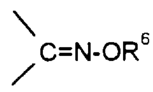 ; 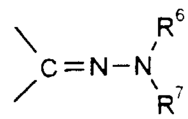 ;

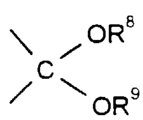 ; 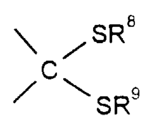 ; 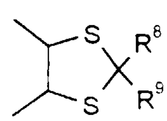 ;

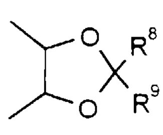 ; 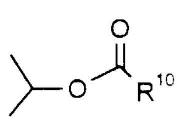 oder 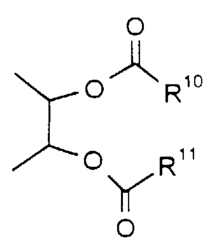

enthält oder durch eine $C_1$-$C_2$-Alkandiylgruppe überbrückt ist.

B und B' stehen unabhängig voneinander bevorzugt für Wasserstoff, Halogen oder $C_1$-$C_6$-Alkyl oder gemeinsam für jeweils gegebenenfalls durch $C_1$-$C_6$-Alkyl substituiertes $C_1$-$C_6$-Alkandiyl oder $C_4$-Alkendiyl.

G steht bevorzugt für Wasserstoff (a) oder für eine der Gruppen

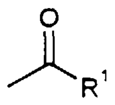 (b), 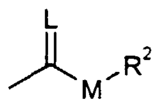 (c), 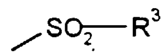 (d), 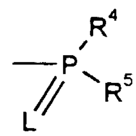 (e),

E (f)

oder

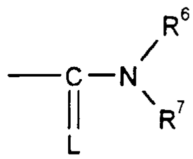 (g),

in welchen

E für ein Metallionäquivalent oder ein Ammoniumion steht,
L für Sauerstoff oder Schwefel steht und
M für Sauerstoff oder Schwefel steht.

$R^1$ steht bevorzugt für jeweils gegebenenfalls einfach oder mehrfach, gleich oder verschieden durch Halogen substituiertes $C_1$-$C_{20}$-Alkyl, $C_2$-$C_{10}$-Alkenyl, $C_1$-$C_8$-Alkoxy-$C_1$-$C_8$-alkyl, $C_1$-$C_8$-Alkylthio-$C_1$-$C_8$-alkyl, Poly-$C_1$-$C_8$-alkoxy-$C_1$-$C_8$-alkyl oder für gegebenenfalls einfach oder mehrfach, gleich oder verschieden durch Halogen, $C_1$-$C_6$-Alkyl oder $C_1$-$C_6$-Alkoxy substituiertes Cycloalkyl mit 3 bis 8 Ringatomen, in welchem mindestens eine Methylengruppe durch ein Sauerstoff- und/oder Schwefelatom ersetzt sein kann,
für gegebenenfalls einfach oder mehrfach, gleich oder verschieden durch Halogen, Nitro, Cyano, $C_1$-$C_6$-Alkyl, $C_1$-$C_6$-Alkoxy, $C_1$-$C_6$-Halogenalkyl oder $C_1$-$C_6$-Halogenalkoxy substituiertes Phenyl,
für gegebenenfalls einfach oder mehrfach, gleich oder verschieden durch Halogen, $C_1$-$C_6$-Alkyl, $C_1$-$C_6$-Alkoxy, $C_1$-$C_6$-Halogenalkyl oder $C_1$-$C_6$-Halogenalkoxy substituiertes Phenyl-$C_1$-$C_6$-alkyl,
für gegebenenfalls einfach oder mehrfach, gleich oder verschieden durch Halogen oder $C_1$-$C_6$-Alkyl substituiertes Hetaryl mit 5 oder 6 Ringatomen,
für gegebenenfalls einfach oder mehrfach, gleich oder verschieden durch Halogen oder $C_1$-$C_6$-Alkyl substituiertes Phenoxy-$C_1$-$C_6$-alkyl oder
für gegebenenfalls einfach oder mehrfach, gleich oder verschieden durch Halogen, Amino oder $C_1$-$C_6$-Alkyl substituiertes Hetaryloxy-$C_1$-$C_6$-alkyl mit 5 oder 6 Ringatomen.

$R^2$ steht bevorzugt für jeweils gegebenenfalls einfach oder mehrfach, gleich oder verschieden durch Halogen substituiertes $C_1$-$C_{20}$-Alkyl, $C_2$-$C_{10}$-Alkenyl, $C_1$-$C_8$-Alkoxy-$C_2$-$C_8$-alkyl, Poly-$C_1$-$C_8$-alkoxy-$C_2$-$C_8$-alkyl,
für gegebenenfalls einfach oder mehrfach, gleich oder verschieden durch Halogen, $C_1$-$C_6$-Alkyl oder $C_1$-$C_6$-Alkoxy substituiertes $C_3$-$C_6$-Cycloalkyl oder
für jeweils gegebenenfalls einfach oder mehrfach, gleich oder verschieden durch Halogen, Nitro, Cyano, $C_1$-$C_6$-Alkyl, $C_1$-$C_6$-Alkoxy, $C_1$-$C_3$-Halogenalkoxy oder $C_1$-$C_3$-Halogenalkyl substituiertes Phenyl oder Benzyl.

$R^3$ steht bevorzugt für jeweils gegebenenfalls einfach oder mehrfach, gleich oder verschieden durch Halogen substi-

tuiertes $C_1$-$C_{12}$-Alkyl oder für jeweils gegebenenfalls einfach oder mehrfach, gleich oder verschieden durch Halogen, $C_1$-$C_6$-Alkyl, $C_1$-$C_6$-Alkoxy, $C_1$-$C_3$-Halogenalkyl, $C_1$-$C_3$-Halogenalkoxy, Cyano oder Nitro substituiertes Phenyl oder Phenyl-$C_1$-$C_4$-alkyl.

$R^4$ und $R^5$ stehen unabhängig voneinander bevorzugt für jeweils gegebenenfalls einfach oder mehrfach, gleich oder verschieden durch Halogen substituiertes $C_1$-$C_8$-Alkyl, $C_1$-$C_8$-Alkoxy, $C_1$-$C_8$-Alkylamino, Di-($C_1$-$C_8$-alkyl)-amino, $C_1$-$C_8$-Alkylthio, $C_3$-$C_5$-Alkenylthio, $C_3$-$C_7$-Cycloalkylthio, für jeweils gegebenenfalls einfach oder mehrfach, gleich oder verschieden durch Halogen, Nitro, Cyano, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl oder $C_1$-$C_4$-Halogenalkyl substituiertes Phenyl, Phenoxy oder Phenylthio.

$R^6$ steht bevorzugt für Wasserstoff, jeweils gegebenenfalls einfach oder mehrfach, gleich oder verschieden durch Halogen substituiertes $C_1$-$C_{10}$-Alkyl, $C_3$-$C_8$-Alkenyl, $C_1$-$C_8$-Alkoxy-$C_2$-$C_8$-alkyl, für gegebenenfalls einfach oder mehrfach, gleich oder verschieden durch Halogen, $C_1$-$C_6$-Alkyl, $C_1$-$C_6$-Alkoxy, $C_1$-$C_3$-Halogenalkyl oder $C_1$-$C_3$-Halogenalkoxy substituiertes $C_3$-$C_{10}$-Cycloalkyl, für gegebenenfalls einfach oder mehrfach, gleich oder verschieden durch Halogen, $C_1$-$C_3$-Halogenalkyl, $C_1$-$C_8$-Alkyl, $C_1$-$C_3$-Halogenalkoxy oder $C_1$-$C_8$-Alkoxy substituiertes Phenyl oder für gegebenenfalls einfach oder mehrfach, gleich oder verschieden durch Halogen, $C_1$-$C_8$-Alkyl, $C_1$-$C_3$-Halogenalkyl, $C_1$-$C_3$-Halogenalkoxy oder $C_1$-$C_8$-Alkoxy substituiertes Benzyl.

$R^7$ steht bevorzugt für Wasserstoff, jeweils gegebenenfalls einfach oder mehrfach, gleich oder verschieden durch Halogen substituiertes $C_1$-$C_{10}$-Alkyl oder $C_3$-$C_{10}$-Alkenyl oder

$R^6$ und $R^7$ stehen bevorzugt gemeinsam mit dem N-Atom, an das sie gebunden sind, für einen gegebenenfalls Sauerstoff oder Schwefel enthaltenden 3- bis 7-gliedrigen Ring.

$R^8$ und $R^9$ stehen unabhängig voneinander bevorzugt für Wasserstoff, gegebenenfalls einfach oder mehrfach, gleich oder verschieden durch Halogen substituiertes $C_1$-$C_6$-Alkyl oder für jeweils gegebenenfalls einfach oder mehrfach, gleich oder verschieden durch Halogen, $C_1$-$C_6$-Alkyl, $C_1$-$C_6$-Alkoxy, $C_1$-$C_6$-Halogenalkyl, $C_1$-$C_6$-Halogenalkoxy, Nitro oder Cyano substituiertes Phenyl oder Phenyl-$C_1$-$C_4$-alkyl,
oder zusammen für gegebenenfalls einfach oder mehrfach, gleich oder verschieden durch Halogen, $C_1$-$C_6$-Alkyl, $C_1$-$C_6$-Alkoxy oder $C_1$-$C_3$-Halogenalkyl substituiertes $C_2$-$C_6$-Alkandiyl.

$R^{10}$ und $R^{11}$ stehen unabhängig voneinander bevorzugt für jeweils gegebenenfalls einfach oder mehrfach, gleich oder verschieden durch Halogen substituiertes $C_1$-$C_{10}$-Alkyl, $C_2$-$C_{10}$-Alkenyl, $C_1$-$C_{10}$-Alkoxy, $C_1$-$C_{10}$-Alkylamino oder Di-($C_1$-$C_{10}$-alkyl)-amino, $C_3$-$C_{10}$-Alkenylamino, Di-($C_3$-$C_{10}$-alkenyl)-amino oder jeweils einfach oder mehrfach, gleich oder verschieden durch Halogen, $C_1$-$C_4$-Alkyl, $C_1$-$C_4$-Alkoxy, $C_1$-$C_4$-Halogenalkyl, Nitro oder Cyano substituiertes Phenyl oder Benzyl.

A und Q stehen gemeinsam besonders bevorzugt für jeweils gegebenenfalls einfach bis dreifach, gleich oder verschieden durch Fluor, Chlor, Brom, Hydroxy, Mercapto, durch jeweils gegebenenfalls einfach bis fünffach, gleich oder verschieden durch Fluor oder Chlor substituiertes $C_1$-$C_8$-Alkyl, $C_1$-$C_4$-Alkoxy, $C_1$-$C_4$-Alkylthio, $C_5$-$C_7$-Cycloalkyl oder Phenyl substituiertes $C_1$-$C_5$-Alkandiyl oder $C_2$-$C_5$-Alkendiyl, welches außerdem gegebenenfalls eine der nachstehenden Gruppierungen

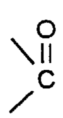 ; 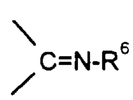 ; 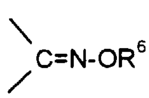 ; 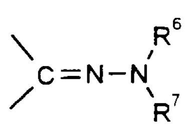 ;

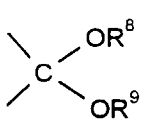 ; 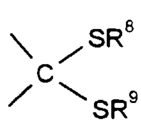 ; 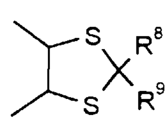 ;

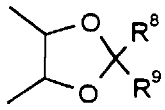 ; 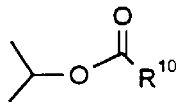 oder 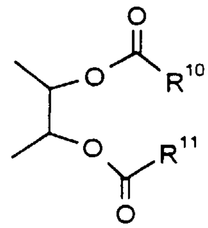

enthält oder durch eine $C_1$-$C_2$-Alkandiylgruppe überbrückt ist.

B und B'stehen unabhängig voneinander besonders bevorzugt für Wasserstoff, Fluor, Chlor oder $C_1$-$C_4$-Alkyl oder gemeinsam für jeweils gegebenenfalls durch $C_1$-$C_4$-Alkyl substituiertes $C_1$-$C_5$-Alkandiyl oder $C_4$-Alkendiyl.

G steht besonders bevorzugt für Wasserstoff (a) oder für eine der Gruppen

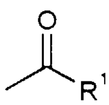 (b), 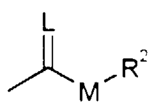 (c), 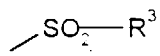 (d), 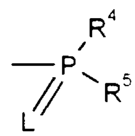 (e),

E (f)

oder

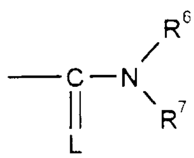 (g),

in welchen

E für ein Metallionäquivalent oder ein Ammoniumion steht,
L für Sauerstoff oder Schwefel steht und
M für Sauerstoff oder Schwefel steht.

$R^1$ steht besonders bevorzugt für jeweils gegebenenfalls einfach bis neunfach, gleich oder verschieden durch Fluor oder Chlor substituiertes $C_1$-$C_{16}$-Alkyl, $C_2$-$C_8$-Alkenyl, $C_1$-$C_6$-Alkoxy-$C_1$-$C_6$-alkyl, $C_1$-$C_6$-Alkylthio-$C_1$-$C_6$-alkyl, Poly-$C_1$-$C_6$-alkoxy-$C_1$-$C_6$-alkyl oder für gegebenenfalls einfach bis dreifach, gleich oder verschieden durch Fluor, Chlor, $C_1$-$C_4$-Alkyl oder $C_1$-$C_4$-Alkoxy substituiertes Cycloalkyl mit 3 bis 7 Ringatomen, in welchem ein oder zwei Methylengruppen durch Sauerstoff- und/oder Schwefelatome ersetzt sein können.
für gegebenenfalls einfach bis dreifach, gleich oder verschieden durch Fluor, Chlor, Brom, Cyano, Nitro, $C_1$-$C_4$-Alkyl, $C_1$-$C_4$-Alkoxy, $C_1$-$C_3$-Halogenalkyl oder $C_1$-$C_3$-Halogenalkoxy substituiertes Phenyl,
für gegebenenfalls einfach bis dreifach, gleich oder verschieden durch Fluor, Chlor, Brom, $C_1$-$C_4$-Alkyl, $C_1$-$C_4$-Alkoxy, $C_1$-$C_3$-Halogenalkyl oder $C_1$-$C_3$-Halogenalkoxy substituiertes Phenyl-$C_1$-$C_4$-alkyl ,
für jeweils gegebenenfalls einfach oder zweifach, gleich oder verschieden durch Fluor, Chlor, Brom oder $C_1$-$C_4$-Alkyl substituiertes Furanyl, Thienyl, Pyridyl, Pyrimidyl, Thiazolyl oder Pyrazolyl,
für gegebenenfalls einfach bis dreifach, gleich oder verschieden durch Fluor, Chlor oder $C_1$-$C_4$-Alkyl substituiertes Phenoxy-$C_1$-$C_5$-alkyl oder
für jeweils gegebenenfalls einfach oder zweifach, gleich oder verschieden durch Fluor, Chlor, Amino oder $C_1$-$C_4$-Alkyl substituiertes Pyridyloxy-$C_1$-$C_6$-alkyl, Pyrimidinyloxy-$C_1$-$C_6$-alkyl oder Thiazolyloxy-$C_1$-$C_6$-alkyl

$R^2$ steht besonders bevorzugt für jeweils gegebenenfalls einfach bis neunfach, gleich oder verschieden durch Fluor oder Chlor substituiertes $C_1$-$C_{16}$-Alkyl, $C_2$-$C_8$-Alkenyl, $C_1$-$C_6$-Alkoxy-$C_2$-$C_6$-alkyl, Poly-$C_1$-$C_6$-alkoxy-$C_2$-$C_6$-alkyl, für gegebenenfalls einfach bis fünffach, gleich oder verschieden durch Fluor, Chlor, $C_1$-$C_4$-Alkyl oder $C_1$-$C_4$-Alkoxy substituiertes $C_3$-$C_6$-Cycloalkyl oder
für jeweils gegebenenfalls einfach bis dreifach, gleich oder verschieden durch Fluor, Chlor, Brom, Nitro, Cyano, $C_1$-$C_4$-Alkyl, $C_1$-$C_4$-Alkoxy, $C_1$-$C_2$-Halogenalkoxy oder $C_1$-$C_2$-Halogenalkyl substituiertes Phenyl oder Benzyl.

$R^3$ steht besonders bevorzugt für gegebenenfalls einfach bis fünffach, gleich oder verschieden durch Fluor oder Chlor substituiertes $C_1$-$C_9$-Alkyl oder für jeweils gegebenenfalls einfach bis dreifach, gleich oder verschieden durch Fluor, Chlor, Brom, $C_1$-$C_4$-Alkyl, $C_1$-$C_4$-Alkoxy, $C_1$-$C_2$-Halogenalkyl, $C_1$-$C_2$-Halogenalkoxy, Cyano oder Nitro substituiertes Phenyl oder Phenyl-$C_1$-$C_2$-alkyl.

$R^4$ und $R^5$stehen unabhängig voneinander besonders bevorzugt für gegebenenfalls einfach bis fünffach, gleich oder verschieden durch Fluor oder Chlor substituiertes $C_1$-$C_6$-Alkyl, $C_1$-$C_6$-Alkoxy, $C_1$-$C_6$-Alkylamino, Di-($C_1$-$C_6$-alkyl)-amino, $C_1$-$C_6$-Alkylthio, $C_3$-$C_4$-Alkenylthio, $C_3$-$C_6$-Cycloalkylthio oder für jeweils gegebenenfalls einfach bis dreifach, gleich oder verschieden durch Fluor, Chlor, Brom, Nitro, Cyano, $C_1$-$C_3$-Alkoxy, $C_1$-$C_3$-Halogenalkoxy, $C_1$-$C_3$-Alkylthio, $C_1$-$C_3$-Halogenalkylthio, $C_1$-$C_3$-Alkyl oder $C_1$-$C_3$-Halogenalkyl substituiertes Phenyl, Phenoxy oder Phenylthio.

$R^6$ steht besonders bevorzugt für Wasserstoff, jeweils gegebenenfalls einfach bis fünffach, gleich oder verschieden durch Fluor oder Chlor substituiertes $C_1$-$C_8$-Alkyl, $C_3$-$C_6$-Alkenyl, $C_1$-$C_6$-Alkoxy-$C_2$-$C_6$-alkyl, für gegebenenfalls einfach bis dreifach, gleich oder verschieden durch Fluor, Chlor, $C_1$-$C_4$-Alkyl, $C_1$-$C_4$-Alkoxy, $C_1$-$C_2$-Halogenalkyl oder $C_1$-$C_2$-Halogenalkoxy substituiertes $C_3$-$C_8$-Cycloalkyl, für gegebenenfalls einfach bis dreifach, gleich oder verschieden durch Fluor, Chlor, Brom, $C_1$-$C_2$-Halogenalkyl, $C_1$-$C_5$-Alkyl, $C_1$-$C_2$-Halogenalkoxy oder $C_1$-$C_5$-Alkoxy substituiertes Phenyl oder für gegebenenfalls einfach bis dreifach, gleich oder verschieden durch Fluor, Chlor, Brom, $C_1$-$C_5$-Alkyl, $C_1$-$C_2$-Halogenalkyl, $C_1$-$C_2$-Halogenalkoxy oder $C_1$-$C_5$-Alkoxy substituiertes Benzyl.

$R^7$ steht besonders bevorzugt für Wasserstoff, jeweils gegebenenfalls einfach bis fünffach, gleich oder verschieden durch Fluor oder Chlor substituiertes $C_1$-$C_8$-Alkyl oder $C_3$-$C_8$-Alkenyl oder

$R^6$ und $R^7$stehen besonders bevorzugt gemeinsam mit dem N-Atom, an das sie gebunden sind, für einen gegebenenfalls Sauerstoff oder Schwefel enthaltenden 4- bis 7-gliedrigen Ring.

$R^8$ und $R^9$stehen unabhängig voneinander besonders bevorzugt für Wasserstoff, gegebenenfalls einfach bis fünffach, gleich oder verschieden durch Fluor oder Chlor substituiertes $C_1$-$C_4$-Alkyl oder für gegebenenfalls einfach bis dreifach, gleich oder verschieden durch Fluor, Chlor, $C_1$-$C_4$-Alkyl, $C_1$-$C_4$-Alkoxy, $C_1$-$C_2$-Halogenalkyl, $C_1$-$C_2$-Halogenalkoxy, Nitro oder Cyano substituiertes Phenyl
oder zusammen für gegebenenfalls einfach bis dreifach, gleich oder verschieden durch Fluor, Chlor, $C_1$-$C_4$-Alkyl, $C_1$-$C_4$-Alkoxy oder $C_1$-$C_2$-Halogenalkyl substituiertes $C_2$-$C_5$-Alkandiyl.

$R^{10}$ und $R^{11}$stehen unabhängig voneinander besonders bevorzugt für jeweils gegebenenfalls einfach bis fünffach, gleich oder verschieden durch Fluor oder Chlor substituiertes $C_1$-$C_8$-Alkyl, $C_2$-$C_8$-Alkenyl, $C_1$-$C_8$-Alkoxy, $C_1$-$C_8$-Alkylamino, $C_3$-$C_8$-Alkenylamino, Di-($C_1$-$C_8$-alkyl)-amino oder Di-($C_3$-$C_8$-alkenyl)-amino.

A und Qstehen gemeinsam ganz besonders bevorzugt für jeweils gegebenenfalls einfach oder zweifach, gleich oder verschieden durch Fluor, Chlor, Hydroxy, Mercapto, durch jeweils gegebenenfalls einfach bis dreifach, gleich oder verschieden durch Fluor oder Chlor substituiertes $C_1$-$C_6$-Alkyl, $C_1$-$C_2$-Alkoxy, $C_1$-$C_2$-Alkylthio, $C_5$-$C_6$-Cycloalkyl oder Phenyl substituiertes $C_1$-$C_4$-Alkandiyl oder $C_2$-$C_4$-Alkendiyl, welches außerdem gegebenenfalls eine der nachstehenden Gruppen

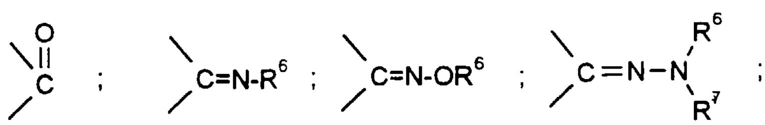

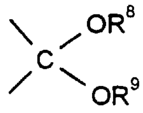 ; 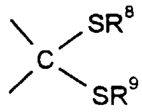 ; 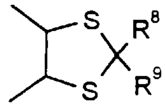 ;

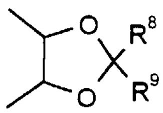 ; 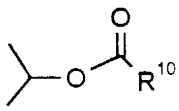 oder 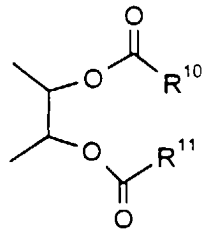

enthält oder durch eine $C_1$-$C_2$-Alkandiylgruppe überbrückt ist.

B und B'stehen unabhängig voneinander ganz besonders bevorzugt für Wasserstoff, Fluor, Chlor, Methyl, Ethyl oder gemeinsam für jeweils gegebenenfalls durch Methyl oder Ethyl substituiertes $C_1$-$C_4$-Alkandiyl oder $C_4$-Alkendiyl.

G steht ganz besonders bevorzugt für Wasserstoff (a) oder für eine der Gruppen

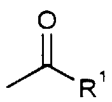 (b), 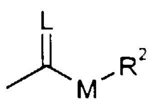 (c), 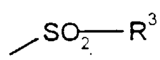 (d), 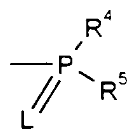 (e),

E (f)

oder

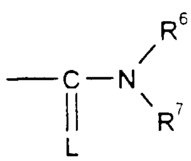 (g),

in welchen

E für ein Metallionäquivalent oder ein Ammoniumion steht,

L für Sauerstoff oder Schwefel steht und

M für Sauerstoff oder Schwefel steht.

$R^1$ steht ganz besonders bevorzugt für jeweils gegebenenfalls einfach bis fünffach, gleich oder verschieden durch Fluor oder Chlor substituiertes $C_1$-$C_{14}$-Alkyl, $C_2$-$C_6$-Alkenyl, $C_1$-$C_4$-Alkoxy-$C_1$-$C_6$-alkyl, $C_1$-$C_4$-Alkylthio-$C_1$-$C_6$-alkyl, Poly-$C_1$-$C_4$-alkoxy-$C_1$-$C_4$-alkyl oder für gegebenenfalls einfach oder zweifach, gleich oder verschieden durch Fluor, Chlor, Methyl, Ethyl, Methoxy oder Ethoxy substituiertes Cycloalkyl mit 3 bis 6 Ringatomen, in welchem ein oder zwei Methylengruppen durch Sauerstoff- und/oder Schwefelatome ersetzt sein können,
für gegebenenfalls einfach oder zweifach, gleich oder verschieden durch Fluor, Chlor, Brom, Methyl, Ethyl, Propyl,

i-Propyl, Methoxy, Ethoxy, Trifluormethyl, Trifluormethoxy, Cyano oder Nitro substituiertes Phenyl,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl oder Trifluormethoxy substituiertes Benzyl,
für jeweils gegebenenfalls einfach oder zweifach, gleich oder verschieden durch Fluor, Chlor, Brom, Methyl oder Ethyl substituiertes Thienyl, Furanyl oder Pyridyl,
für gegebenenfalls einfach oder zweifach, gleich oder verschieden durch Fluor, Chlor, Methyl oder Ethyl substituiertes Phenoxy-$C_1$-$C_4$-alkyl oder
für jeweils gegebenenfalls einfach oder zweifach, gleich oder verschieden durch Fluor, Chlor, Amino, Methyl oder Ethyl substituiertes Pyridyloxy-$C_1$-$C_4$-alkyl, Pyrimidyloxy-$C_1$-$C_4$-alkyl oder Thiazolyloxy-$C_1$-$C_4$-alkyl.

$R^2$ steht ganz besonders bevorzugt für jeweils gegebenenfalls einfach bis fünffach, gleich oder verschieden durch Fluor oder Chlor substituiertes $C_1$-$C_{14}$-Alkyl, $C_2$-$C_6$-Alkenyl, $C_1$-$C_4$-Alkoxy-$C_2$-$C_6$-alkyl, Poly-$C_1$-$C_4$-alkoxy-$C_2$-$C_6$-alkyl, für gegebenenfalls einfach bis dreifach, gleich oder verschieden durch Fluor, Chlor, Methyl, Ethyl, Methoxy oder Ethoxy substituiertes $C_3$-$C_6$-Cycloalkyl
oder für jeweils gegebenenfalls einfach oder zweifach, gleich oder verschieden durch Fluor, Chlor, Nitro, Cyano, Methyl, Ethyl, Propyl, i-Propyl, Methoxy, Ethoxy, Trifluormethoxy oder Trifluormethyl substituiertes Phenyl oder Benzyl.

$R^3$ steht ganz besonders bevorzugt für gegebenenfalls einfach bis dreifach, gleich oder verschieden durch Fluor oder Chlor substituiertes $C_1$-$C_6$-Alkyl oder für jeweils gegebenenfalls einfach oder zweifach durch Fluor, Chlor, Brom, Methyl, Ethyl, Methoxy, Ethoxy, Trifluormethyl, Trifluormethoxy, Cyano oder Nitro substituiertes Phenyl oder Benzyl.

$R^4$ und $R^5$stehen unabhängig voneinander ganz besonders bevorzugt für jeweils gegebenenfalls einfach bis dreifach, gleich oder verschieden durch Fluor oder Chlor substituiertes $C_1$-$C_4$-Alkyl, $C_1$-$C_4$-Alkoxy, $C_1$-$C_4$-Alkylamino, Di-($C_1$-$C_4$-alkyl)-amino, $C_1$-$C_4$-Alkylthio oder für jeweils gegebenenfalls einfach oder zweifach, gleich oder verschieden durch Fluor, Chlor, Brom, Nitro, Cyano, $C_1$-$C_2$-Alkoxy, Trifluormethoxy, $C_1$-$C_2$-Alkylthio, Trifluormethyl oder $C_1$-$C_3$-Alkyl substituiertes Phenyl, Phenoxy oder Phenylthio.

$R^6$ steht ganz besonders bevorzugt für Wasserstoff, jeweils gegebenenfalls einfach bis dreifach, gleich oder verschieden durch Fluor oder Chlor substituiertes $C_1$-$C_6$-Alkyl, $C_1$-$C_6$-Alkoxy-$C_2$-$C_4$-alkyl, für gegebenenfalls einfach oder zweifach, gleich oder verschieden durch Fluor, Chlor, Methyl, Methoxy, Trifluormethyl oder Trifluormethoxy substituiertes $C_3$-$C_6$-Cycloalkyl, für gegebenenfalls einfach oder zweifach, gleich oder verschieden durch Fluor, Chlor, Trifluormethyl, $C_1$-$C_4$-Alkyl, Trifluormethoxy oder $C_1$-$C_4$-Alkoxy substituiertes Phenyl oder für gegebenenfalls einfach oder zweifach, gleich oder verschieden durch Fluor, Chlor, $C_1$-$C_4$-Alkyl, Trifluormethyl, Trifluormethoxy oder $C_1$-$C_4$-Alkoxy substituiertes Benzyl.

$R^7$ steht ganz besonders bevorzugt für Wasserstoff, jeweils gegebenenfalls einfach bis dreifach, gleich oder verschieden durch Fluor oder Chlor substituiertes $C_1$-$C_6$-Alkyl oder $C_3$-$C_6$-Alkenyl oder

$R^6$ und $R^7$stehen ganz besonders bevorzugt gemeinsam mit dem N-Atom, an das sie gebunden sind, für einen gegebenenfalls Sauerstoff oder Schwefel enthaltenden 5- bis 7-gliedrigen Ring.

$R^8$ und $R^9$stehen unabhängig voneinander ganz besonders bevorzugt für Wasserstoff, gegebenenfalls einfach bis dreifach, gleich oder verschieden durch Fluor oder Chlor substituiertes $C_1$-$C_4$-Alkyl oder für gegebenenfalls einfach oder zweifach, gleich oder verschieden durch Fluor, Chlor, Methyl, Ethyl, Methoxy, Ethoxy, Trifluormethyl, Trifluormethoxy, Nitro oder Cyano substituiertes Phenyl
oder zusammen für gegebenenfalls einfach oder zweifach, gleich oder verschieden durch Fluor, Chlor, Methyl, Ethyl, Methoxy, Ethoxy oder Trifluormethyl substituiertes $C_2$-$C_5$-Alkandiyl.

$R^{10}$ und $R^{11}$stehen unabhängig voneinander ganz besonders bevorzugt für $C_1$-$C_6$-Alkyl oder $C_2$-$C_6$-Alkenyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.
In den oben aufgeführten Definitionen können gesättigte oder ungesättigte Kohlenwasserstoffreste, auch in Verbindung mit Heteroatomen (beispielsweise Alkoxy oder Alkenylthio) jeweils, soweit möglich, geradkettig oder verzweigt sein.
Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Im einzelnen seien außer den bei den Herstellungsbeispielen genannten Verbindungen die folgenden 2-Mesityl-3-hydroxy-$\Delta^2$-cyclopentenon-Derivate der Formeln (Ia) bis (Ig) (Tabellen 1 bis 6) genannt.

**Tabelle 1**

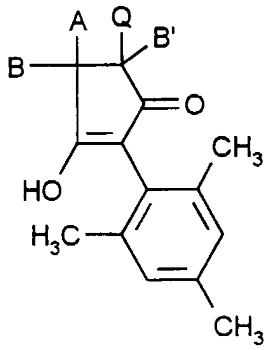

(Ia)

| A Q | B | B' |
|---|---|---|
| $-CH_2-$ | H | H |
| $-(CH_2)_2-$ | H | H |
| 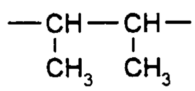 | H | H |
| $-(CH_2)_2-$ | $CH_3$ | $CH_3$ |
| $-(CH_2)_3-$ | H | H |
| $-(CH_2)_2-CHCH_3-$ | H | H |
| $-(CH_2)_2-CHOCH_3-$ | H | H |
| $-CH_2-CHCH_3-CH_2-$ | H | H |
| $-CH_2-CHOCH_3-CH_2-$ | H | H |
| $-CH_2-CHCH_3-CHCH_3-$ | H | H |
| $-CH_2-CHOCH_3-CHOCH_3-$ | H | H |
| $-(CH_2)_4-$ | H | H |
| $-(CH_2)_3-CHCH_3-$ | H | H |
| $-(CH_2)_3-CHOCH_3-$ | H | H |
| $-(CH_2)_2-CHCH_3-CH_2-$ | H | H |
| $-(CH_2)_2-CHOCH_3-CH_2-$ | H | H |

**Tabelle 1 (Fortsetzung)**

| A Q | B | B' |
|---|---|---|
| $-(CH_2)_3-CHCH_3-$ | H | H |
| $-(CH_2)_3-CHOCH_3-$ | H | H |
| $-(CH_2)_4-$ | $CH_3$ | H |
| $-CH_2-CHCH_3-CHCH_3-CH_2-$ | H | H |
| $-(CH_2)_2-CHCH_3-CHCH_3-$ | H | H |
| $-(CH_2)_2-CHOH-CH_2-$ | H | H |
| $-CH_2-CHOH-CHOH-CH_2-$ | H | H |
| 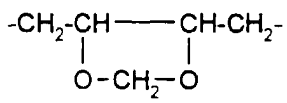 | H | H |
| 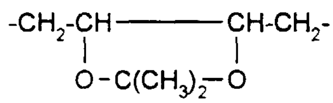 | H | H |
| $-(CH_2)_4-$ | $-CH_2-$ | |
| $-(CH_2)_4-$ | $-(CH_2)_2-$ | |
| 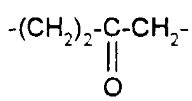 | H | H |
| 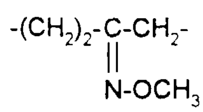 | H | H |

**Tabelle 2**

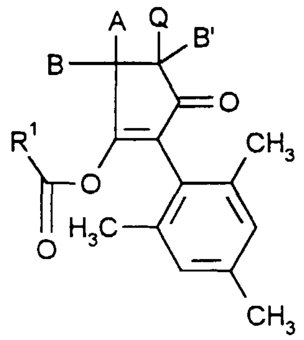

(Ib)

| A Q | B | B' | $R^1$ |
|---|---|---|---|
| $-CH_2-$ | H | H | $CH_3$ |
| $-(CH_2)_2-$ | H | H | $CH_3$ |
| 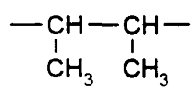 | H | H | $CH_3$ |
| $-(CH_2)_2-$ | $CH_3$ | $CH_3$ | $CH_3$ |
| $-(CH_2)_3-$ | H | H | $CH_3$ |
| $-(CH_2)_2-CHCH_3-$ | H | H | $CH_3$ |
| $-(CH_2)_2-CHOCH_3-$ | H | H | $CH_3$ |
| $-CH_2-CHCH_3-CH_2-$ | H | H | $CH_3$ |
| $-CH_2-CHOCH_3-CH_2-$ | H | H | $CH_3$ |
| $-CH_2-CHCH_3-CHCH_3-$ | H | H | $CH_3$ |
| $-CH_2-CHOCH_3-CHOCH_3-$ | H | H | $CH_3$ |
| $-(CH_2)_4-$ | H | H | $CH_3$ |
| $-(CH_2)_3-CHCH_3-$ | H | H | $CH_3$ |
| $-(CH_2)_3-CHOCH_3-$ | H | H | $CH_3$ |
| $-(CH_2)_2-CHCH_3-CH_2-$ | H | H | $CH_3$ |
| $-(CH_2)_2-CHOCH_3-CH_2-$ | H | H | $CH_3$ |

**Tabelle 2 (Fortsetzung)**

| A Q | B | B' | $R^1$ |
|---|---|---|---|
| $-(CH_2)_3-CHCH_3-$ | H | H | $CH_3$ |
| $-(CH_2)_3-CHOCH_3-$ | H | H | $CH_3$ |
| $-(CH_2)_4-$ | $CH_3$ | H | $CH_3$ |
| $-CH_2-CHCH_3-CHCH_3-CH_2-$ | H | H | $CH_3$ |
| $-(CH_2)_2-CHCH_3-CHCH_3-$ | H | H | $CH_3$ |
| $-(CH_2)_2-CHOH-CH_2-$ | H | H | $CH_3$ |
| $-CH_2-CHOH-CHOH-CH_2-$ | H | H | $CH_3$ |
| 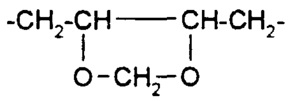 | H | H | $CH_3$ |
| 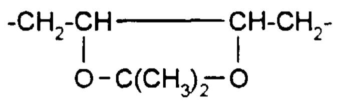 | H | H | $CH_3$ |
| $-(CH_2)_4-$ | $-CH_2-$ | | $CH_3$ |
| $-(CH_2)_4-$ | $-(CH_2)_2-$ | | $CH_3$ |
| 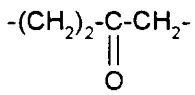 | H | H | $CH_3$ |
| 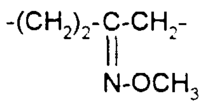 | H | H | $CH_3$ |

**Tabelle 2 (Fortsetzung)**

| A Q | B | B' | $R^1$ |
|---|---|---|---|
| $-CH_2-$ | H | H | $i-C_3H_7$ |
| $-(CH_2)_2-$ | H | H | $i-C_3H_7$ |
| 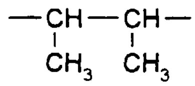 | H | H | $i-C_3H_7$ |
| $-(CH_2)_2-$ | $CH_3$ | $CH_3$ | $i-C_3H_7$ |
| $-(CH_2)_3-$ | H | H | $i-C_3H_7$ |
| $-(CH_2)_2-CHCH_3-$ | H | H | $i-C_3H_7$ |
| $-(CH_2)_2-CHOCH_3-$ | H | H | $i-C_3H_7$ |
| $-CH_2-CHCH_3-CH_2-$ | H | H | $i-C_3H_7$ |
| $-CH_2-CHOCH_3-CH_2-$ | H | H | $i-C_3H_7$ |
| $-CH_2-CHCH_3-CHCH_3-$ | H | H | $i-C_3H_7$ |
| $-CH_2-CHOCH_3-CHOCH_3-$ | H | H | $i-C_3H_7$ |
| $-(CH_2)_4-$ | H | H | $i-C_3H_7$ |
| $-(CH_2)_3-CHCH_3-$ | H | H | $i-C_3H_7$ |
| $-(CH_2)_3-CHOCH_3-$ | H | H | $i-C_3H_7$ |
| $-(CH_2)_2-CHCH_3-CH_2-$ | H | H | $i-C_3H_7$ |
| $-(CH_2)_2-CHOCH_3-CH_2-$ | H | H | $i-C_3H_7$ |

**Tabelle 2 (Fortsetzung)**

| A Q | B | B' | $R^1$ |
|---|---|---|---|
| $-(CH_2)_3-CHCH_3-$ | H | H | $i-C_3H_7$ |
| $-(CH_2)_3-CHOCH_3-$ | H | H | $i-C_3H_7$ |
| $-(CH_2)_4-$ | $CH_3$ | H | $i-C_3H_7$ |
| $-CH_2-CHCH_3-CHCH_3-CH_2-$ | H | H | $i-C_3H_7$ |
| $-(CH_2)_2-CHCH_3-CHCH_3-$ | H | H | $i-C_3H_7$ |
| $-(CH_2)_2-CHOH-CH_2-$ | H | H | $i-C_3H_7$ |
| $-CH_2-CHOH-CHOH-CH_2-$ | H | H | $i-C_3H_7$ |
| 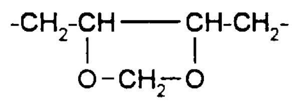 | H | H | $i-C_3H_7$ |
| 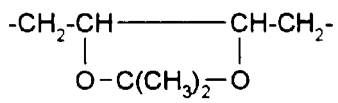 | H | H | $i-C_3H_7$ |
| $-(CH_2)_4-$ | $-CH_2-$ | | $i-C_3H_7$ |
| $-(CH_2)_4-$ | $-(CH_2)_2-$ | | $i-C_3H_7$ |
| 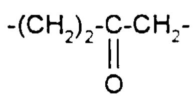 | H | H | $i-C_3H_7$ |
| 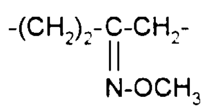 | H | H | $i-C_3H_7$ |

**Tabelle 2 (Fortsetzung)**

| A Q | B | B' | $R^1$ |
|---|---|---|---|
| $-CH_2-$ | H | H | t-$C_4H_9$ |
| $-(CH_2)_2-$ | H | H | t-$C_4H_9$ |
| 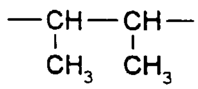 | H | H | t-$C_4H_9$ |
| $-(CH_2)_2-$ | $CH_3$ | $CH_3$ | t-$C_4H_9$ |
| $-(CH_2)_3-$ | H | H | t-$C_4H_9$ |
| $-(CH_2)_2-CHCH_3-$ | H | H | t-$C_4H_9$ |
| $-(CH_2)_2-CHOCH_3-$ | H | H | t-$C_4H_9$ |
| $-CH_2-CHCH_3-CH_2-$ | H | H | t-$C_4H_9$ |
| $-CH_2-CHOCH_3-CH_2-$ | H | H | t-$C_4H_9$ |
| $-CH_2-CHCH_3-CHCH_3-$ | H | H | t-$C_4H_9$ |
| $-CH_2-CHOCH_3-CHOCH_3-$ | H | H | t-$C_4H_9$ |
| $-(CH_2)_4-$ | H | H | t-$C_4H_9$ |
| $-(CH_2)_3-CHCH_3-$ | H | H | t-$C_4H_9$ |
| $-(CH_2)_3-CHOCH_3-$ | H | H | t-$C_4H_9$ |
| $-(CH_2)_2-CHCH_3-CH_2-$ | H | H | t-$C_4H_9$ |
| $-(CH_2)_2-CHOCH_3-CH_2-$ | H | H | t-$C_4H_9$ |

**Tabelle 2 (Fortsetzung)**

| A Q | B | B' | $R^1$ |
|---|---|---|---|
| $-(CH_2)_3-CHCH_3-$ | H | H | $t-C_4H_9$ |
| $-(CH_2)_3-CHOCH_3-$ | H | H | $t-C_4H_9$ |
| $-(CH_2)_4-$ | $CH_3$ | H | $t-C_4H_9$ |
| $-CH_2-CHCH_3-CHCH_3-CH_2-$ | H | H | $t-C_4H_9$ |
| $-(CH_2)_2-CHCH_3-CHCH_3-$ | H | H | $t-C_4H_9$ |
| $-(CH_2)_2-CHOH-CH_2-$ | H | H | $t-C_4H_9$ |
| $-CH_2-CHOH-CHOH-CH_2-$ | H | H | $t-C_4H_9$ |
| 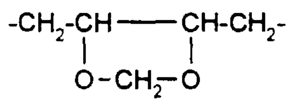 | H | H | $t-C_4H_9$ |
| 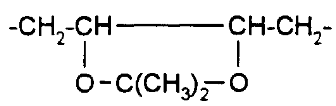 | H | H | $t-C_4H_9$ |
| $-(CH_2)_4-$ | $-CH_2-$ | | $t-C_4H_9$ |
| $-(CH_2)_4-$ | $-(CH_2)_2-$ | | $t-C_4H_9$ |
| 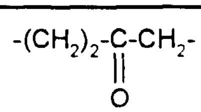 | H | H | $t-C_4H_9$ |
| 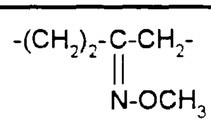 | H | H | $t-C_4H_9$ |

**Tabelle 2 (Fortsetzung)**

| A Q | B | B' | $R^1$ |
|---|---|---|---|
| $-CH_2-$ | H | H | Ph (= Phenyl) |
| $-(CH_2)_2-$ | H | H | Ph |
| 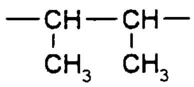 | H | H | Ph |
| $-(CH_2)_2-$ | $CH_3$ | $CH_3$ | Ph |
| $-(CH_2)_3-$ | H | H | Ph |
| $-(CH_2)_2-CHCH_3-$ | H | H | Ph |
| $-(CH_2)_2-CHOCH_3-$ | H | H | Ph |
| $-CH_2-CHCH_3-CH_2-$ | H | H | Ph |
| $-CH_2-CHOCH_3-CH_2-$ | H | H | Ph |
| $-CH_2-CHCH_3-CHCH_3-$ | H | H | Ph |
| $-CH_2-CHOCH_3-CHOCH_3-$ | H | H | Ph |
| $-(CH_2)_4-$ | H | H | Ph |
| $-(CH_2)_3-CHCH_3-$ | H | H | Ph |
| $-(CH_2)_3-CHOCH_3-$ | H | H | Ph |
| $-(CH_2)_2-CHCH_3-CH_2-$ | H | H | Ph |
| $-(CH_2)_2-CHOCH_3-CH_2-$ | H | H | Ph |

**Tabelle 2 (Fortsetzung)**

| A Q | B | B' | $R^1$ |
|---|---|---|---|
| $-(CH_2)_3-CHCH_3-$ | H | H | Ph |
| $-(CH_2)_3-CHOCH_3-$ | H | H | Ph |
| $-(CH_2)_4-$ | $CH_3$ | H | Ph |
| $-CH_2-CHCH_3-CHCH_3-CH_2-$ | H | H | Ph |
| $-(CH_2)_2-CHCH_3-CHCH_3-$ | H | H | Ph |
| $-(CH_2)_2-CHOH-CH_2-$ | H | H | Ph |
| $-CH_2-CHOH-CHOH-CH_2-$ | H | H | Ph |
| 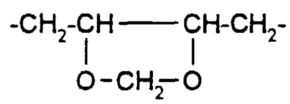 | H | H | Ph |
| 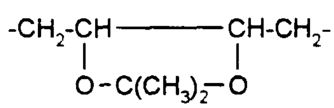 | H | H | Ph |
| $-(CH_2)_4-$ | $-CH_2-$ | | Ph |
| $-(CH_2)_4-$ | $-(CH_2)_2-$ | | Ph |
| 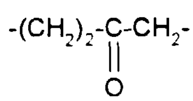 | H | H | Ph |
| 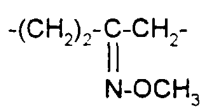 | H | H | Ph |

**Tabelle 3**

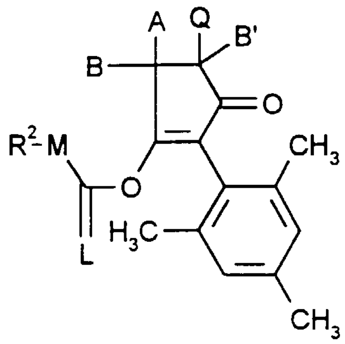

(Ic)

| A Q | B | B' | L | M | $R^2$ |
|---|---|---|---|---|---|
| $-CH_2-$ | H | H | O | O | $CH_3$ |
| $-(CH_2)_2-$ | H | H | O | O | $CH_3$ |
| 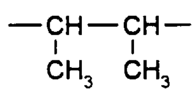 | H | H | O | O | $CH_3$ |
| $-(CH_2)_2-$ | $CH_3$ | $CH_3$ | O | O | $CH_3$ |
| $-(CH_2)_3-$ | H | H | O | O | $CH_3$ |
| $-(CH_2)_2-CHCH_3-$ | H | H | O | O | $CH_3$ |
| $-(CH_2)_2-CHOCH_3-$ | H | H | O | O | $CH_3$ |
| $-CH_2-CHCH_3-CH_2-$ | H | H | O | O | $CH_3$ |
| $-CH_2-CHOCH_3-CH_2-$ | H | H | O | O | $CH_3$ |
| $-CH_2-CHCH_3-CHCH_3-$ | H | H | O | O | $CH_3$ |
| $-CH_2-CHOCH_3-CHOCH_3-$ | H | H | O | O | $CH_3$ |
| $-(CH_2)_4-$ | H | H | O | O | $CH_3$ |
| $-(CH_2)_3-CHCH_3-$ | H | H | O | O | $CH_3$ |
| $-(CH_2)_3-CHOCH_3-$ | H | H | O | O | $CH_3$ |
| $-(CH_2)_2-CHCH_3-CH_2-$ | H | H | O | O | $CH_3$ |
| $-(CH_2)_2-CHOCH_3-CH_2-$ | H | H | O | O | $CH_3$ |

**Tabelle 3 (Fortsetzung)**

| A Q | B | B' | L | M | $R^2$ |
|---|---|---|---|---|---|
| $-(CH_2)_3-CHCH_3-$ | H | H | O | O | $CH_3$ |
| $-(CH_2)_3-CHOCH_3-$ | H | H | O | O | $CH_3$ |
| $-(CH_2)_4-$ | $CH_3$ | H | O | O | $CH_3$ |
| $-CH_2-CHCH_3-CHCH_3-CH_2-$ | H | H | O | O | $CH_3$ |
| $-(CH_2)_2-CHCH_3-CHCH_3-$ | H | H | O | O | $CH_3$ |
| $-(CH_2)_2-CHOH-CH_2-$ | H | H | O | O | $CH_3$ |
| $-CH_2-CHOH-CHOH-CH_2-$ | H | H | O | O | $CH_3$ |
| 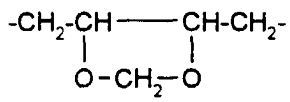 | H | H | O | O | $CH_3$ |
| 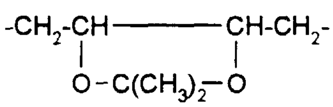 | H | H | O | O | $CH_3$ |
| $-(CH_2)_4-$ | $-CH_2-$ | | O | O | $CH_3$ |
| $-(CH_2)_4-$ | $-(CH_2)_2-$ | | O | O | $CH_3$ |
| 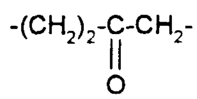 | H | H | O | O | $CH_3$ |
| 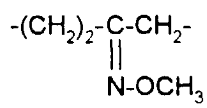 | H | H | O | O | $CH_3$ |

**Tabelle 3 (Fortsetzung)**

| A Q | B | B' | L | M | $-R^2$ |
|---|---|---|---|---|---|
| $-CH_2-$ | H | H | O | O | $i-C_3H_7$ |
| $-(CH_2)_2-$ | H | H | O | O | $i-C_3H_7$ |
| 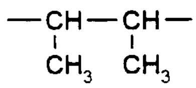 | H | H | O | O | $i-C_3H_7$ |
| $-(CH_2)_2-$ | $CH_3$ | $CH_3$ | O | O | $i-C_3H_7$ |
| $-(CH_2)_3-$ | H | H | O | O | $i-C_3H_7$ |
| $-(CH_2)_2-CHCH_3-$ | H | H | O | O | $i-C_3H_7$ |
| $-(CH_2)_2-CHOCH_3-$ | H | H | O | O | $i-C_3H_7$ |
| $-CH_2-CHCH_3-CH_2-$ | H | H | O | O | $i-C_3H_7$ |
| $-CH_2-CHOCH_3-CH_2-$ | H | H | O | O | $i-C_3H_7$ |
| $-CH_2-CHCH_3-CHCH_3-$ | H | H | O | O | $i-C_3H_7$ |
| $-CH_2-CHOCH_3-CHOCH_3-$ | H | H | O | O | $i-C_3H_7$ |
| $-(CH_2)_4-$ | H | H | O | O | $i-C_3H_7$ |
| $-(CH_2)_3-CHCH_3-$ | H | H | O | O | $i-C_3H_7$ |
| $-(CH_2)_3-CHOCH_3-$ | H | H | O | O | $i-C_3H_7$ |
| $-(CH_2)_2-CHCH_3-CH_2-$ | H | H | O | O | $i-C_3H_7$ |
| $-(CH_2)_2-CHOCH_3-CH_2-$ | H | H | O | O | $i-C_3H_7$ |

**Tabelle 3 (Fortsetzung)**

| A Q | B | B' | L | M | $R^2$ |
|---|---|---|---|---|---|
| $-(CH_2)_3-CHCH_3-$ | H | H | O | O | $i-C_3H_7$ |
| $-(CH_2)_3-CHOCH_3-$ | H | H | O | O | $i-C_3H_7$ |
| $-(CH_2)_4-$ | $CH_3$ | H | O | O | $i-C_3H_7$ |
| $-CH_2-CHCH_3-CHCH_3-CH_2-$ | H | H | O | O | $i-C_3H_7$ |
| $-(CH_2)_2-CHCH_3-CHCH_3-$ | H | H | O | O | $i-C_3H_7$ |
| $-(CH_2)_2-CHOH-CH_2-$ | H | H | O | O | $i-C_3H_7$ |
| $-CH_2-CHOH-CHOH-CH_2-$ | H | H | O | O | $i-C_3H_7$ |
| 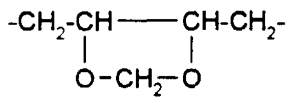 | H | H | O | O | $i-C_3H_7$ |
| 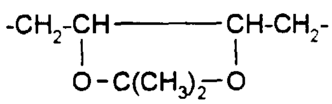 | H | H | O | O | $i-C_3H_7$ |
| $-(CH_2)_4-$ | $-CH_2-$ | | O | O | $i-C_3H_7$ |
| $-(CH_2)_4-$ | $-(CH_2)_2-$ | | O | O | $i-C_3H_7$ |
| 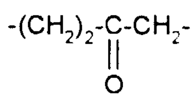 | H | H | O | O | $i-C_3H_7$ |
| 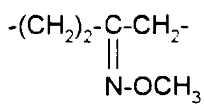 | H | H | O | O | $i-C_3H_7$ |

**Tabelle 3 (Fortsetzung)**

| A Q | B | B' | L | M | $R^2$ |
|---|---|---|---|---|---|
| $-CH_2-$ | H | H | O | O | $i-C_4H_9$ |
| $-(CH_2)_2-$ | H | H | O | O | $i-C_4H_9$ |
| 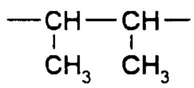 | H | H | O | O | $i-C_4H_9$ |
| $-(CH_2)_2-$ | $CH_3$ | $CH_3$ | O | O | $i-C_4H_9$ |
| $-(CH_2)_3-$ | H | H | O | O | $i-C_4H_9$ |
| $-(CH_2)_2-CHCH_3-$ | H | H | O | O | $i-C_4H_9$ |
| $-(CH_2)_2-CHOCH_3-$ | H | H | O | O | $i-C_4H_9$ |
| $-CH_2-CHCH_3-CH_2-$ | H | H | O | O | $i-C_4H_9$ |
| $-CH_2-CHOCH_3-CH_2-$ | H | H | O | O | $i-C_4H_9$ |
| $-CH_2-CHCH_3-CHCH_3-$ | H | H | O | O | $i-C_4H_9$ |
| $-CH_2-CHOCH_3-CHOCH_3-$ | H | H | O | O | $i-C_4H_9$ |
| $-(CH_2)_4-$ | H | H | O | O | $i-C_4H_9$ |
| $-(CH_2)_3-CHCH_3-$ | H | H | O | O | $i-C_4H_9$ |
| $-(CH_2)_3-CHOCH_3-$ | H | H | O | O | $i-C_4H_9$ |
| $-(CH_2)_2-CHCH_3-CH_2-$ | H | H | O | O | $i-C_4H_9$ |
| $-(CH_2)_2-CHOCH_3-CH_2-$ | H | H | O | O | $i-C_4H_9$ |

**Tabelle 3 (Fortsetzung)**

| A Q | B | B' | L | M | $R^2$ |
|---|---|---|---|---|---|
| $-(CH_2)_3-CHCH_3-$ | H | H | O | O | $i-C_4H_9$ |
| $-(CH_2)_3-CHOCH_3-$ | H | H | O | O | $i-C_4H_9$ |
| $-(CH_2)_4-$ | $CH_3$ | H | O | O | $i-C_4H_9$ |
| $-CH_2-CHCH_3-CHCH_3-CH_2-$ | H | H | O | O | $i-C_4H_9$ |
| $-(CH_2)_2-CHCH_3-CHCH_3-$ | H | H | O | O | $i-C_4H_9$ |
| $-(CH_2)_2-CHOH-CH_2-$ | H | H | O | O | $i-C_4H_9$ |
| $-CH_2-CHOH-CHOH-CH_2-$ | H | H | O | O | $i-C_4H_9$ |
| 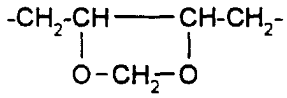 | H | H | O | O | $i-C_4H_9$ |
| 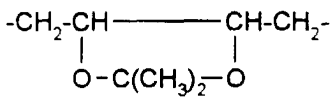 | H | H | O | O | $i-C_4H_9$ |
| $-(CH_2)_4-$ | $-CH_2-$ | | O | O | $i-C_4H_9$ |
| $-(CH_2)_4-$ | $-(CH_2)_2-$ | | O | O | $i-C_4H_9$ |
| 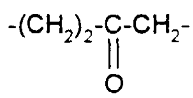 | H | H | O | O | $i-C_4H_9$ |
| 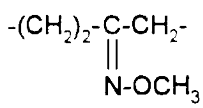 | H | H | O | O | $i-C_4H_9$ |

**Tabelle 3 (Fortsetzung)**

| A Q | B | B' | L | M | $R^2$ |
|---|---|---|---|---|---|
| $-CH_2-$ | H | H | O | O | Ph (=Phenyl) |
| $-(CH_2)_2-$ | H | H | O | O | Ph |
| 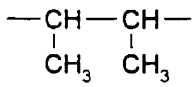 | H | H | O | O | Ph |
| $-(CH_2)_2-$ | $CH_3$ | $CH_3$ | O | O | Ph |
| $-(CH_2)_3-$ | H | H | O | O | Ph |
| $-(CH_2)_2-CHCH_3-$ | H | H | O | O | Ph |
| $-(CH_2)_2-CHOCH_3-$ | H | H | O | O | Ph |
| $-CH_2-CHCH_3-CH_2-$ | H | H | O | O | Ph |
| $-CH_2-CHOCH_3-CH_2-$ | H | H | O | O | Ph |
| $-CH_2-CHCH_3-CHCH_3-$ | H | H | O | O | Ph |
| $-CH_2-CHOCH_3-CHOCH_3-$ | H | H | O | O | Ph |
| $-(CH_2)_4-$ | H | H | O | O | Ph |
| $-(CH_2)_3-CHCH_3-$ | H | H | O | O | Ph |
| $-(CH_2)_3-CHOCH_3-$ | H | H | O | O | Ph |
| $-(CH_2)_2-CHCH_3-CH_2-$ | H | H | O | O | Ph |
| $-(CH_2)_2-CHOCH_3-CH_2-$ | H | H | O | O | Ph |

**Tabelle 3 (Fortsetzung)**

| A Q | B | B' | L | M | $R^2$ |
|---|---|---|---|---|---|
| $-(CH_2)_3-CHCH_3-$ | H | H | O | O | Ph |
| $-(CH_2)_3-CHOCH_3-$ | H | H | O | O | Ph |
| $-(CH_2)_4-$ | $CH_3$ | H | O | O | Ph |
| $-CH_2-CHCH_3-CHCH_3-CH_2-$ | H | H | O | O | Ph |
| $-(CH_2)_2-CHCH_3-CHCH_3-$ | H | H | O | O | Ph |
| $-(CH_2)_2-CHOH-CH_2-$ | H | H | O | O | Ph |
| $-CH_2-CHOH-CHOH-CH_2-$ | H | H | O | O | Ph |
| 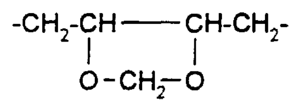 | H | H | O | O | Ph |
| 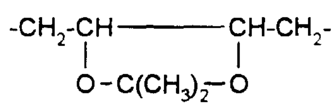 | H | H | O | O | Ph |
| $-(CH_2)_4-$ | $-CH_2-$ | | O | O | Ph |
| $-(CH_2)_4-$ | $-(CH_2)_2-$ | | O | O | Ph |
| 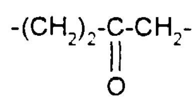 | H | H | O | O | Ph |
| 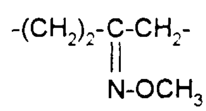 | H | H | O | O | Ph |

**Tabelle 3 (Fortsetzung)**

| A Q | B | B' | L | M | $R^2$ |
|---|---|---|---|---|---|
| $-CH_2-$ | H | H | O | O | Bz (=Benzyl) |
| $-(CH_2)_2-$ | H | H | O | O | Bz |
| 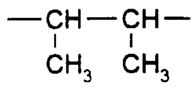 | H | H | O | O | Bz |
| $-(CH_2)_2-$ | $CH_3$ | $CH_3$ | O | O | Bz |
| $-(CH_2)_3-$ | H | H | O | O | Bz |
| $-(CH_2)_2-CHCH_3-$ | H | H | O | O | Bz |
| $-(CH_2)_2-CHOCH_3-$ | H | H | O | O | Bz |
| $-CH_2-CHCH_3-CH_2-$ | H | H | O | O | Bz |
| $-CH_2-CHOCH_3-CH_2-$ | H | H | O | O | Bz |
| $-CH_2-CHCH_3-CHCH_3-$ | H | H | O | O | Bz |
| $-CH_2-CHOCH_3-CHOCH_3-$ | H | H | O | O | Bz |
| $-(CH_2)_4-$ | H | H | O | O | Bz |
| $-(CH_2)_3-CHCH_3-$ | H | H | O | O | Bz |
| $-(CH_2)_3-CHOCH_3-$ | H | H | O | O | Bz |
| $-(CH_2)_2-CHCH_3-CH_2-$ | H | H | O | O | Bz |
| $-(CH_2)_2-CHOCH_3-CH_2-$ | H | H | O | O | Bz |

**Tabelle 3 (Fortsetzung)**

| A Q | B | B' | L | M | $R^2$ |
|---|---|---|---|---|---|
| $-(CH_2)_3-CHCH_3-$ | H | H | O | O | Bz |
| $-(CH_2)_3-CHOCH_3-$ | H | H | O | O | Bz |
| $-(CH_2)_4-$ | $CH_3$ | H | O | O | Bz |
| $-CH_2-CHCH_3-CHCH_3-CH_2-$ | H | H | O | O | Bz |
| $-(CH_2)_2-CHCH_3-CHCH_3-$ | H | H | O | O | Bz |
| $-(CH_2)_2-CHOH-CH_2-$ | H | H | O | O | Bz |
| $-CH_2-CHOH-CHOH-CH_2-$ | H | H | O | O | Bz |
| 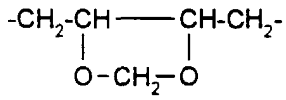 | H | H | O | O | Bz |
| 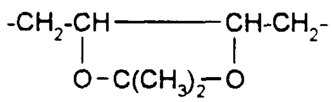 | H | H | O | O | Bz |
| $-(CH_2)_4-$ | $-CH_2-$ | | O | O | Bz |
| $-(CH_2)_4-$ | $-(CH_2)_2-$ | | O | O | Bz |
| 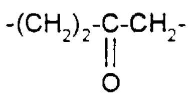 | H | H | O | O | Bz |
| 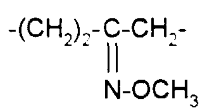 | H | H | O | O | Bz |

**Tabelle 3 (Fortsetzung)**

| A Q | B | B' | L | M | $R^2$ |
|---|---|---|---|---|---|
| $-CH_2-$ | H | H | O | S | $i-C_3H_7$ |
| $-(CH_2)_2-$ | H | H | O | S | $i-C_3H_7$ |
| 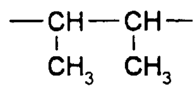 | H | H | O | S | $i-C_3H_7$ |
| $-(CH_2)_2-$ | $CH_3$ | $CH_3$ | O | S | $i-C_3H_7$ |
| $-(CH_2)_3-$ | H | H | O | S | $i-C_3H_7$ |
| $-(CH_2)_2-CHCH_3-$ | H | H | O | S | $i-C_3H_7$ |
| $-(CH_2)_2-CHOCH_3-$ | H | H | O | S | $i-C_3H_7$ |
| $-CH_2-CHCH_3-CH_2-$ | H | H | O | S | $i-C_3H_7$ |
| $-CH_2-CHOCH_3-CH_2-$ | H | H | O | S | $i-C_3H_7$ |
| $-CH_2-CHCH_3-CHCH_3-$ | H | H | O | S | $i-C_3H_7$ |
| $-CH_2-CHOCH_3-CHOCH_3-$ | H | H | O | S | $i-C_3H_7$ |
| $-(CH_2)_4-$ | H | H | O | S | $i-C_3H_7$ |
| $-(CH_2)_3-CHCH_3-$ | H | H | O | S | $i-C_3H_7$ |
| $-(CH_2)_3-CHOCH_3-$ | H | H | O | S | $i-C_3H_7$ |
| $-(CH_2)_2-CHCH_3-CH_2-$ | H | H | O | S | $i-C_3H_7$ |
| $-(CH_2)_2-CHOCH_3-CH_2-$ | H | H | O | S | $i-C_3H_7$ |

**Tabelle 3 (Fortsetzung)**

| A Q | B | B' | L | M | $R^2$ |
|---|---|---|---|---|---|
| $-(CH_2)_3-CHCH_3-$ | H | H | O | S | $i-C_3H_7$ |
| $-(CH_2)_3-CHOCH_3-$ | H | H | O | S | $i-C_3H_7$ |
| $-(CH_2)_4-$ | $CH_3$ | H | O | S | $i-C_3H_7$ |
| $-CH_2-CHCH_3-CHCH_3-CH_2-$ | H | H | O | S | $i-C_3H_7$ |
| $-(CH_2)_2-CHCH_3-CHCH_3-$ | H | H | O | S | $i-C_3H_7$ |
| $-(CH_2)_2-CHOH-CH_2-$ | H | H | O | S | $i-C_3H_7$ |
| $-CH_2-CHOH-CHOH-CH_2-$ | H | H | O | S | $i-C_3H_7$ |
| 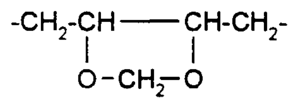 | H | H | O | S | $i-C_3H_7$ |
| 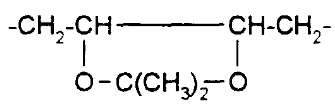 | H | H | O | S | $i-C_3H_7$ |
| $-(CH_2)_4-$ | $-CH_2-$ | | O | S | $i-C_3H_7$ |
| $-(CH_2)_4-$ | $-(CH_2)_2-$ | | O | S | $i-C_3H_7$ |
| 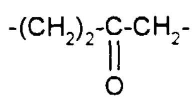 | H | H | O | S | $i-C_3H_7$ |
| 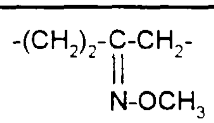 | H | H | O | S | $i-C_3H_7$ |

**Tabelle 3 (Fortsetzung)**

| A Q | B | B' | L | M | $R^2$ |
|---|---|---|---|---|---|
| $-CH_2-$ | H | H | O | S | $t-C_4H_9$ |
| $-(CH_2)_2-$ | H | H | O | S | $t-C_4H_9$ |
| 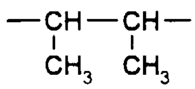 | H | H | O | S | $t-C_4H_9$ |
| $-(CH_2)_2-$ | $CH_3$ | $CH_3$ | O | S | $t-C_4H_9$ |
| $-(CH_2)_3-$ | H | H | O | S | $t-C_4H_9$ |
| $-(CH_2)_2-CHCH_3-$ | H | H | O | S | $t-C_4H_9$ |
| $-(CH_2)_2-CHOCH_3-$ | H | H | O | S | $t-C_4H_9$ |
| $-CH_2-CHCH_3-CH_2-$ | H | H | O | S | $t-C_4H_9$ |
| $-CH_2-CHOCH_3-CH_2-$ | H | H | O | S | $t-C_4H_9$ |
| $-CH_2-CHCH_3-CHCH_3-$ | H | H | O | S | $t-C_4H_9$ |
| $-CH_2-CHOCH_3-CHOCH_3-$ | H | H | O | S | $t-C_4H_9$ |
| $-(CH_2)_4-$ | H | H | O | S | $t-C_4H_9$ |
| $-(CH_2)_3-CHCH_3-$ | H | H | O | S | $t-C_4H_9$ |
| $-(CH_2)_3-CHOCH_3-$ | H | H | O | S | $t-C_4H_9$ |
| $-(CH_2)_2-CHCH_3-CH_2-$ | H | H | O | S | $t-C_4H_9$ |
| $-(CH_2)_2-CHOCH_3-CH_2-$ | H | H | O | S | $t-C_4H_9$ |

**Tabelle 3 (Fortsetzung)**

| A Q | B | B' | L | M | $R^2$ |
|---|---|---|---|---|---|
| $-(CH_2)_3-CHCH_3-$ | H | H | O | S | $t-C_4H_9$ |
| $-(CH_2)_3-CHOCH_3-$ | H | H | O | S | $t-C_4H_9$ |
| $-(CH_2)_4-$ | $CH_3$ | H | O | S | $t-C_4H_9$ |
| $-CH_2-CHCH_3-CHCH_3-CH_2-$ | H | H | O | S | $t-C_4H_9$ |
| $-(CH_2)_2-CHCH_3-CHCH_3-$ | H | H | O | S | $t-C_4H_9$ |
| $-(CH_2)_2-CHOH-CH_2-$ | H | H | O | S | $t-C_4H_9$ |
| $-CH_2-CHOH-CHOH-CH_2-$ | H | H | O | S | $t-C_4H_9$ |
| 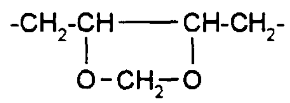 | H | H | O | S | $t-C_4H_9$ |
| 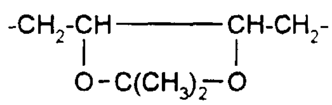 | H | H | O | S | $t-C_4H_9$ |
| $-(CH_2)_4-$ | $-CH_2-$ | | O | S | $t-C_4H_9$ |
| $-(CH_2)_4-$ | $-(CH_2)_2-$ | | O | S | $t-C_4H_9$ |
| 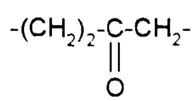 | H | H | O | S | $t-C_4H_9$ |
| 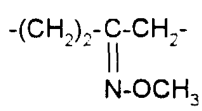 | H | H | O | S | $t-C_4H_9$ |

**Tabelle 4**

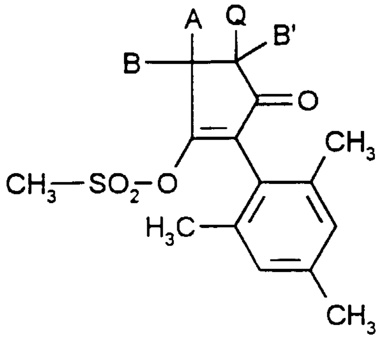

(Id)

| A Q | B | B' |
|---|---|---|
| $-CH_2-$ | H | H |
| $-(CH_2)_2-$ | H | H |
| 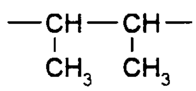 | H | H |
| $-(CH_2)_2-$ | $CH_3$ | $CH_3$ |
| $-(CH_2)_3-$ | H | H |
| $-(CH_2)_2-CHCH_3-$ | H | H |
| $-(CH_2)_2-CHOCH_3-$ | H | H |
| $-CH_2-CHCH_3-CH_2-$ | H | H |
| $-CH_2-CHOCH_3-CH_2-$ | H | H |
| $-CH_2-CHCH_3-CHCH_3-$ | H | H |
| $-CH_2-CHOCH_3-CHOCH_3-$ | H | H |
| $-(CH_2)_4-$ | H | H |
| $-(CH_2)_3-CHCH_3-$ | H | H |
| $-(CH_2)_3-CHOCH_3-$ | H | H |
| $-(CH_2)_2-CHCH_3-CH_2-$ | H | H |
| $-(CH_2)_2-CHOCH_3-CH_2-$ | H | H |

**Tabelle 4 (Fortsetzung)**

| A Q | B | B' |
|---|---|---|
| $-(CH_2)_3-CHCH_3-$ | H | H |
| $-(CH_2)_3-CHOCH_3-$ | H | H |
| $-(CH_2)_4-$ | $CH_3$ | H |
| $-CH_2-CHCH_3-CHCH_3-CH_2-$ | H | H |
| $-(CH_2)_2-CHCH_3-CHCH_3-$ | H | H |
| $-(CH_2)_2-CHOH-CH_2-$ | H | H |
| $-CH_2-CHOH-CHOH-CH_2-$ | H | H |
| 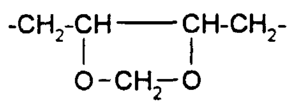 | H | H |
| 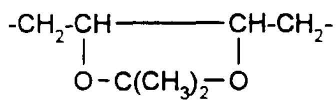 | H | H |
| $-(CH_2)_4-$ | $-CH_2-$ | |
| $-(CH_2)_4-$ | $-(CH_2)_2-$ | |
| 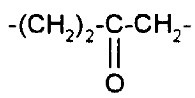 | H | H |
| 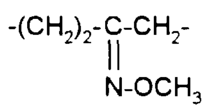 | H | H |

**Tabelle 5**

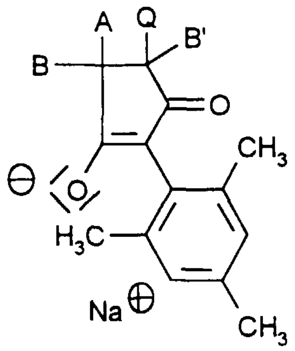

(If)

| A Q | B | B' |
|---|---|---|
| $-CH_2-$ | H | H |
| $-(CH_2)_2-$ | H | H |
| 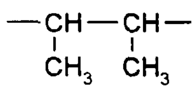 | H | H |
| $-(CH_2)_2-$ | $CH_3$ | $CH_3$ |
| $-(CH_2)_3-$ | H | H |
| $-(CH_2)_2-CHCH_3-$ | H | H |
| $-(CH_2)_2-CHOCH_3-$ | H | H |
| $-CH_2-CHCH_3-CH_2-$ | H | H |
| $-CH_2-CHOCH_3-CH_2-$ | H | H |
| $-CH_2-CHCH_3-CHCH_3-$ | H | H |
| $-CH_2-CHOCH_3-CHOCH_3-$ | H | H |
| $-(CH_2)_4-$ | H | H |
| $-(CH_2)_3-CHCH_3-$ | H | H |
| $-(CH_2)_3-CHOCH_3-$ | H | H |
| $-(CH_2)_2-CHCH_3-CH_2-$ | H | H |
| $-(CH_2)_2-CHOCH_3-CH_2-$ | H | H |

**Tabelle 5 (Fortsetzung)**

| A Q | B | B' |
|---|---|---|
| $-(CH_2)_3-CHCH_3-$ | H | H |
| $-(CH_2)_3-CHOCH_3-$ | H | H |
| $-(CH_2)_4-$ | $CH_3$ | H |
| $-CH_2-CHCH_3-CHCH_3-CH_2-$ | H | H |
| $-(CH_2)_2-CHCH_3-CHCH_3-$ | H | H |
| $-(CH_2)_2-CHOH-CH_2-$ | H | H |
| $-CH_2-CHOH-CHOH-CH_2-$ | H | H |
| 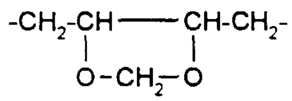 | H | H |
| 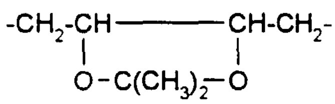 | H | H |
| $-(CH_2)_4-$ | $-CH_2-$ | |
| $-(CH_2)_4-$ | $-(CH_2)_2-$ | |
| 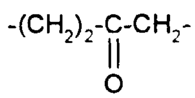 | H | H |
| 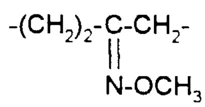 | H | H |

**Tabelle 5**

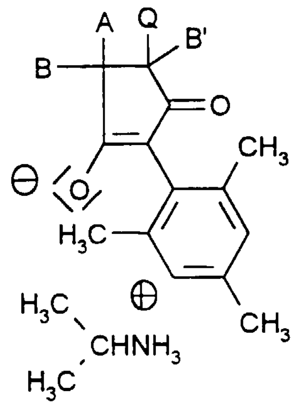

(Ia)

| A Q | B | B' |
|---|---|---|
| $-CH_2-$ | H | H |
| $-(CH_2)_2-$ | H | H |
| 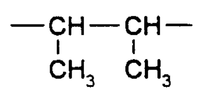 | H | H |
| $-(CH_2)_2-$ | $CH_3$ | $CH_3$ |
| $-(CH_2)_3-$ | H | H |
| $-(CH_2)_2-CHCH_3-$ | H | H |
| $-(CH_2)_2-CHOCH_3-$ | H | H |
| $-CH_2-CHCH_3-CH_2-$ | H | H |
| $-CH_2-CHOCH_3-CH_2-$ | H | H |
| $-CH_2-CHCH_3-CHCH_3-$ | H | H |
| $-CH_2-CHOCH_3-CHOCH_3-$ | H | H |
| $-(CH_2)_4-$ | H | H |
| $-(CH_2)_3-CHCH_3-$ | H | H |
| $-(CH_2)_3-CHOCH_3-$ | H | H |
| $-(CH_2)_2-CHCH_3-CH_2-$ | H | H |
| $-(CH_2)_2-CHOCH_3-CH_2-$ | H | H |

**Tabelle 5 (Fortsetzung)**

| A Q | B | B' |
|---|---|---|
| $-(CH_2)_3-CHCH_3-$ | H | H |
| $-(CH_2)_3-CHOCH_3-$ | H | H |
| $-(CH_2)_4-$ | $CH_3$ | H |
| $-CH_2-CHCH_3-CHCH_3-CH_2-$ | H | H |
| $-(CH_2)_2-CHCH_3-CHCH_3-$ | H | H |
| $-(CH_2)_2-CHOH-CH_2-$ | H | H |
| $-CH_2-CHOH-CHOH-CH_2-$ | H | H |
| 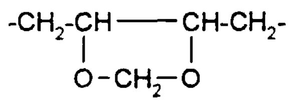 | H | H |
| 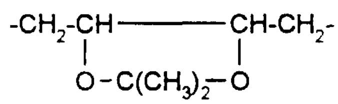 | H | H |
| $-(CH_2)_4-$ | $-CH_2-$ | |
| $-(CH_2)_4-$ | $-(CH_2)_2-$ | |
| 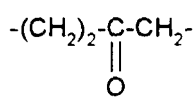 | H | H |
| 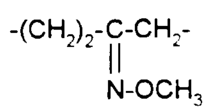 | H | H |

**Tabelle 6**

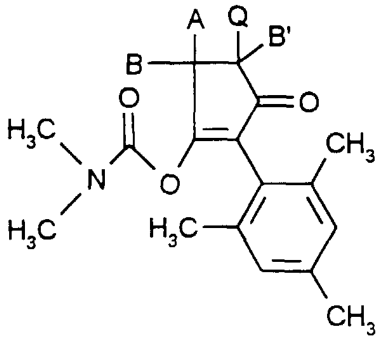

(Ig)

| A Q | B | B' |
|---|---|---|
| $-CH_2-$ | H | H |
| $-(CH_2)_2-$ | H | H |
| 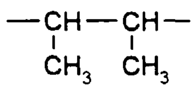 | H | H |
| $-(CH_2)_2-$ | $CH_3$ | $CH_3$ |
| $-(CH_2)_3-$ | H | H |
| $-(CH_2)_2-CHCH_3-$ | H | H |
| $-(CH_2)_2-CHOCH_3-$ | H | H |
| $-CH_2-CHCH_3-CH_2-$ | H | H |
| $-CH_2-CHOCH_3-CH_2-$ | H | H |
| $-CH_2-CHCH_3-CHCH_3-$ | H | H |
| $-CH_2-CHOCH_3-CHOCH_3-$ | H | H |
| $-(CH_2)_4-$ | H | H |
| $-(CH_2)_3-CHCH_3-$ | H | H |
| $-(CH_2)_3-CHOCH_3-$ | H | H |
| $-(CH_2)_2-CHCH_3-CH_2-$ | H | H |
| $-(CH_2)_2-CHOCH_3-CH_2-$ | H | H |

**Tabelle 6 (Fortsetzung)**

| A Q | B | B' |
|---|---|---|
| $-(CH_2)_3-CHCH_3-$ | H | H |
| $-(CH_2)_3-CHOCH_3-$ | H | H |
| $-(CH_2)_4-$ | $CH_3$ | H |
| $-CH_2-CHCH_3-CHCH_3-CH_2-$ | H | H |
| $-(CH_2)_2-CHCH_3-CHCH_3-$ | H | H |
| $-(CH_2)_2-CHOH-CH_2-$ | H | H |
| $-CH_2-CHOH-CHOH-CH_2-$ | H | H |
| 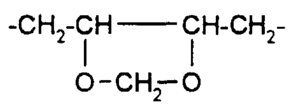 | H | H |
| 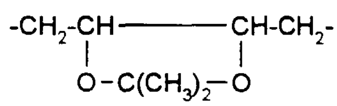 | H | H |
| $-(CH_2)_4-$ | $-CH_2-$ | |
| $-(CH_2)_4-$ | $-(CH_2)_2-$ | |
| 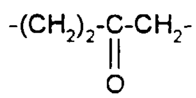 | H | H |
| 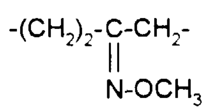 | H | H |

**Tabelle 6**

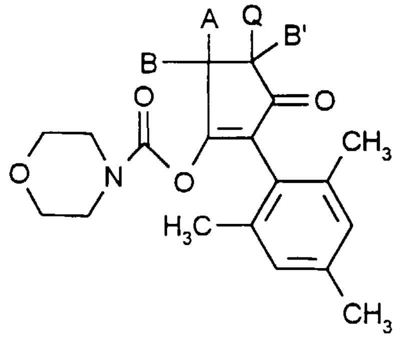

(Ig)

| A Q | B | B' |
|---|---|---|
| $-CH_2-$ | H | H |
| $-(CH_2)_2-$ | H | H |
| 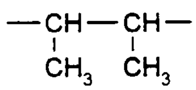 | H | H |
| $-(CH_2)_2-$ | $CH_3$ | $CH_3$ |
| $-(CH_2)_3-$ | H | H |
| $-(CH_2)_2-CHCH_3-$ | H | H |
| $-(CH_2)_2-CHOCH_3-$ | H | H |
| $-CH_2-CHCH_3-CH_2-$ | H | H |
| $-CH_2-CHOCH_3-CH_2-$ | H | H |
| $-CH_2-CHCH_3-CHCH_3-$ | H | H |
| $-CH_2-CHOCH_3-CHOCH_3-$ | H | H |
| $-(CH_2)_4-$ | H | H |
| $-(CH_2)_3-CHCH_3-$ | H | H |
| $-(CH_2)_3-CHOCH_3-$ | H | H |
| $-(CH_2)_2-CHCH_3-CH_2-$ | H | H |
| $-(CH_2)_2-CHOCH_3-CH_2-$ | H | H |

**Tabelle 6 (Fortsetzung)**

| A Q | B | B' |
|---|---|---|
| $-(CH_2)_3-CHCH_3-$ | H | H |
| $-(CH_2)_3-CHOCH_3-$ | H | H |
| $-(CH_2)_4-$ | $CH_3$ | H |
| $-CH_2-CHCH_3-CHCH_3-CH_2-$ | H | H |
| $-(CH_2)_2-CHCH_3-CHCH_3-$ | H | H |
| $-(CH_2)_2-CHOH-CH_2-$ | H | H |
| $-CH_2-CHOH-CHOH-CH_2-$ | H | H |
| 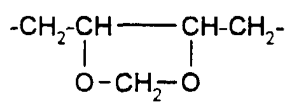 | H | H |
| 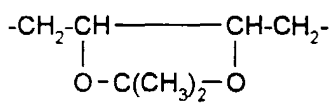 | H | H |
| $-(CH_2)_4-$ | $-CH_2-$ | |
| $-(CH_2)_4-$ | $-(CH_2)_2-$ | |
| 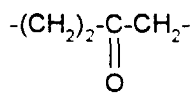 | H | H |
| 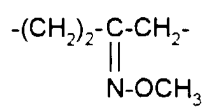 | H | H |

Verwendet man gemäß Verfahren (A) 5-(2,4,6-Trimethylphenyl)-2,3-tetramethylen-4-oxo-valeriansäureethylester, so kann der Verlauf des erfindungsgemäßen Verfahrens durch folgendes Reaktionsschema wiedergegeben werden:

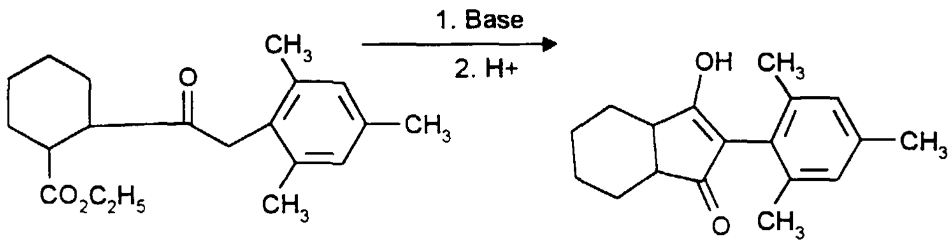


Verwendet man gemäß Verfahren (B) (Variante α) 2-(2,4,6-Trimethylphenyl)-4,5-(2,3-dimethyl)-tetramethylen-3-hydroxy-2-cyclopenten-1-on und Pivaloylchlorid als Ausgangsstoffe, so kann der Verlauf des erfindungsgemäßen Verfahrens durch folgendes Reaktionsschema wiedergegeben werden:

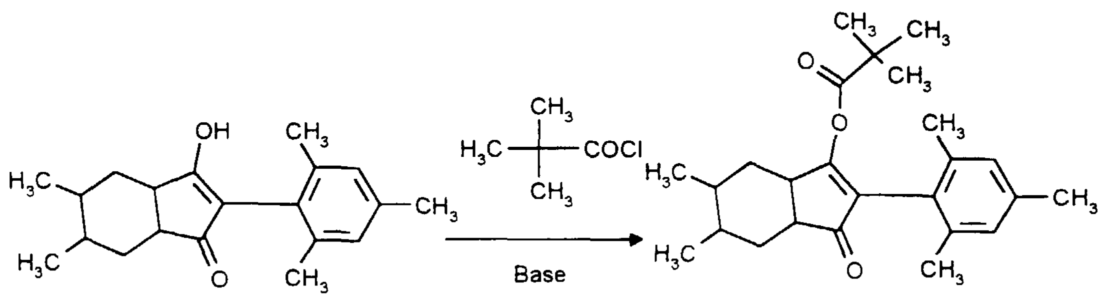


Verwendet man gemäß Verfahren B (Variante β) 2-(2,4,6-Trimethylphenyl)-4,5-methylen-3-hydroxy-2-cyclopenten-1-on und Acetanhydrid als Ausgangsverbindungen, so kann der Verlauf des erfindungsgemäßen Verfahrens durch folgendes Reaktionsschema wiedergegeben werden:

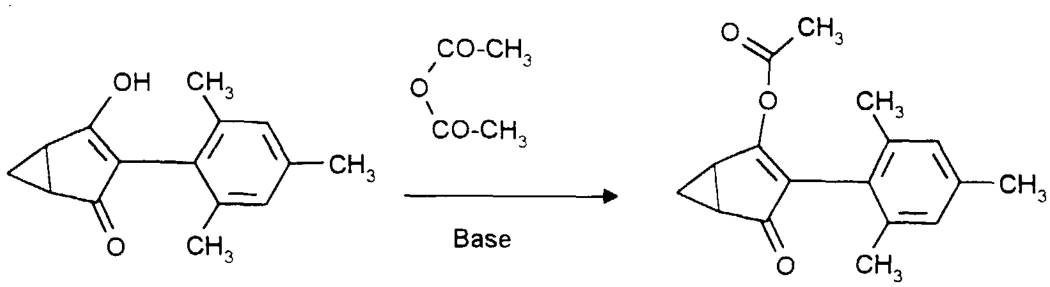


Verwendet man gemäß Verfahren (C) 2-(2,4,6-Trimethylphenyl)-4,5-(3-oxo)-tetramethylen-3-hydroxy-2-cyclopenten-1 -on und Chlorameisensäureethoxyethylester als Ausgangsverbindungen, so kann der Verlauf des erfindungsgemäßen Verfahrens durch folgendes Reaktionsschema wiedergegeben werden.

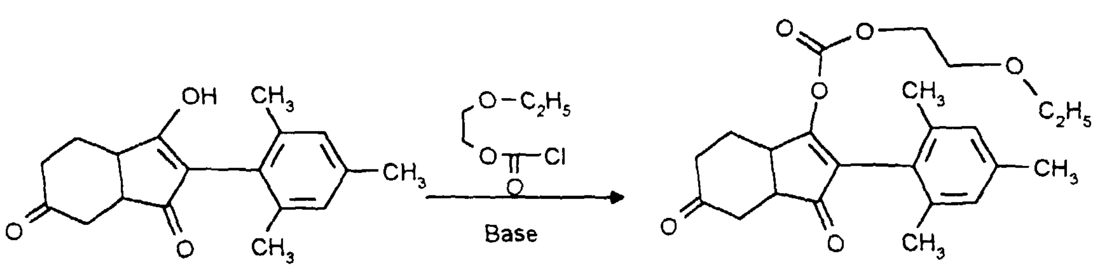


Verwendet man gemäß Verfahren ($D_{\alpha}$) 2-(2,4,6-Trimethylphenyl)-4,5-(3-methyl)-tetramethylen-3-hydroxy-2-cyclopenten-1-on und Chlormonothioameisensäuremethylester als Ausgangsprodukte, so kann der Reaktionsverlauf wie folgt wiedergegeben werden:

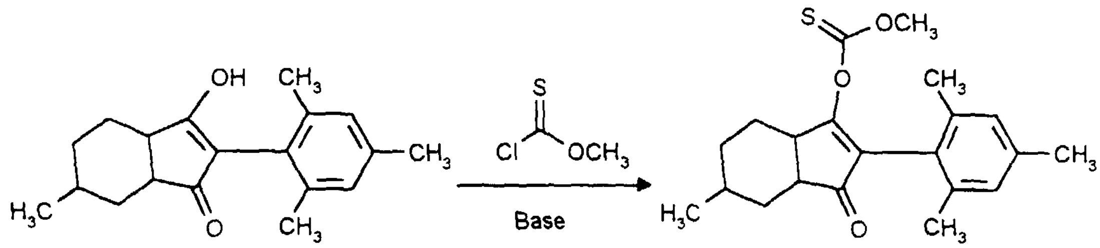


Verwendet man gemäß Verfahren ($D_\beta$) 2-(2,4,6-Trimethylphenyl)-4,5-trimethylen-3-hydroxy-2-cyclopenten-1-on, Schwefelkohlenstoff und Methyliodid als Ausgangskomponenten, so kann der Reaktionsverlauf wie folgt wiedergegeben werden:

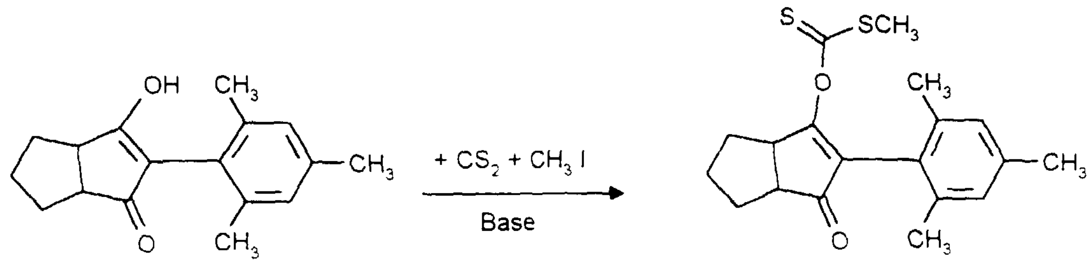


Verwendet man gemäß Verfahren (E) 2-(2,4,6-Trimethylphenyl)-4,5-(3-methoxy)-tetramethyl en-3 -hydroxy-2-cyclopenten-1-on und Methansulfonsäurechlorid als Ausgangsprodukt, so kann der Reaktionsverlauf durch folgendes Reaktionsschema wiedergegeben werden:

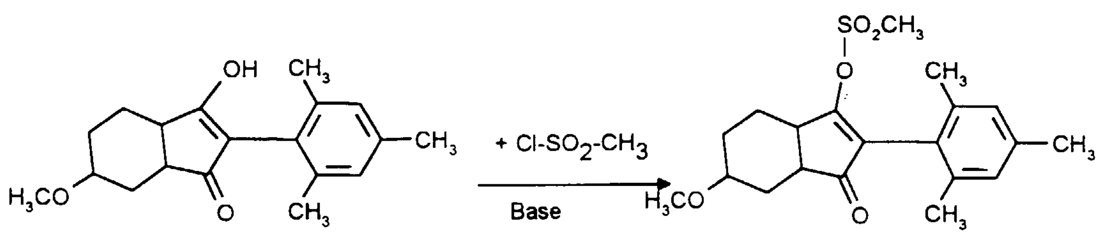


Verwendet man gemäß Verfahren (F) 2-(2,4,6-Trimethylphenyl)-4,5-(4-methyl)-tetramethylen-3-hydroxy-2-cyclopenten-1-on und Methanthio-phosphonsäurechlorid-(2,2,2-trifluorethylester) als Ausgangsprodukte, so kann der Reaktionsverlauf durch folgendes Reaktionsschema wiedergegeben werden:

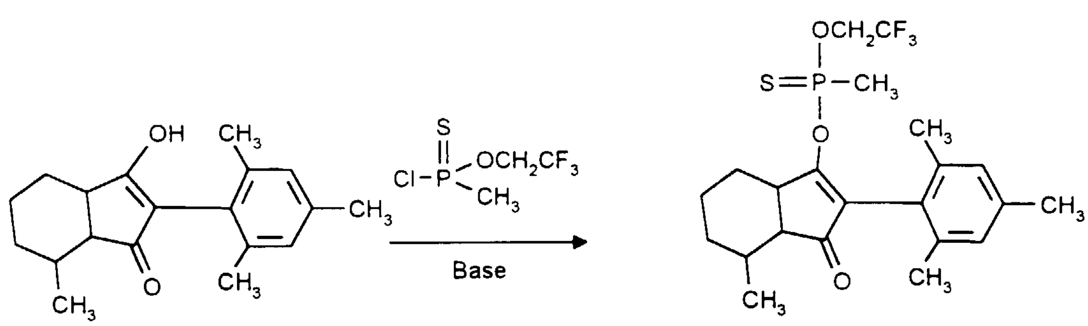


Verwendet man gemäß Verfahren (G) 2-(2,4,6-Trimethylphenyl)-4,5-(3,3-ethylendioxy)-tetramethylen-3-hydroxy-2-cyclopenten-1-on und NaOH als Komponenten, so kann der Verlauf des erfindungsgemäßen Verfahrens durch

folgendes Reaktionsschema wiedergegeben werden:

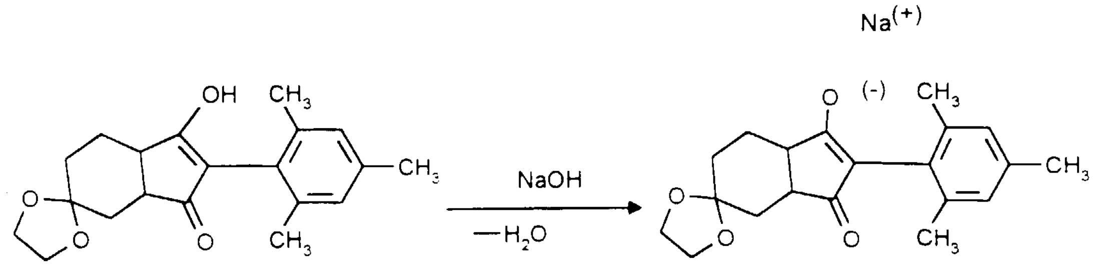


Verwendet man gemäß Verfahren ($H_\alpha$) 2-(2,4,6-Trimethylphenyl)-4,5-(3-methoxy)-tetramethylen-3-hydroxy-2-cyclopenten-1-on und Ethylisocyanat als Ausgangsprodukte, so kann der Reaktionsverlauf durch folgendes Schema wiedergegeben werden:

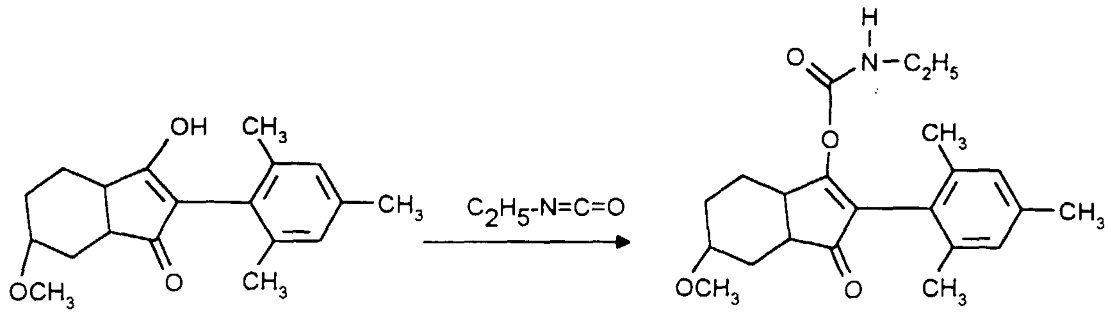


Verwendet man gemäß Verfahren ($H_\beta$) 2-(2,4,6-Trimethylphenyl)-4,5-tertramethylen-3-hydroxy-2-cyclopenten-1-on und Dimethylcarbamidsäurechlorid als Ausgangsprodukte, so kann der Reaktionsverlauf durch folgendes Schema wiedergegeben werden:

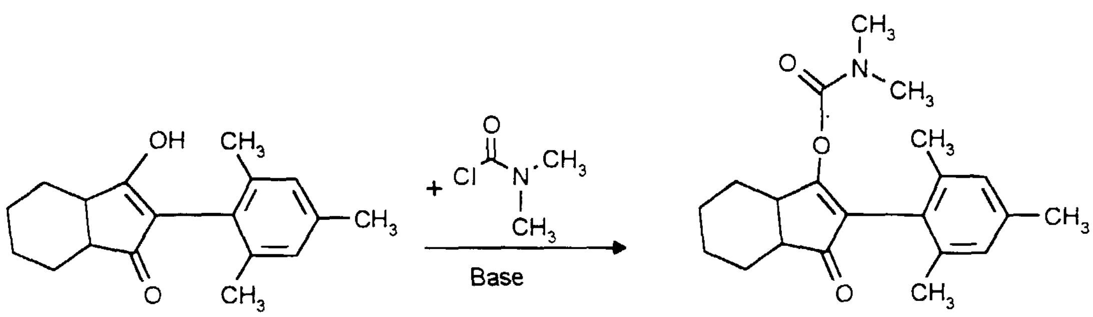


Die bei dem obigen Verfahren (A) als Ausgangsstoffe benötigten Verbindungen der Formel (II)

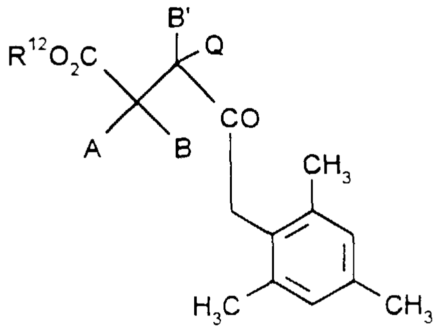

(II)

in welcher
A, B, B', Q und $R^{12}$ die oben angegebene Bedeutung haben,
sind neu. Sie lassen sich nach im Pnnzip bekannten Methoden herstellen. Man erhält die 5-Aryl-4-ketocarbonsäureester der Formel (II) beispielsweise, wenn man 5-Aryl-4-ketocarbonsäuren der Formel (XIV)

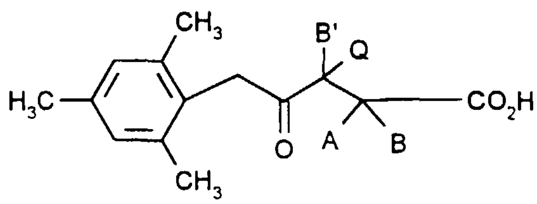

(XIV)

in welcher
A, B, B' und Q die oben angegebene Bedeutung haben,
verestert, (vgl. z.B. Organikum, 15. Auflage, Berlin, 1977, Seite 499).
Die 5-Aryl-4-ketocarbonsäuren der Formel (XIV)

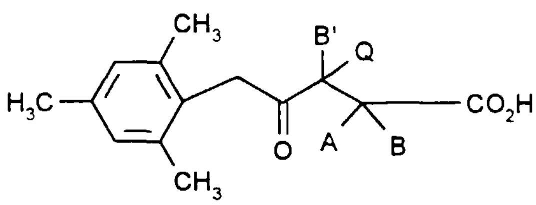

(XIV)

in welcher
A, B, B' und Q die oben angegebene Bedeutung haben,
sind neu, lassen sich aber nach im Prinzip bekannten Methoden herstellen.

Man erhält die 5-Aryl-4-ketocarbonsäuren der Formel (XIV) beispielsweise, wenn man Carbonsäureanhydride der Formel (XV)

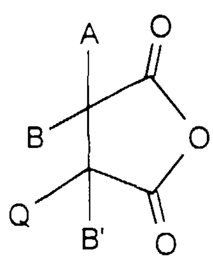

(XV)

in welcher
A, B, B' und Q die oben angegebene Bedeutung haben,
mit metallorganischen Verbindungen der Formel (XVI)

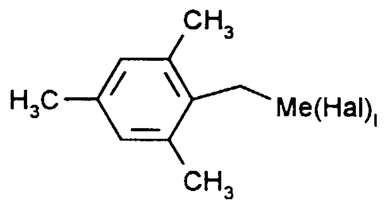

(XVI)

in welcher

Me für ein- oder zweiwertige Metallionen (beispielsweise des Lithiums oder Magnesiums),
Hal für Chlor oder Brom

und

l für eine Zahl 0 oder 1 steht,

in Gegenwart eines Verdünnungsmittels umsetzt (vgl. z.B. Organikum, 15. Auflage, Berlin, 1977, Seite 623).

Die Verbindungen (XV) und (XVI) sind teilweise bekannt und/oder lassen sich nach bekannten Verfahren in einfacher Weise darstellen (vgl. z.B. Organikum, 15. Auflage, Berlin, 1977, Seiten 525, 526 und 623).

Weiterhin erhält man 5-Aryl-4-ketocarbonsäuren der Formel (XIV)

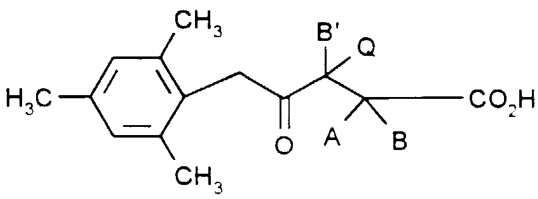

(XIV)

in welcher
A, B, B' und Q die oben angegebene Bedeutung haben,
wenn man 2-Mesityl-3-oxo-adipinsäureester der Formel (XVII)

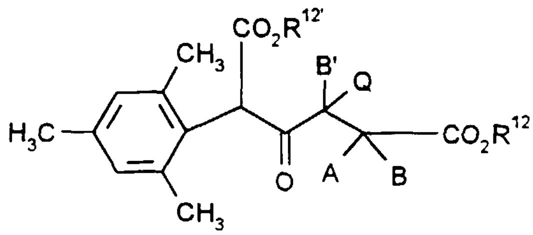

(XVII)

in welcher
A, B, B' und Q die oben angegebene Bedeutung haben und
$R^{12}$ und $R^{12'}$ für Alkyl (bevorzugt $C_1$-$C_6$-Alkyl), stehen,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XVII)

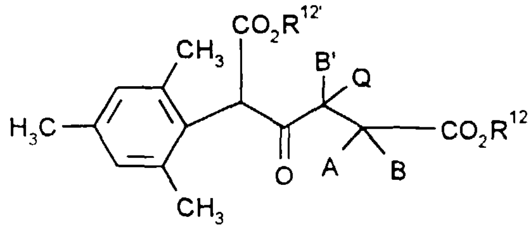

(XVII)

in welcher
A, B, B', Q, $R^{12}$ und $R^{12'}$ die oben angegebene Bedeutung haben,
sind neu und erhältlich,
wenn man Dicarbonsäurehalbesterchloride der Formel (XVIII),

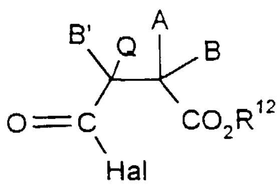

(XVIII)

in welcher
A, B, B', Q und $R^{12}$ die oben angegebene Bedeutung haben und
Hal für Chlor oder Brom steht,
mit einem substituierten Phenylessigsäureester der Formel (XIX)

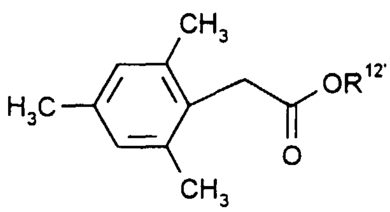

(XIX)

in welcher

$R^{12'}$ für Alkyl steht,

in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228).

Die Verbindungen der Formel XVIII und XIX sind teilweise bekannt und/oder lassen sich nach bekannten Verfahren darstellen.

Das Verfahren (A) ist dadurch gekennzeichnet, daß man Verbindungen der Formel (II), in welcher A, B, B', Q und $R^{12}$ die oben angegebene Bedeutung haben, in Gegenwart von Basen einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (A) alle üblichen Protonenakzeptoren eingesetzt werden.

Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl($C_8$-$C_{10}$)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecan (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Bei dem erfindungsgemäßen Verfahren (Bβ) können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethylanilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat.

Als Verdünnungsmittel können bei dem erfindungsgemäßen Verfahren (C) alle gegenüber den Ausgangsstoffen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Methyl-tert.-butylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (Dα) setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten organischen Lösungsmittel in Frage, wie halogenierte Kohlenwasserstoffe, Ether, Amide, Alkohole, Nitrile, Sulfone und Sulfoxide.

Vorzugsweise werden Acetonitril, Dimethylsulfoxid, Methyl-tert.-butylether, Tetrahydrofuran, Dimethylformamid, Ethylacetat oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Dβ) setzt man pro Mol Ausgangsverbindung der Formel (II) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Als Basen können beim Verfahren (Dβ) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise verwendbar sind aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Oft ist es zweckmäßig, zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung der Formel (Ia) so lange mit Schwefelkohlenstoff um bis die Bildung der Zwischenverbindung abgeschlossen ist, z.B. nach mehrstündigem Rühren bei Raumtemperatur.

Die weitere Umsetzung mit dem Alkylhalogenid der Formel (VII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (E) setzt man pro Mol Ausgangsverbindung der Formel (Ia) ca. 1 Mol Sulfonsäurechlorid der Formel (VIII) bei 0 bis 150°C, vorzugsweise bei 20 bis 70°C um.

Als gegebenenfalls zugesetzte Verdünnungsmittel kommen alle inerten organischen Lösungsmittel in Frage wie halogenierte Kohlenwasserstoffe, Ether, Amide, Carbonsäureester, Nitrile, Sulfone oder Sulfoxide.

Vorzugsweise werden Acetonitril, Dimethylsulfoxid, Ethylacetat, Methyl-tert.-butylether, Tetrahydrofuran, Dimethylformamid oder Methylenchlorid eingesetzt.

Stellt man in einer bevorzugten Ausführungsform durch Zusatz von starken Deprotonierungsmitteln (wie z. B. Natriumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat und Pyridin aufgeführt.

Die Reaktion kann bei Normaldruck oder unter erhöhtem Druck durchgeführt werden, vorzugsweise wird bei

Normaldruck gearbeitet. Die Aufarbeitung geschieht nach üblichen Methoden.

Beim Herstellungsverfahren (E) kann gegebenenfalls unter Phasen-Transfer-Bedingungen gearbeitet werden (W.J. Spillane et. al.; J. Chem. Soc., Perkin Trans I, (3) 677-9 (1982)). In diesem Fall setzt man pro Mol Ausgangsverbindung der Formel (Ia) 0,3 bis 5 Mol Sulfonsäurechlorid der Formel (VIII), bevorzugt 1 Mol bei 0° bis 150°C, vorzugsweise bei 20 bis 70°C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol oder Toluol eingesetzt.

Beim Herstellungsverfahren (F) setzt man zum Erhalt von Verbindungen der Formel (Ie) auf 1 Mol der Verbindung der Formel (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C, ein.

Als gegebenenfalls zugesetzte Verdünnungsmittel kommen alle inerten organischen Lösungsmittel in Frage wie halogenierte Kohlenwasserstoffe, Ether, Amide, Nitrile, Carbonsäureester, Sulfone, Sulfoxide etc.

Vorzugsweise werden Acetonitril, Dimethylsulfoxid, Ethylacetat, Methyl-tert.-butylether, Tetrahydrofuran, Dimethylformamid oder Methylenchlorid eingesetzt.

Als gegebenenfalls zugesetzte Säurebindemittel kommen übliche anorganische oder organische Basen in Frage wie Hydroxide, Amine, Carbonate. Beispielhaft seien Natriumhydroxid, Natriumcarbonat, Kaliumcarbonat, Pyridin, Triethylamin oder DABCO aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, daß man Verbindungen der Formel (Ia) mit Metallverbindungen der Formel (X) oder Aminen der Formel (XI) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgeführt. Die Reaktionstemperaturen liegen im allgemeinen zwischen -20°C und 100°C, vorzugsweise zwischen 0°C und 50°C.

Bei der Durchführung des erfindungsgemäßen Verfahrens (H) werden die Ausgangsstoffe der Formeln (Ia) bzw. (XII) oder (XIII) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittel einengt.

Beim Herstellungsverfahren (Hα) setzt man pro Mol Ausgangsverbindung der Formel (Ia) ca. 1 Mol Isocyanat bzw. Isothiocyanat der Formel (XII) bei 0 bis 100°C, vorzugsweise bei 20 bis 50°C um.

Als gegebenenfalls zugesetzte Verdünnungsmittel kommen alle inerten organischen Lösungsmittel in Frage, wie Kohlenwasserstoffe, halogenierte Kohlenwasserstoffe, Ether, Carbonsäureester, Amide, Nitrile, Sulfone, Sulfoxide.

Vorzugsweise werden Toluol, Methylenchlorid, Tetrahydrofuran, Ethylacetat, Dimethylformamid oder Dimethylsulfoxid eingesetzt.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (Hβ) setzt man pro Mol Ausgangsverbindung der Formel (Ia) ca. 1 Mol Carbamidsäurechlorid bzw. Thiocarbamidsäurechlorid der Formel (XIII) bei 0 bis 150°C, vorzugsweise bei 20 bis 70°C um.

Als gegebenenfalls zugesetzte Verdünnungsmittel kommen aller inerten polaren organischen Lösungsmittel in Frage wie halogenierte Kohlenwasserstoffe, Carbonsäureester, Ether, Amide, Nitrile, Sulfone oder Sulfoxide.

Vorzugsweise werden Acetonitril, Ethylacetat, Dimethylsulfoxid, Methyl-tert.-butylether, Tetrahydrofuran, Dimethylformamid oder Methylenchlorid eingesetzt.

Stellt man in einer bevorzugten Ausführungsform durch Zusatz von starken Deprotonierungsmitteln (wie z. B. Natnumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin oder DABCO aufgeführt.

Die Reaktion kann bei Normaldruck oder unter erhöhtem Druck durchgeführt werden, vorzugswiese wird bei Normaldruck gearbeitet. Die Aufarbeitung geschieht nach üblichen Methoden.

Beispielhaft seien folgende Verbindungen der Formel (II) genannt:

3,3-Dimethyl-2-(2,4,6-trimethylphenyl)-acetyl-cyclopropancarbonsäuremethylester,
2-(2,4,6-Trimethylphenyl)-acetyl-cyclobutancarbonsäuremethylester,

1,2-Dimethyl-2-(2,4,6-trimethylphenyl)-acetyl-cyclobutancarbonsäuremethylester,
3,4-Dimethyl-2-(2,4,6-trimethylphenyl)-acetyl-cyclobutancarbonsäuremethylester,
2-(2,4,6-Trimethylphenyl)-acetyl-cyclopentancarbonsäuremethylester,
3-Methyl-2-(2,4,6-trimethylphenyl)-acetyl-cyclopentancarbonsäuremethylester,
3 -Methoxy-2-(2,4,6-trimethylphenyl)-acetyl-cyclopentancarbonsäuremethylester,
4-Methyl-2-(2,4,6-trimethylphenyl)-acetyl-cyclopentancarbonsäuremethylester,
4-Methoxy-2-(2,4,6-trimethylphenyl)-acetyl-cyclopentancarbonsäuremethylester,
3,4-Dimethyl-2-(2,4,6-trimethylphenyl)-acetyl-cyclopentancarbonsäuremethylester,
3,4-Dimethoxy-2-(2,4,6-trimethylphenyl)-acetyl-cyclopentancarbonsäuremethylester,
3,4-Dihydroxy-2-(2,4,6-trimethylphenyl)-acetyl-cyclopentancarbonsäuremethylester,
3-Oxo-2-(2,4,6-trimethylphenyl)-acetyl-cyclopentancarbonsäuremethylester,
4-Oxo-2-(2,4,6-trimethylphenyl)-acetyl-cyclopentancarbonsäuremethylester,
3,4-Methylendioxy-2-(2,4,6-trimethylphenyl)-acetyl-cyclopentancarbonsäuremethylester,
4,5-Methylendioxy-2-(2,4,6-trimethylphenyl)-acetyl-cyclohexancarbonsäuremethylester,
2-(2,4,6-Trimethylphenyl)-acetyl-cyclohexancarbonsäuremethylester,
3-Methyl-2-(2,4,6-trimethylphenyl)-acetyl-cyclohexancarbonsäuremethylester,
3-Methoxy-2-(2,4,6-trimethylphenyl)-acetyl-cyclohexancarbonsäuremethylester,
4-Methyl-2-(2,4,6-trimethylphenyl)-acetyl-cyclohexancarbonsäuremethylester,
4-Methoxy-2-(2,4,6-trimethylphenyl)-acetyl-cylcohexancarbonsäuremethylester,
5-Methyl-2-(2,4,6-trimethylphenyl)-acetyl-cyclohexancarbonsäuremethylester,
5-Methoxy-2-(2,4,6-trimethylphenyl)-acetyl-cyclohexancarbonsäuremethylester,
6-Methyl-2-(2,4,6-trimethylphenyl)-acetyl-cyclohexancarbonsäuremethylester,
6-Methoxy-2-(2,4,6-trimethylphenyl)-acetyl-cyclohexancarbonsäuremethylester,
1 -Methyl-2-(2,4,6-trimethylphenyl)-acetyl-cyclohexancarbonsäuremethylester,
2-Methyl-2-(2,4,6-trimethylphenyl)-acetyl-cyclohexancarbonsäuremethylester,
3,4-Dimethyl-2-(2,4,6-trimethylphenyl)-acetyl-cyclohexancarbonsäuremethylester,
5,6-Dimethyl-2-(2,4,6-trimethylphenyl)-acetyl-cyclohexancarbonsäuremethylester,
4,5-Dimethyl-2-(2,4,6-trimethylphenyl)-acetyl-cyclohexancarbonsäuremethylester,
4,5-Dihydroxy-2-(2,4,6-trimethylphenyl)-acetyl-cyclohexancarbonsäuremethylester,
4,5-Methylendioxy-2-(2,4,6-trimethylphenyl)-acetyl-cyclohexancarbonsäuremethylester,
4,5-Dihydroxy-4,5-O-Methyliden-2-(2,4,6-trimethylphenyl)-acetyl-cyclohexancarbonsäuremethylester,
1,2-Methylen-2-(2,4,6-trimethylphenyl)-acetyl-cyclohexancarbonsäuremethylester,
4-Oxo-2-(2,4,6-trimethylphenyl)-acetyl-cyclohexancarbonsäuremethylester,
1,2-Tetramethylen-2-(2,4,6-trimethylphenyl)-acetyl-cyclohexancarbonsäuremethylester,
3,6-Methylen-2-(2,4,6-trimethylphenyl)-acetyl-cyclohexancarbonsäuremethylester,
3,6-Ethylen-2-(2,4,6-trimethylphenyl)-acetyl-cyclohexancarbonsäuremethylester,
3,3-Dimethyl-2-(2,4,6-trimethylphenyl)-acetyl-cyclopropancarbonsäureethylester,
2-(2,4,6-Trimethylphenyl)-acetyl-cyclobutancarbonsäureethylester,
1,2-Dimethyl-2-(2,4,6-trimethylphenyl)-acetyl-cyclobutancarbonsäureethylester,
3,4-Dimethyl-2-(2,4,6-trimethylphenyl)-acetyl-cyclobutancarbonsäureethylester,
2-(2,4,6-Trimethylphenyl)-acetyl-cyclopentancarbonsäureethylester,
3-Methyl-2-(2,4,6-trimethylphenyl)-acetyl-cyclopentancarbonsäureethylester,
3-Methoxy-2-(2,4,6-trimethylphenyl)-acetyl-cyclopentancarbonsäureethylester,
4-Methyl-2-(2,4,6-trimethylphenyl)-acetyl-cyclopentancarbonsäureethylester,
4-Methoxy-2-(2,4,6-trimethylphenyl)-acetyl-cyclopentancarbonsäureethylester,
3,4-Dimethyl-2-(2,4,6-trimethylphenyl)-acetyl-cyclopentancarbonsäureethylester,
3,4-Dimethoxy-2-(2,4,6-trimethylphenyl)-acetyl -cyclopentancarbonsäureethylester,
3,4-Dihydroxy-2-(2,4,6-trimethylphenyl)-acetyl-cyclopentancarbonsäureethylester,
3-Oxo-2-(2,4,6-trimethylphenyl)-acetyl-cyclopentancarbonsäureethylester,
4-Oxo-2-(2,4,6-trimethylphenyl)-acetyl-cyclopentancarbonsäureethylester,
3,4-Methylethylidendioxy-(2,4,6-trimethylphenyl)-acetyl-cyclopentancarbonsäureethylester,
4,5-Methylethylidendioxy-2-(2,4,6-trimethylphenyl)-acetyl-cyclohexancarbonsäureethylester,
3-Methyl-2-(2,4,6-trimethylphenyl)-acetyl-cyclohexancarbonsäureethylester,
3-Methoxy-2-(2,4,6-trimethylphenyl)-acetyl-cyclohexancarbonsaureethylester,
4-Methyl-2-(2,4,6-trimethylphenyl)-acetyl-cyclohexancarbonsäureethylester,
4-Methoxy-2-(2,4,6-trimethylphenyl)-acetyl-cyclohexancarbonsäureethylester,
5-Methyl-2-(2,4,6-trimethylphenyl)-acetyl-cyclohexancarbonsäureethylester,
5-Methoxy-2-(2,4,6-trimethylphenyl)-acetyl-cyclohexancarbonsäureethylester,

6-Methyl-2-(2,4,6-trimethylphenyl)-acetyl-cyclohexancarbonsäureethylester,
6-Methoxy-2-(2,4,6-trimethylphenyl)-acetyl-cyclohexancarbonsäureethylester,
1-Methyl-2-(2,4,6-trimethylphenyl)-acetyl-cyclohexancarbonsäureethylester,
2-Methyl-2-(2,4,6-trimethylphenyl)-acetyl-cyclohexancarbonsäureethylester,
3,4-Dimethyl-2-(2,4,6-trimethylphenyl)-acetyl-cyclohexancarbonsäureethylester,
5,6-Dimethyl-2-(2,4,6-trimethylphenyl)-acetyl-cyclohexancarbonsäureethylester,
4,5-Dimethyl-2-(2,4,6-trimethylphenyl)-acetyl-cyclohexancarbonsäureethylester,
4,5-Dihydroxy-2-(2,4,6-trimethylphenyl)-acetyl-cyclohexancarbonsäureethylester,
4,5-Methylidendioxy-2-(2,4,6-trimethylphenyl)-acetyl-cyclohexancarbonsäureethylester,
4,5-Methylidendioxy-2-(2,4,6-trimethylphenyl)-acetyl-cyclohexancarbonsäureethylester,
1,2-Methylen-2-(2,4,6-trimethylphenyl)-acetyl-cyclohexancarbonsäureethylester,
4-Oxo-2-(2,4,6-trimethylphenyl)-acetyl-cyclohexancarbonsäureethylester,
1,2-Tetramethylen-2-(2,4,6-trimethylphenyl)-acetyl-cyclohexancarbonsäureethylester,
3,6-Methylen-2-(2,4,6-trimethylphenyl)-acetyl-cyclohexancarbonsäureethylester,
3,6-Ethylen-2-(2,4,6-trimethylphenyl)-acetyl-cyclohexancarbonsäureethylester.

Beispielhaft seien folgende Verbindungen der Formel (XIV) genannt:

3,3-Dimethyl-2-(2,4,6-trimethylphenyl)-acetyl-cyclopropancarbonsäure,
2-(2,4,6-Tnmethylphenyl)-acetyl-cyclobutancarbonsäure,
1,2-Dimethyl-2-(2,4,6-trimethylphenyl)-acetyl-cyclobutancarbonsäure,
2-(2,4,6-Trimethylphenyl)-acetyl-cyclobutancarbonsäure,
3,4-Dimethyl-2-(2,4,6-trimethylphenyl)-acetyl-cyclopentancarbonsäure,
3-Methyl-2-(2,4,6-trimethylphenyl)-acetyl-cyclopentancarbonsäure,
3-Methoxy-2-(2,4,6-trimethylphenyl)-acetyl-cyclopentancarbonsäure,
4-Methyl-2-(2,4,6-trimethylphenyl)-acetyl-cyclopentancarbonsäure,
4-Methoxy-2-(2,4,6-trimethylphenyl)-acetyl-cyclopentancarbonsäure,
3,4-Dimethyl-2-(2,4,6-trimethylphenyl)-acetyl-cyclopentancarbonsäure,
3,4-Dimethoxy-2-(2,4,6-trimethylphenyl)-acetyl-cyclopentancarbonsäure,
3,4-Dihydroxy-2-(2,4,6-trimethylphenyl)-acetyl-cyclopentancarbonsäure,
3-Oxo-2-(2,4,6-trimethylphenyl)-acetyl-cyclopentancarbonsäure,
4-Oxo-2-(2,4,6-trimethylphenyl)-acetyl-cyclopentancarbonsäure,
3,4-Methylethylidendioxy-2-(2,4,6-trimethylphenyl)-acetyl-cyclopentancarbonsäure,
2-(2,4,6-Trimethylphenyl)-acetyl-cyclohexancarbonsäure,
3-Methyl-2-(2,4,6-trimethylphenyl)-acetyl-cyclohexancarbonsäure,
3 -Methoxy-2-(2,4,6-trimethylphenyl)-acetyl-cyclohexancarbonsäure,
4-Methyl-2-(2,4,6-trimethylphenyl)-acetyl-cyclohexancarbonsäure,
4-Methoxy-2-(2,4,6-trimethylphenyl)-acetyl-cyclohexancarbonsäure,
5-Methyl-2-(2,4,6-trimethylphenyl)-acetyl-cyclohexancarbonsäure,
5-Methoxy-2-(2,4,6-trimethylphenyl)-acetyl-cyclohexancarbonsäure,
6-Methyl 2-(2,4,6-trimethylphenyl)-acetyl-cyclohexancarbonsäure,
6-Methoxy-2-(2,4,6-trimethylphenyl)-acetyl-cyclohexancarbonsäure,
1-Methyl 2-(2,4,6-trimethylphenyl)-acetyl-cyclohexancarbonsäure,
2-Methyl-2-(2,4,6-trimethylphenyl)-acetyl-cyclohexancarbonsäure,
3,4-Dimethyl-2-(2,4,6-trimethylphenyl)-acetyl-cyclohexancarbonsäure,
5,6-Dimethyl-2-(2,4,6-trimethylphenyl)-acetyl-cyclohexancarbonsäure,
4,5-Dimethyl-2-(2,4,6-trimethylphenyl)-acetyl-cyclohexancarbonsäure,
4,5 -Dihydroxy-2-(2,4,6-trimethylphenyl)-acetyl-cyclohexancarbonsäure,
4,5-Methylethylidendioxy-2-(2,4,6-trimethylphenyl)-acetyl-cyclohexancarbonsäure,
4,5-Methylendioxy-2-(2,4,6-trimethylphenyl)-acetyl-cyclohexancarbonsäure,
1,2-Methylen-2-(2,4,6-trimethylphenyl)-acetyl-cyclohexancarbonsäure,
4-Oxo-2-(2,4,6-trimethylphenyl)-acetyl-cyclohexancarbonsäure,
1,2-Tetramethylen-2-(2,4,6-trimethylphenyl)-acetyl-cyclohexancarbonsäure,
3,6-Methylen-2-(2,4,6-trimethylphenyl)-acetyl-cyclohexancarbonsäure,
3,6-Ethylen-2-(2,4,6-trimethylphenyl)-acetyl-cyclohexancarbonsäure.

Die erfindungsgemäßen Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria, Supella spp..

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Phthirus spp., Pediculus spp., Haematopinus spp., Linognathus spp., Solenopotes spp.. Aus der Ordnung der Mallophaga z.B. Trichodectes spp., Damalinea spp., Trimenopon spp., Monopon spp., Trinoton spp., Bovicola spp., Werneckiella spp., Lepikentron spp., Felicola spp..

Aus der Ordnung der Thysanoptera z.B. Hercinothrips femoralis, Thrips tabaci. Aus der Ordnung der Heteroptera z.B. Eurygaster spp., Dysdercus intermedius, Piesma quadrata, Cimex lectularius, Rhodnius prolixus, Triatoma spp., Panstrongylus spp..

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea Pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora spp., Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Hydrotaea spp., Stomoxys spp., Haematobia spp., Morellia spp., Fannia spp., Glossina spp., Calliphora spp., Wohlfahrtia spp., Sarcophaga spp., Oestrus spp., Hypoderma spp., Gasterophilus spp., Hippobosca spp., Lipoptena spp., Melophagus spp., Muscina spp..

Aus der Ordnung der Siphonapterida z.B. Xenopsylla spp., Ceratophyllus spp., Pulex spp., Ctenocephalides spp..

Aus der Ordnung der Arachnida z.B. Scorpio maurus, Latrodectus mactans.

Aus der Ordnung der Acarina z.B. Myocoptes spp., Otodectes spp., Acarus siro, Argas spp., Ornithodoros spp., Ornithonyssus spp., Dermanyssus spp., Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Dermacentor spp., Haemaphysalis spp., Raillietia spp., Pneumonyssus spp., Sternostorma spp., Varroa spp..

Aus der Ordnung der Actinedida (Prostigmata) und Acaridida (Astigmata) z.B.: Acarapis spp., Cheyletiella

spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten verwenden, wie beispielsweise gegen die Larven des Meerrettichblattkäfers (Phaedon cochleariae) oder gegen die Larven der grünen Reiszikade (Nephotettix cincticeps) oder gegen die Raupen der Kohlschabe (Plutella maculipennis).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotvle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotvle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.

Monokotvle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindunngen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat,

sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

**Fungizide:**

2-Aminobutan; 2-Anilino-4-methyl-6-cyclopropyl-pyrimidin; 2',6'-Dibromo-2-methyl-4'-trifluoromethoxy-4'-trifluoromethyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,

Benalaxyl, Benodanil, Benomyl, Binapacryl, Biphenyl, Bitertanol, Blasticidin-S, Bromuconazole, Bupirimate, Buthiobate,

Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,

Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,

Edifenphos, Epoxyconazole, Ethirimol, Etridiazol,

Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,

Guazatine,

Hexachlorobenzol, Hexaconazol, Hymexazol,

Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol,

Ofurace, Oxadixyl, Oxamocarb, Oxycarboxin,

Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),

Schwefel und Schwefel-Zubereitungen,

Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,

Validamycin A, Vinclozolin,

Zineb, Ziram.

**Bakterizide:**

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen, Quinalphos, RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

**Herbizide:**

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten Boli, des feed-through-Verfahrens, von Zäpfchen, durch parenterale Verabreichung, wie z.B. durch Injektion (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die Herstellung und die Verwendung der erfindungsgemäßen Stoffe wird durch die folgenden Beispiele veranschaulicht.

## Herstellungsbeispiele

### Beispiel Ia-1 und Ia-2

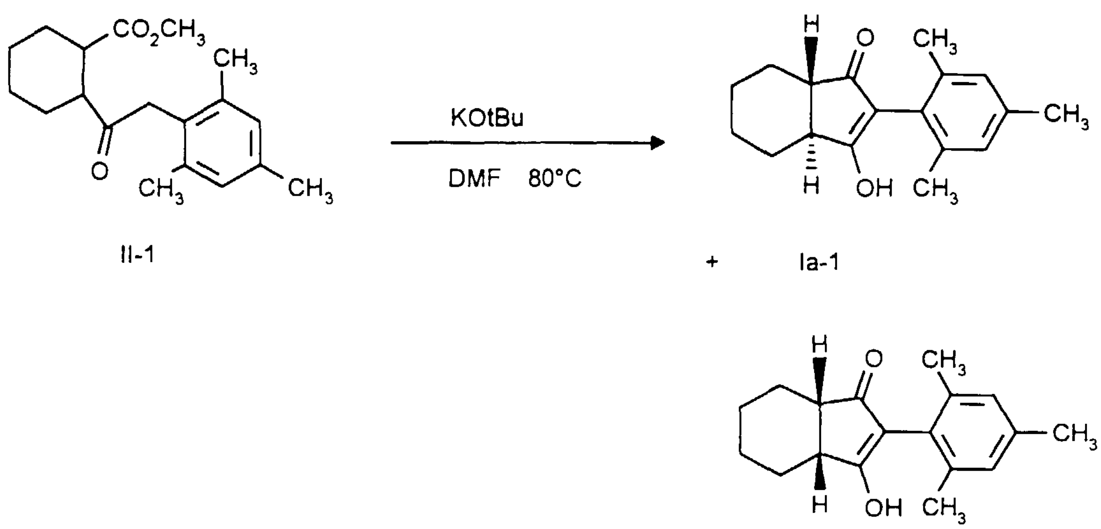


II-1

\+ Ia-1

Ia-2

Zu einer Lösung der Verbindung gemäß Beispiel II-1 (4,96 g; 16,4 mmol) in DMF (30 ml) gibt man 2,8 g Kalium-tert-butylat und rührt 1 Stunde bei 80°C. Man gibt 5 ml Essigsäure zu, engt ein und chromatographiert an Kieselgel (1:1 Ethylacetat:Hexan). Man isoliert zuerst 1,96 g (44 %) der oben gezeigten Verbindung Ia-1 (Farbloser Feststoff, Fp.: 192°C) und dann 1,27 g (29 %) der oben gezeigten Verbindung Ia-2 (Farbloser Feststoff, Fp. 184°C).

**Beispiel Ia-5**

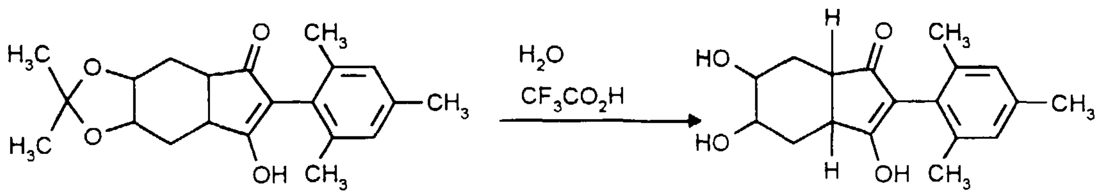


Ia-3
Ia-4

Ia-5

Eine Mischung der Verbindungen gemäß Bsp. Ia-3 und Ia-4 (cis-trans-Gemisch) (300 mg), Trifluoressigsäure (10 ml) und Wasser (10 ml) wird 24 Stunden bei Raumtemperatur gerührt und eingeengt. Der feste Rückstand wird mit heißem Cyclohexan gewaschen.
Ausbeute: 110 mg, Diastereomerengemisch.
$^1$H-NMR ($CDCl_3$, δ ppm): 2,25 (s, 3H); 4.69 und 4.80 (2s, 1H); 6.88 (s, 2H).
Analog zu den Beispielen Ia-1, Ia-2 und Ia-5 und gemäß den allgemeinen Angaben zur Herstellung wurden die in Tabelle 7 aufgeführten Verbindungen der Formel (Ia) synthetisiert.

**Tabelle 7**

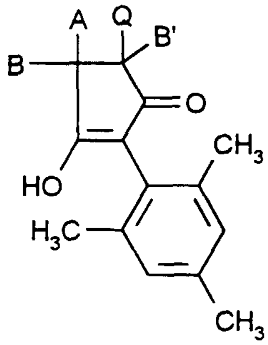

(Ia)

| Bsp.-Nr. | A Q | B | B' | Isomer | Fp.°C |
|---|---|---|---|---|---|
| Ia-3 | 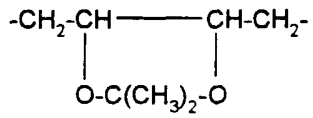 | H | H | trans | 160°C |
| Ia-4 | 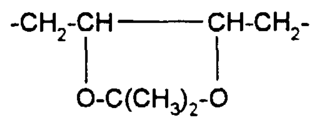 | H | H | cis | 130°C |
| Ia-6 | 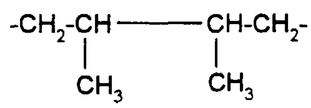 | H | H | trans | Öl |
| Ia-7 | 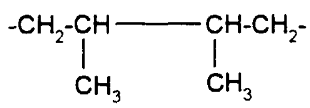 | H | H | cis | Öl |
| Ia-8 | 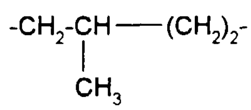 | H | H | trans | Öl |
| Ia-9 | 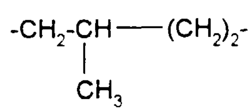 | H | H | cis | Öl |
| Ia-10 | 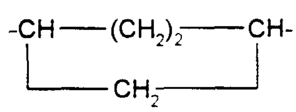 | H | H | cis/trans | 235°C |
| Ia-11 | $-(CH_2)_4-$ | $CH_3$ | H | cis/trans | 115°C |

**Beispiel Ib-1**

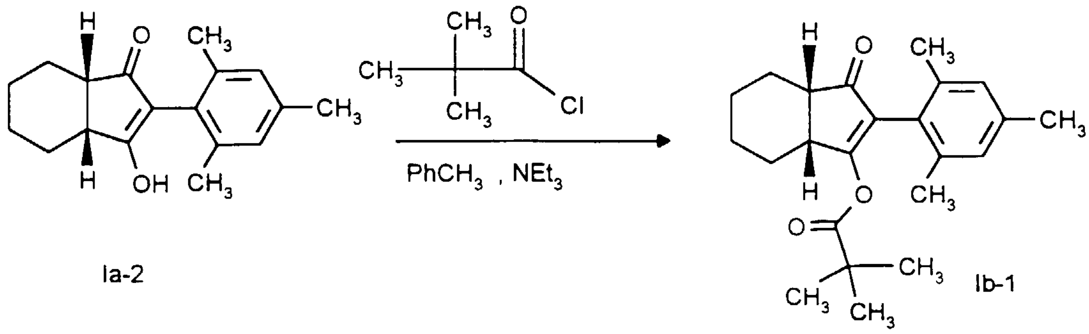


Ia-2

Ib-1

Zu einer Lösung der Verbindung gemäß Beispiel Ia-2 (1000 mg) in Toluol (10 ml) und Triethylamin (1 ml) gibt man Pivaloylchlorid (0,75 ml). Nach einer Stunde Rückfluß wird das Reaktionsgemisch direkt über Kieselgel filtriert (Laufmittel 1:4 Ethylether:Petrolether). Man erhält 1,02 g eines Öls. Ausbeute: 79 %.

$^1$H-NMR (200 MHz, $CDCl_3$, $\delta$ ppm): 1.10 (s, 9H); 1.4-2.3 (m, 8H); 2.05 (s, 3H); 2.10 (s, 3H); 2.23 (s, 3H); 2.84 (q, 1H, J = 6.5 Hz); 3.38 (q, 1H, J = 6.5 Hz); 6.82 (bs, 2H).

Analog zu Beispiel Ib-1 und gemäß den allgemeinen Angaben zur Herstellung wurden die in der Tabelle 8 aufgeführten Verbindungen der Formel (Ib) hergestellt:

**Tabelle 8**

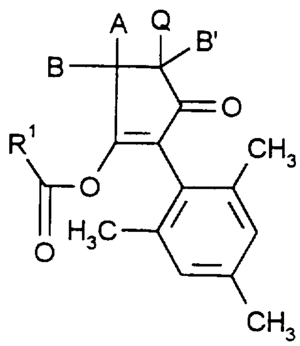

(Ib)

| Bsp.-Nr. | A Q | B | B' | $R^1$ | Isomer | $^1$H-NMR 200 MHz, $CDCl_3$, δ ppm |
|---|---|---|---|---|---|---|
| Ib-2 | $-(CH_2)_4-$ | H | H | $-CH_3$ | cis | 2.28 (s, 3H); 2.84 (q, 1H, J = 6.5 Hz); 3.43 (q, 1H, J = 6,5 Hz). |
| Ib-3 | $-(CH_2)_4-$ | H | H | $-CH_3$ | trans | 2.39 (m, 1H); 2.98 (m, 1H); 6.85 (bs, 2H). |
| Ib-4 | $-(CH_2)_4-$ | H | H | $-t-C_4H_9$ | trans | 1.11 (s, 9H); 6.81 (bs, 2H). |
| Ib-5 | 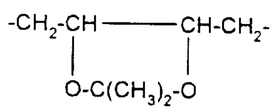 | H | H | $-t-C_4H_9$ | trans | Fp.: 148°C |
| Ib-6 | 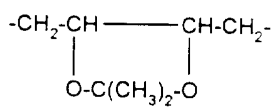 | H | H | $-CH_3$ | trans | Fp.: 150°C |
| Ib-7 | 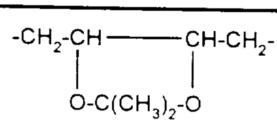 | H | H | $Cl-CH_2-$ | trans | Fp.: 130°C |
| Ib-8 | 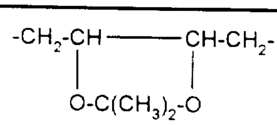 | H | H | 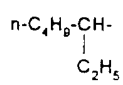 | trans | Fp.: 98°C |
| Ib-9 | $-(CH_2)_4-$ | H | H | 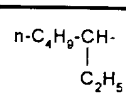 | cis | 2.22 (s, 3H); 6.81 (s, 2H). |

| Bsp.-Nr. | A Q | B | B' | $R^1$ | Isomer | $^1$H-NMR 200 MHz, $CDCl_3$, δ ppm |
|---|---|---|---|---|---|---|
| Ib-10 | $-(CH_2)_4-$ | H | H | 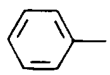 | cis | Fp.: 111°C |
| Ib-11 | 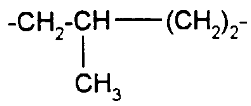 | H | H | $-t-C_4H_9$ | cis/trans | 1.08 (s, 9H); 6.81 (s, 2H). |
| Ib-12 | 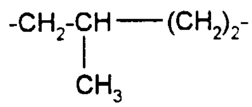 | H | H | $-CH_3$ | cis/trans | 2.26 (s, 3H); 6.86 (s, 2H). |
| Ib-13 | 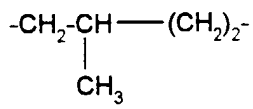 | H | H | 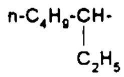 | cis/trans | 2.23 (s, 3H); 6.82 (s, 2H). |
| Ib-14 | 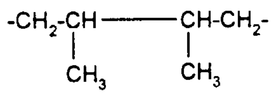 | H | H | $-t-C_4H_9$ | cis/trans | 2.25 (s, 3H); 6.82 (s, 2H). |
| Ib-15 | 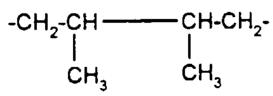 | H | H | $-CH_3$ | cis/trans | 2.28 (s, 3H); 6.86 (s, 2H). |
| Ib-16 | 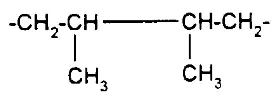 | H | H | 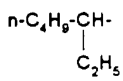 | cis/trans | 2.25 (s, 3H); 6.82 (s, 2H). |
| Ib-17 | 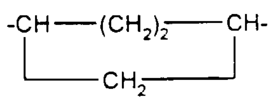 | H | H | $t-C_4H_9$ | cis/trans | Fp.: 106°C |
| Ib-18 | 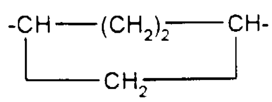 | H | H | $CH_3$ | cis/trans | Fp.: 142°C |

**Beispiel Ic-1**

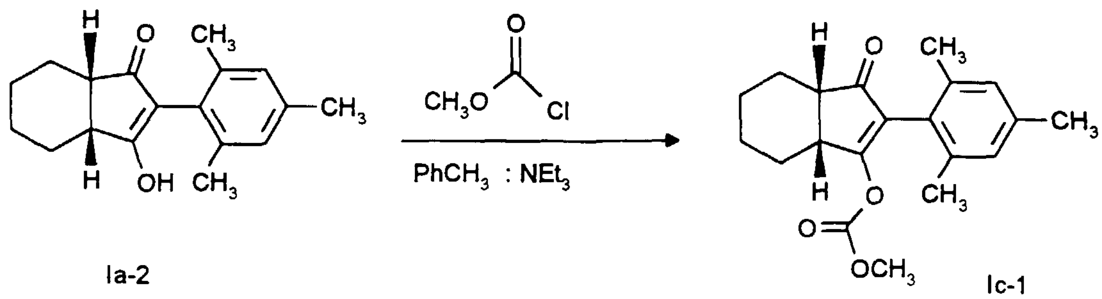


Ia-2

Ic-1

Zu einer Lösung der Verbindung gemäß Beispiel Ia-2 (1000 mg) in Toluol (10 ml) und Triethylamin (1 ml) gibt man Methylchloroformat (1,5 ml). Nach einer Stunde Rückfluß wird das Reaktionsgemisch direkt über Kieselgel filtriert (Laufmittel 1:4 Ethylether:Petrolether). Man erhält 1,19 g eines Öls. Ausbeute: 98 %.

$^1$H-NMR (200 MHz, $CDCl_3$, δ ppm): 1.4-1.85 (m, 8H); 2.06 (s, 3H); 2.11 (s, 3H); 2.28 (s, 4H); 2.87 (q, 1H, J = 6.5 Hz); 3.48 (q, 1H, J = 6,5 Hz); 3.72 (s, 3H); 6.87 (bs, 2H).

Analog zu Beispiel Ic-1 und gemäß den allgemeinen Angaben zur Herstellung wurde die in der Tabelle 9 aufgeführte Verbindung der Formel (Ic) hergestellt:

**Tabelle 9**

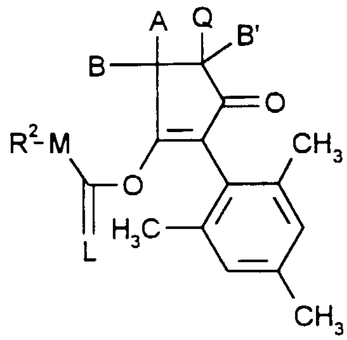

(Ic)

| Bsp.-Nr. | A Q | B | B' | L | M | $R^2$ | Isomer | $^1$H-NMR 200 MHz, $CDCl_3$, δ ppm |
|---|---|---|---|---|---|---|---|---|
| Ic-2 | $-(CH_2)_4-$ | H | H | O | O | $i-C_3H_7$ | cis | 2.83 (q, 1H, J = 6.5 Hz); 3.45 (q, 1H, J = 6.5 Hz). |
| Ic-3 | 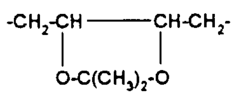 | H | H | O | O | $-CH_3$ | trans | Fp.: 162°C |
| Ic-4 | 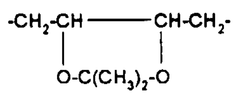 | H | H | O | O | $i-C_3H_7$ | trans | Fp.: 120°C |
| Ic-5 | 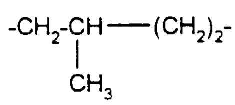 | H | H | O | O | $i-C_3H_7$ | cis/trans | 2.26 (s, 3H); 6.86 (s, 2H). |
| Ic-6 | 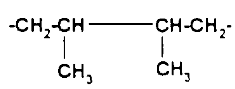 | H | H | O | O | $i-C_3H_7$ | cis/trans | 2.26 (s, 3H); 6.85 (s, 2H). |
| Ic-7 | 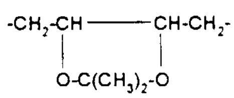 | H | H | O | O | $s-C_4H_9$ | trans | Fp.: 103°C |
| Ic-8 | 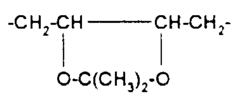 | H | H | O | O | $i-C_4H_9$ | trans | Fp.: 148°C |
| Ic-9 | 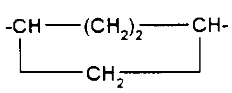 | H | H | O | O | $i-C_3H_7$ | cis/trans | Fp.: 104°C |

**Herstellung der Ausgangsverbindungen**

**Beispiel II-1**

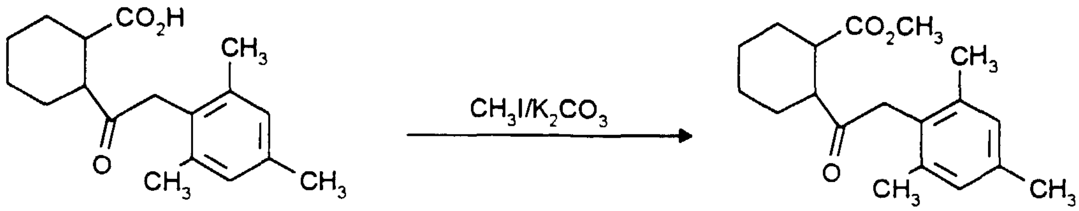


XIV-1 II-1

Eine Mischung der Verbindung gemäß Beispiel XIV-1 (2.13 g, 7,4 mmol), Kaliumcarbonat (1 g), Aceton (15 ml) und Iodmethan (1,4 ml) wird 5 Stunden unter Rückfluß gekocht, mit Ethylether (100 ml) verdünnt, über Kieselgel filtriert und eingeengt. Man erhält 2,22 g (99 %) der oben gezeigten Verbindung II-1. Weißer Feststoff, Fp.: 97°C.

Analog zu Beispiel II-1 und gemäß den allgemeinen Angaben zur Herstellung wurden die in der Tabelle 10 aufgeführten Verbindungen der Formel (II) hergestellt:

## Tabelle 10

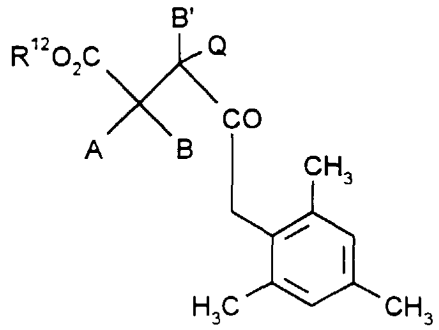

(II)

| Bsp. | A Q | B | B' | $R^{12}$ | $^1$H-NMR ($CDCl_3$, δppm oder Fp. (°C) |
|---|---|---|---|---|---|
| II-2 | (Z)-$CH_2$-CH=CH-$CH_2$- | H | H | $CH_3$ | 110°C |
| II-3 | (Z)-$CH_2$-C($CH_3$)=C($CH_3$)-$CH_2$- | H | H | $CH_3$ | 193°C |
| II-4 | 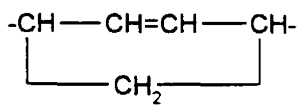 | H | H | $CH_3$ | 6.12 (m, 1H); 6.27 (m, 1H); 6.87 (s, 2H). |
| II-5 | 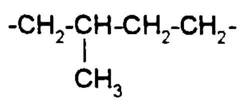 | H | H | $CH_3$ | 2.17 (s, 6H); 3.90 (m, 2H). |
| II-6 | (Z)-$CH_2$-CH=CH-$CH_2$- | H | $CH_3$ | $CH_3$ | 5.60-5.75 (m, 2H); 6.85 (s, 2H). |

**Beispiel II-9**

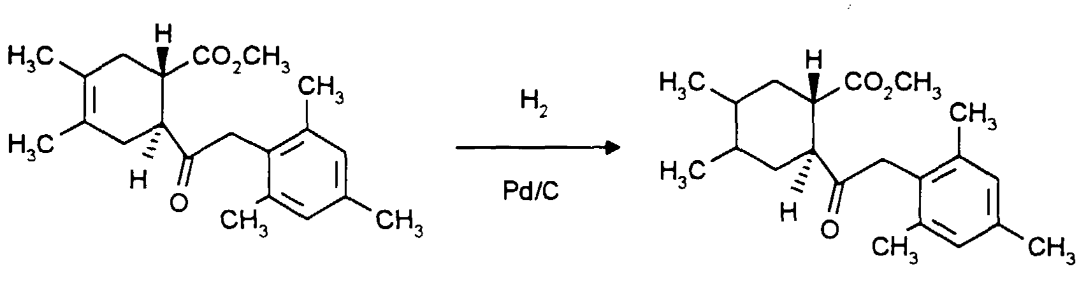


II-3 II-9

Eine Suspension der Verbindung gemäß Bsp. II-3 (15 g), 5 % Pd/C (4,5 g) in Methanol (300 ml) wird im Stahlautoklaven unter einem Wasserstoffdruck von 100 bar gerührt (100°C, 24 Stunden). Das Reaktionsgemisch wird gekühlt, filtriert und eingeengt.

Man erhält 13 g der oben gezeigten Verbindung II-9 als Diastereomerengemisch (Öl).

$^1H$-NMR ($CDCl_3$, δ ppm): 3.60 (s, 3H); 3.80-3.90 (m, 2H); 6.85 (bs, 2H).

Analog zu Beispiel II-9 und gemäß den allgemeinen Angaben zur Herstellung wurde die in der Tabelle 11 aufgeführte Verbindung der Formel (II) hergestellt:

**Tabelle 11:**

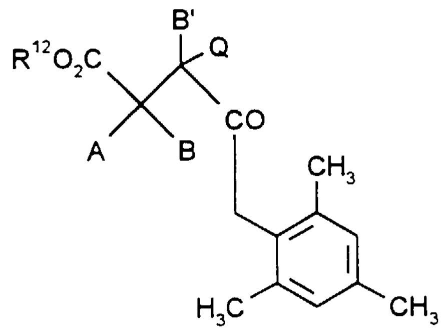

(II)

| Bsp. | A Q | B | B' | $R^{12}$ | $^1H$-NMR ($CDCl_3$, δ ppm) |
|---|---|---|---|---|---|
| II-7 | 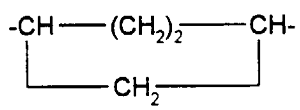 | H | H | $CH_3$ | 2.25 (s, 3H); 6.86 (s, 2H). |
| II-8 | $-(CH_2)_4-$ | H | $CH_3$ | $CH_3$ | 3.65 (s, 3H); 6.85 (s, 2H). |

**Beispiel II-10**

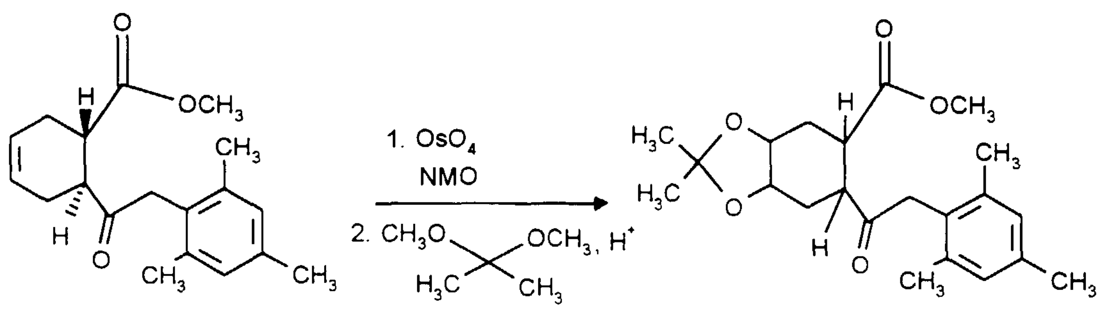


II-2

II-10

Zu einer Lösung von N-Methylmorpholinoxid (NMO) (21,4 g) in Aceton (145 ml) und Wasser (28 ml) gibt man 43,6 g der Verbindung gemäß Beispiel II-2 und 8,7 ml 2,5 %iges $OsO_4$ in t-Butanol. Man rührt 4 Stunden bei Raumtemperatur, gibt 17,4 g Natriumthiosulfat zu und rührt eine halbe Stunde bei Raumtemperatur. Das Produkt wird mit Ether extrahiert.

Man erhält 45,5 g (94,5 % der Theorie) farblosen Feststoff (Diastereomerengemisch).

Dieses Material wird in 2,2-Dimethoxypropan (100 ml) gelöst und mit 200 mg 4-Toluolsulfonsäurehydrat versetzt. Nach 20 Stunden bei Raumtemperatur wäscht man das Reaktionsgemisch mit 10 %iger wäßriger Kaliumcarbonatlösung, trocknet und engt ein.
Man erhält 45 g der oben gezeigten Verbindung II-10 (Öl, Diastereomerengemisch).
$^1$H-NMR ($CDCl_3$, δ ppm): 2.26 (s, 3H), 3.62 (s, 3H); 6.82 (bs, 2H).

**Beispiel XIV-1**

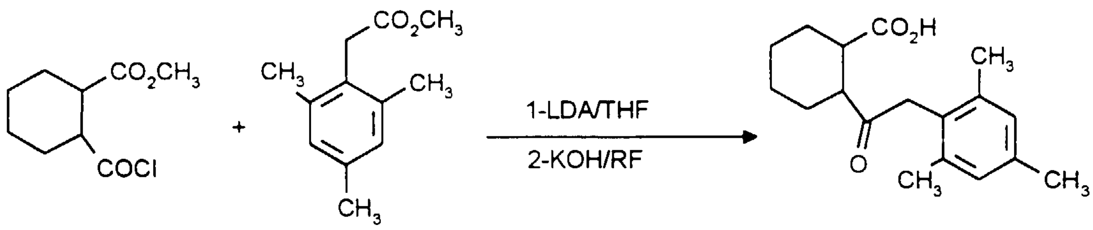


XIV-1

Zu einer Lösung von Lithiumdiisopropylamid (75 mmol) in THF (100 ml) gibt man 13,2 g (68,7 mmol) 2,4,6-Trimethylphenylessigsäuremethylester. Nach 30 Minuten bei Raumtemperatur gibt man 14 g 3,4-Tetramethylenbernsteinsäuremethylesterchlorid zu und rührt bei Raumtemperatur (1 Stunde). Dann gibt man 100 ml Wasser und 30 g Ammoniumchlorid zu. Das Zwischenprodukt wird mit Ether extrahiert und über Kieselgel filtriert. Nach Einengen wird der Rückstand (Öl, ca. 25 g) mit 75 g Kaliumhydroxid und 250 ml Wasser unter Rückfluß gekocht (2 Tage). Man kühlt ab, säuert an (konz. HCl) und filtriert den Feststoff ab. Nach Umkristallisieren aus Ethylacetat: Cyclohexan 1:1 erhält man 7,5 g (38 %) der oben gezeigten Verbindung XIV-1.
Weißer Feststoff, Fp.: 195°C.
Analog zu Beispiel XIV-1 und gemäß den allgemeinen Angaben zur Herstellung wurde die in der Tabelle 12 aufgeführte Verbindung der Formel (XIV) hergestellt:

**Tabelle 12**

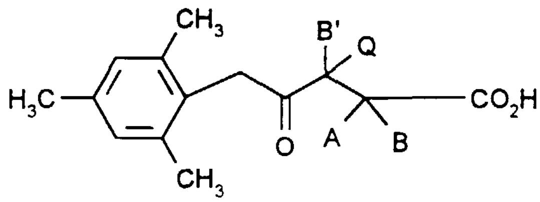

(XIV)

| Bsp.-Nr. | A Q | B | B' | $^1$H-NMR ($CDCl_3$, δ ppm) oder Fp. (°C) |
|---|---|---|---|---|
| XIV-2 | (Z)-$CH_2$-CH=CH-$CH_2$- | H | H | 193°C |
| XIV-3 | 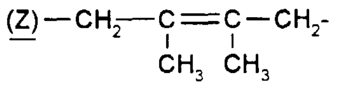 | H | H | 1.11 (bs, 6H); 3.90 (s, 2H). |
| XIV-4 | 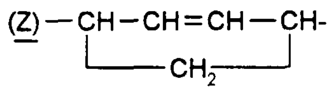 | H | H | 6.17 (m, 1H); 6.27 (m, 1H); 6.87 (s, 2H). |
| XIV-5 | 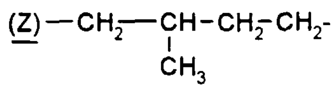 | H | H | 3.88 (m, 2H); 6.85 (s, 2H) |
| XIV-6 | (Z)-$CH_2$-CH=CH-$CH_2$- | H | $CH_3$ | Öl |

**Anwendungsbeispiele**

**Beispiel A**

| Pre-emergence-Test | | |
|---|---|---|
| Lösungsmittel | 5 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

| | |
|---|---|
| O % = | keine Wirkung (wie unbehandelte Kontrolle) |

(fortgesetzt)

| | |
|---|---|
| 100 % = | totale Vernichtung |

In diesem Test zeigte z.B. die Verbindung Ia-1 bei einer beispielhaften Aufwandmenge von 250 g/ha 100 % Wirkung gegen Alopecurus myosuroides, Avena fatua und Setaria viridis.

**Beispiel B**

| Post-emergence-Test | | |
|---|---|---|
| Lösungsmittel | 5 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

| | |
|---|---|
| O % = | keine Wirkung (wie unbehandelte Kontrolle) |
| 100 % = | totale Vernichtung |

In diesem Test zeigte z.B. die Verbindung Ib-1 bei einer beispielhaften Aufwandmenge von 250 g/ha 100 % Wirkung gegen Avena fatua und Setaria viridis.

**Beispiel C**

| Test mit Boophilus microplus resistent / SP-resistenter Parkhurst-Stamm | |
|---|---|
| Testtiere | Adulte gesogene Weibchen |
| Lösungsmittel | Dimethylsulfoxid |

20 mg Wirkstoff werden in 1 ml Dimethylsulfoxid gelöst, geringere Konzentrationen werden durch Verdünnen mit dem gleichen Lösungsmittel hergestellt.

Der Test wird in 5-fach Bestimmung durchgeführt. 1 μl der Lösungen wird in das Abdomen injiziert, die Tiere in Schalen überführt und in einem klimatisierten Raum aufbewahrt. Die Wirkungskontrolle erfolgt nach 7 Tagen auf Ablage fertiler Eier. Eier, deren Fertilität nicht äußerlich sichtbar ist, werden in Glasröhrchen bis zum Larvenschlupf im Klimaschrank aufbewahrt. Eine Wirkung von 100 % bedeutet, daß keine Zecke fertile Eier gelegt hat.

In diesem Test hatten z.B. die Verbindungen gemäß den Herstellungsbeispielen Ia-1 und Ia-2 bei einer beispielhaften Wirkstoffkonzentration von 1000 ppm eine Wirkung von 100 %.

**Beispiel D**

| Blowfly-Larven-Test / Entwicklungshemmende Wirkung | |
|---|---|
| Testtiere | Lucilia cuprina-Larven |

(fortgesetzt)

| Blowfly-Larven-Test / Entwicklungshemmende Wirkung | |
|---|---|
| Lösungsmittel | 35 Gewichtsteile Ethylenglykolmonomethylether<br>35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

Etwa 20 Lucilia cuprina-Larven werden in ein Teströhrchen gebracht, welches ca. 1 $cm^3$ Pferdefleisch und 0,5 ml der zu testenden Wirkstoffzubereitung enthält. Nach 24 und 48 Stunden wird die Wirksamkeit der Wirkstoffzubereitung ermittelt. Die Teströhrchen werden in Becher mit Sand-bedecktem Boden überführt. Nach weiteren 2 Tagen werden die Teströhrchen entfernt und die Puppen ausgezählt.

Die Wirkung der Wirkstoffzubereitung wird nach der Zahl der geschlüpften Fliegen nach 1,5-facher Entwicklungsdauer und unbehandelter Kontrolle beurteilt. Dabei bedeutet 100 %, daß keine Fliegen geschlüpft sind; 0 % bedeutet, daß alle Fliegen normal geschlüpft sind.

In diesem Test hatten z.B. die Verbindungen gemäß den Herstellungsbeispielen Ia-1, Ib-2 und Ib-4 bei einer beispielhaften Konzentration von 1000 ppm eine Wirkung von 100 %.

## Beispiel E

| Tetranychus-Test (OP-resistent/Tauchbehandlung) | | |
|---|---|---|
| Lösungsmittel | 3 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatten z.B. die Verbindungen gemäß den Herstellungsbeispielen Ia-1 und Ia-2 bei einer beispielhaften Wirkstoffkonzentration von 0,1 % eine Wirkung von mindestens 98 % nach 13 Tagen.

## Beispiel F

| Phaedon-Larven-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen Ia-1, Ia-2, Ib-1 und Ib-4 bei einer beispielhaften Wirkstoffkonzentration von 0,1 % eine Abtötung von 100 % nach 7 Tagen.

**Beispiel G**

| Plutella-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.
Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.
Nach der gewünschten Zeit wird die Abtötung in % bestimmt. Dabei bedeutet 100 %, daß alle Raupen abgetötet wurden; 0 % bedeutet, daß keine Raupen abgetötet wurden.
In diesem Test bewirkten z.B. die Verbindungen gemäß den Herstellungsbeispielen Ia-1, Ia-2, Ib-1, Ib-2 und Ib-4 bei einer beispielhaften Wirkstoffkonzentration von 0,1 % eine Abtötung von 100 % nach 7 Tagen.

**Beispiel H**

| Spodoptera-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.
Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.
Nach der gewünschten Zeit wird die Abtötung in % bestimmt. Dabei bedeutet 100 %, daß alle Raupen abgetötet wurden; 0 % bedeutet, daß keine Raupen abgetötet wurden.
In diesem Test bewirkte z.B. die Verbindung gemäß Herstellungsbeispiel Ia-1 bei einer beispielhaften Wirkstoffkonzentration von 0,1 % eine Abtötung von 100 % nach 7 Tagen.

**Beispiel I**

| Nephotettix-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.
Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.
Nach der gewünschten Zeit wird die Abtötung in % bestimmt. Dabei bedeutet 100 %, daß alle Zikaden abgetötet wurden; 0 % bedeutet, daß keine Zikaden abgetötet wurden.
In diesem Test bewirkten z.B. die Verbindungen gemäß den Herstellungsbeispielen Ia-1, Ia-2, Ib-1, Ib-2 und Ib-4 bei einer beispielhaften Wirkstoffkonzentration von 0,1 % eine Abtötung von 100 % nach 6 Tagen.

## Patentansprüche

**1.** Verbindungen der Formel (I)

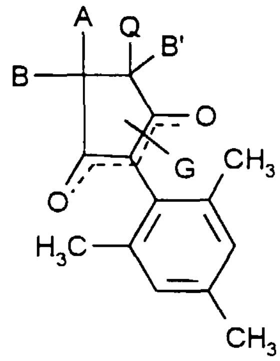

(I)

in welcher

A und Q gemeinsam für jeweils gegebenenfalls durch Halogen, Hydroxy, Mercapto, jeweils gegebenenfalls substituiertes Alkyl, Alkoxy, Alkylthio, Cycloalkyl, Benzyloxy oder Aryl substituiertes Alkandiyl oder Alkendiyl stehen, welches außerdem gegebenenfalls eine der nachstehenden Gruppen

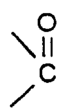 ; 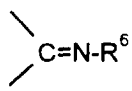 ; 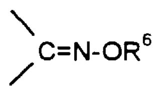 ; 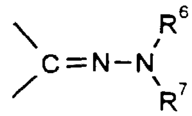 ;

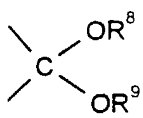 ; 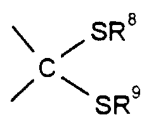 ; 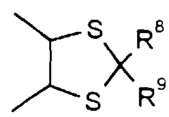 ;

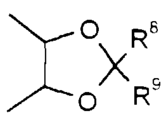 ; 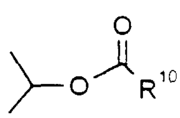 oder 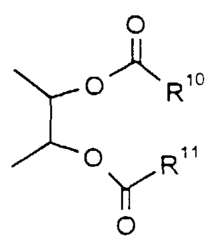

enthält oder durch eine Alkandiylgruppe überbrückt ist,

B und B' unabhängig voneinander für Wasserstoff, Halogen oder Alkyl oder gemeinsam für jeweils gegebenenfalls substituiertes Alkandiyl oder Alkendiyl stehen,

G für Wasserstoff (a) oder für eine der Gruppen

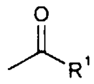 (b), 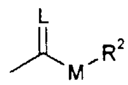 (c), 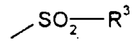 (d), 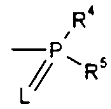 (e),

E (f)

oder

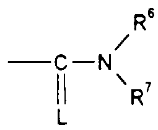 (g),

steht,
in welchen

E für ein Metallionäquivalent oder ein Ammoniumion steht,

L für Sauerstoff oder Schwefel steht und

M für Sauerstoff oder Schwefel steht,

$R^1$ für jeweils gegebenenfalls substituiertes Alkyl, Alkenyl, Alkoxyalkyl, Alkylthioalkyl, Polyalkoxyalkyl oder Cycloalkyl, das mindestens ein Heteroatom enthalten kann, jeweils gegebenenfalls substituiertes Phenyl, Phenylalkyl, Hetaryl, Phenoxyalkyl oder Hetaryloxyalkyl steht,

$R^2$ für jeweils gegebenenfalls substituiertes Alkyl, Cycloalkyl, Alkenyl, Alkoxyalkyl, Polyalkoxyalkyl oder jeweils gegebenenfalls substituiertes Phenyl oder Benzyl steht,

$R^3$ für jeweils gegebenenfalls substituiertes Alkyl, Phenyl oder Phenylalkyl steht,

$R^4$ und $R^5$ unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Phenoxy oder Phenylthio stehen,

$R^6$ für Wasserstoff, jeweils gegebenenfalls durch Halogen substituiertes Alkyl, Alkenyl, Alkoxyalkyl, für jeweils gegebenenfalls substituiertes Cycloalkyl oder Phenyl oder für gegebenenfalls substituiertes Benzyl steht,

$R^7$ für Wasserstoff, jeweils gegebenenfalls durch Halogen substituiertes Alkyl oder Alkenyl steht oder

$R^6$ und $R^7$ gemeinsam mit dem N-Atom, an das sie gebunden sind, für einen gegebenenfalls Sauerstoff oder Schwefel enthaltenden Ring stehen,

$R^8$ und $R^9$ unabhängig voneinander für Wasserstoff oder jeweils gegebenenfalls substituiertes Alkyl, Phenyl oder Phenylalkyl stehen oder
zusammen für einen gegebenenfalls substituierten Alkandiylrest stehen und

$R^{10}$ und $R^{11}$ unabhängig voneinander für jeweils gegebenenfalls durch Halogen substituiertes Alkyl, Alkenyl, Alkoxy, Alkylamino, Dialkylamino, Alkenylamino oder Dialkenylamino oder jeweils gegebenenfalls substituiertes Phenyl oder Benzyl stehen.

2. Verbindungen der Formel (I) gemäß Anspruch 1, welche unter Einbeziehung der verschiedenen Bedeutungen (a), (b), (c), (d), (e), (f) und (g) der Gruppe G folgende Strukturen (Ia) bis (Ig) besitzen:

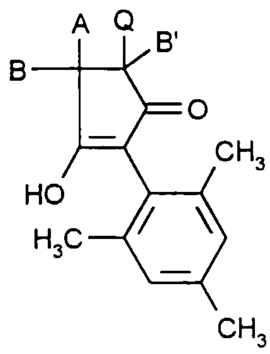

(Ia)

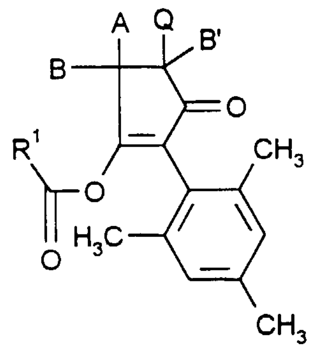

(Ib)

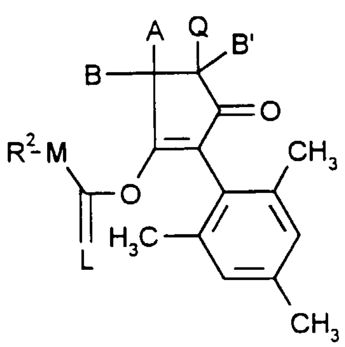

(Ic)

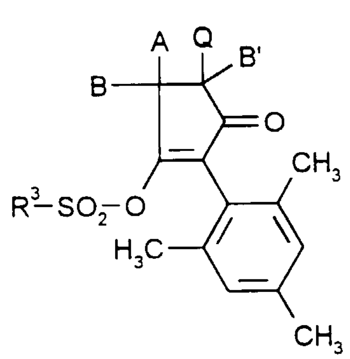

(Id)

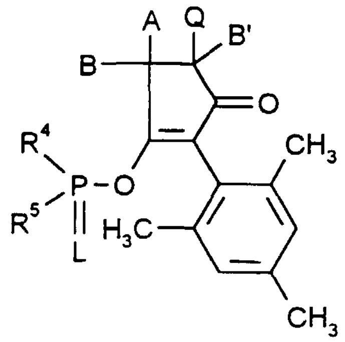

(Ie)

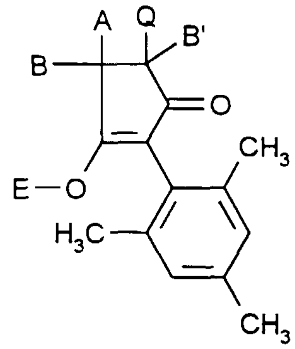

(If)

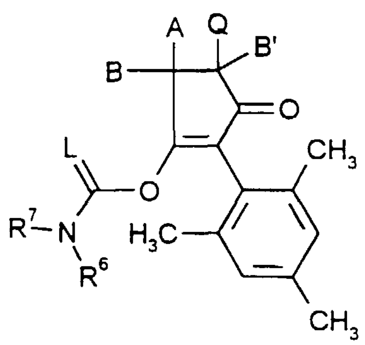

(Ig)

worin
A, B, B', E, L, M, Q, $R^1$, $R^2$, $R^3$, $R^4$, $R^5$, $R^6$ und $R^7$ die oben angegebenen Bedeutungen besitzen.

**3.** Verbindungen der Formel (I) gemäß Anspruch 1, in welcher

A und Q gemeinsam für jeweils gegebenenfalls einfach bis dreifach, gleich oder verschieden durch Halogen, Hydroxy, Mercapto, jeweils gegebenenfalls einfach bis neunfach, gleich oder verschieden durch Halogen substituiertes $C_1$-$C_{10}$-Alkyl, $C_1$-$C_6$-Alkoxy, $C_1$-$C_6$-Alkylthio, $C_3$-$C_7$-Cycloalkyl oder jeweils gegebenenfalls einfach bis fünffach, gleich oder verschieden durch Halogen, $C_1$-$C_6$-Alkyl oder $C_1$-$C_6$-Alkoxy substituiertes Benzyloxy oder Phenyl substituiertes $C_1$-$C_6$-Alkandiyl oder $C_2$-$C_6$-Alkendiyl stehen, welches außerdem gegebenenfalls eine der nachstehenden Gruppierungen

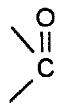 ; 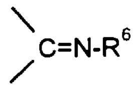 ; 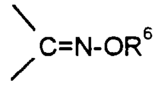 ; 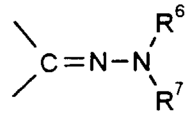 ;

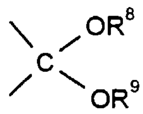 ; 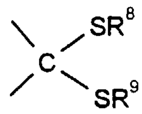 ; 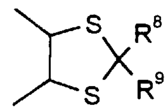 ;

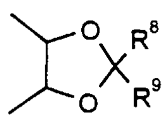 ; 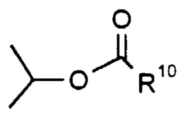 oder 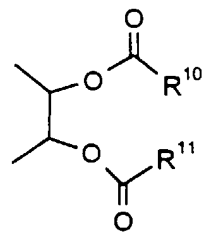

enthält oder durch eine $C_1$-$C_2$-Alkandiylgruppe überbrückt ist,

B und B' unabhängig voneinander für Wasserstoff, Halogen oder $C_1$-$C_6$-Alkyl oder gemeinsam für jeweils gegebenenfalls durch $C_1$-$C_6$-Alkyl substituiertes $C_1$-$C_6$-Alkandiyl oder $C_4$-Alkendiyl stehen,

G für Wasserstoff (a) oder für eine der Gruppen

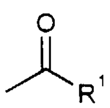 (b), 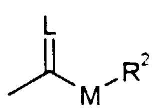 (c), 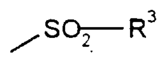 (d), 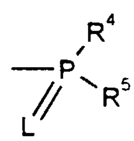 (e),

E (f)

oder

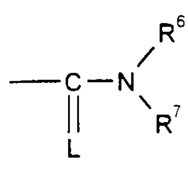 (g),

in welchen

E für ein Metallionäquivalent oder ein Ammoniumion steht,
L für Sauerstoff oder Schwefel steht,
M für Sauerstoff oder Schwefel steht,

$R^1$ für jeweils gegebenenfalls einfach oder mehrfach, gleich oder verschieden durch Halogen substituiertes $C_1$-

$C_{20}$-Alkyl, $C_2$-$C_{10}$-Alkenyl, $C_1$-$C_8$-Alkoxy-$C_1$-$C_8$-alkyl, $C_1$-$C_8$-Alkylthio-$C_1$-$C_8$-alkyl, Poly-$C_1$-$C_8$-alkoxy-$C_1$-$C_8$-alkyl oder für gegebenenfalls einfach oder mehrfach, gleich oder verschieden durch Halogen, $C_1$-$C_6$-Alkyl oder $C_1$-$C_6$-Alkoxy substituiertes Cycloalkyl mit 3 bis 8 Ringatomen, in welchem mindestens eine Methylengruppe durch ein Sauerstoff- und/oder Schwefelatom ersetzt sein kann,
für gegebenenfalls einfach oder mehrfach, gleich oder verschieden durch Halogen, Nitro, Cyano, $C_1$-$C_6$-Alkyl, $C_1$-$C_6$-Alkoxy, $C_1$-$C_6$-Halogenalkyl oder $C_1$-$C_6$-Halogenalkoxy substituiertes Phenyl,
für gegebenenfalls einfach oder mehrfach, gleich oder verschieden durch Halogen, $C_1$-$C_6$-Alkyl, $C_1$-$C_6$-Alkoxy, $C_1$-$C_6$-Halogenalkyl oder $C_1$-$C_6$-Halogenalkoxy substituiertes Phenyl-$C_1$-$C_6$-alkyl,
für gegebenenfalls einfach oder mehrfach, gleich oder verschieden durch Halogen oder $C_1$-$C_6$-Alkyl substituiertes Hetaryl mit 5 oder 6 Ringatomen,
für gegebenenfalls einfach oder mehrfach, gleich oder verschieden durch Halogen oder $C_1$-$C_6$-Alkyl substituiertes Phenoxy-$C_1$-$C_6$-alkyl oder
für gegebenenfalls einfach oder mehrfach, gleich oder verschieden durch Halogen, Amino oder $C_1$-$C_6$-Alkyl substituiertes Hetaryloxy-$C_1$-$C_6$-alkyl mit 5 oder 6 Ringatomen steht,

$R^2$ für jeweils gegebenenfalls einfach oder mehrfach, gleich oder verschieden durch Halogen substituiertes $C_1$-$C_{20}$-Alkyl, $C_2$-$C_{10}$-Alkenyl, $C_1$-$C_8$-Alkoxy-$C_2$-$C_8$-alkyl, Poly-$C_1$-$C_8$-alkoxy-$C_2$-$C_8$-alkyl,
für gegebenenfalls einfach oder mehrfach, gleich oder verschieden durch Halogen, $C_1$-$C_6$-Alkyl oder $C_1$-$C_6$-Alkoxy substituiertes $C_3$-$C_6$-Cycloalkyl oder
für jeweils gegebenenfalls einfach oder mehrfach, gleich oder verschieden durch Halogen, Nitro, Cyano, $C_1$-$C_6$-Alkyl, $C_l$-$C_6$-Alkoxy, $C_1$-$C_3$-Halogenalkoxy oder $C_1$-$C_3$-Halogenalkyl substituiertes Phenyl oder Benzyl steht,

$R^3$ für jeweils gegebenenfalls einfach oder mehrfach, gleich oder verschieden durch Halogen substituiertes $C_1$-$C_{12}$-Alkyl oder für jeweils gegebenenfalls einfach oder mehrfach, gleich oder verschieden durch Halogen, $C_1$-$C_6$-Alkyl, $C_1$-$C_6$-Alkoxy, $C_1$-$C_3$-Halogenalkyl, $C_1$-$C_3$-Halogenalkoxy, Cyano oder Nitro substituiertes Phenyl oder Phenyl-$C_1$-$C_4$-alkyl steht,

$R^4$ und $R^5$unabhängig voneinander für jeweils gegebenenfalls einfach oder mehrfach, gleich oder verschieden durch Halogen substituiertes $C_1$-$C_8$-Alkyl, $C_1$-$C_8$-Alkoxy, $C_1$-$C_8$-Alkylamino, Di-($C_1$-$C_8$-alkyl)-amino, $C_1$-$C_8$-Alkylthio, $C_3$-$C_5$-Alkenylthio, $C_3$-$C_7$-Cycloalkylthio, für jeweils gegebenenfalls einfach oder mehrfach, gleich oder verschieden durch Halogen, Nitro, Cyano, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl oder $C_1$-$C_4$-Halogenalkyl substituiertes Phenyl, Phenoxy oder Phenylthio stehen,

$R^6$ für Wasserstoff, jeweils gegebenenfalls einfach oder mehrfach, gleich oder verschieden durch Halogen substituiertes $C_1$-$C_{10}$-Alkyl, $C_3$-$C_8$-Alkenyl, $C_1$-$C_8$-Alkoxy-$C_2$-$C_8$-alkyl, für gegebenenfalls einfach oder mehrfach, gleich oder verschieden durch Halogen, $C_1$-$C_6$-Alkyl, $C_1$-$C_6$-Alkoxy, $C_1$-$C_3$-Halogenalkyl oder $C_1$-$C_3$-Halogenalkoxy substituiertes $C_3$-$C_{10}$-Cycloalkyl, für gegebenenfalls einfach oder mehrfach, gleich oder verschieden durch Halogen, $C_1$-$C_3$-Halogenalkyl, $C_1$-$C_8$-Alkyl, $C_1$-$C_3$-Halogenalkoxy oder $C_1$-$C_8$-Alkoxy substituiertes Phenyl oder für gegebenenfalls einfach oder mehrfach, gleich oder verschieden durch Halogen, $C_1$-$C_8$-Alkyl, $C_1$-$C_3$-Halogenalkyl, $C_1$-$C_3$-Halogenalkoxy oder $C_1$-$C_8$-Alkoxy substituiertes Benzyl steht,

$R^7$ für Wasserstoff, jeweils gegebenenfalls einfach oder mehrfach, gleich oder verschieden durch Halogen substituiertes $C_1$-$C_{10}$-Alkyl oder $C_3$-$C_{10}$-Alkenyl steht oder

$R^6$ und $R^7$gemeinsam mit dem N-Atom, an das sie gebunden sind, für einen gegebenenfalls Sauerstoff oder Schwefel enthaltenden 3- bis 7-gliedrigen Ring stehen,

$R^8$ und $R^9$unabhängig voneinander für Wasserstoff, gegebenenfalls einfach oder mehrfach, gleich oder verschieden durch Halogen substituiertes $C_1$-$C_6$-Alkyl oder für jeweils gegebenenfalls einfach oder mehrfach, gleich oder verschieden durch Halogen, $C_1$-$C_6$-Alkyl, $C_1$-$C_6$-Alkoxy, $C_1$-$C_6$-Halogenalkyl, $C_1$-$C_6$-Halogenalkoxy, Nitro oder Cyano substituiertes Phenyl oder Phenyl-$C_1$-$C_4$-alkyl,
oder zusammen für gegebenenfalls einfach oder mehrfach, gleich oder verschieden durch Halogen, $C_1$-$C_6$-Alkyl, $C_1$-$C_6$-Alkoxy oder $C_1$-$C_3$-Halogenalkyl substituiertes $C_2$-$C_6$-Alkandiyl stehen und

$R^{10}$ und $R^{11}$unabhängig voneinander für jeweils gegebenenfalls einfach oder mehrfach, gleich oder verschieden durch Halogen substituiertes $C_1$-$C_{10}$-Alkyl, $C_2$-$C_{10}$-Alkenyl, $C_1$-$C_{10}$-Alkoxy, $C_1$-$C_{10}$-Alkylamino oder Di-($C_1$-$C_{10}$-alkyl)-amino, $C_3$-$C_{10}$-Alkenylamino, Di-($C_3$-$C_{10}$-alkenyl)-amino oder jeweils einfach oder mehrfach, gleich

oder verschieden durch Halogen, $C_1$-$C_4$-Alkyl, $C_1$-$C_4$-Alkoxy, $C_1$-$C_4$-Halogenalkyl, Nitro oder Cyano substituiertes Phenyl oder Benzyl stehen.

**4.** Verbindungen der Formel (I) gemäß Anspruch 1, in welcher

A und Q gemeinsam für jeweils gegebenenfalls einfach bis dreifach, gleich oder verschieden durch Fluor, Chlor, Brom, Hydroxy, Mercapto, durch jeweils gegebenenfalls einfach bis fünffach, gleich oder verschieden durch Fluor oder Chlor substituiertes $C_1$-$C_8$-Alkyl, $C_1$-$C_4$-Alkoxy, $C_1$-$C_4$-Alkylthio, $C_5$-$C_7$-Cycloalkyl oder Phenyl substituiertes $C_1$-$C_5$-Alkandiyl oder $C_2$-$C_5$-Alkendiyl stehen, welches außerdem gegebenenfalls eine der nachstehenden Gruppierungen

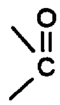 ; 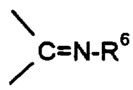 ; 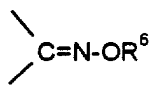 ; 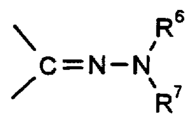 ;

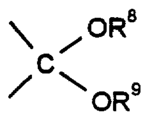 ; 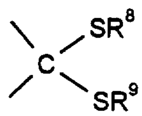 ; 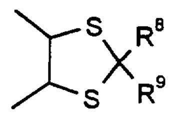 ;

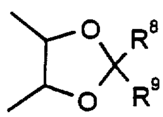 ; 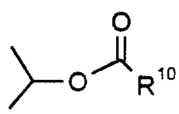 oder 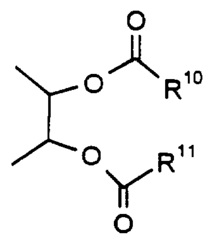

enthält oder durch eine $C_1$-$C_2$-Alkandiylgruppe überbrückt ist,

B und B' unabhängig voneinander für Wasserstoff, Fluor, Chlor oder $C_1$-$C_4$-Alkyl oder gemeinsam für jeweils gegebenenfalls durch $C_1$-$C_4$-Alkyl substituiertes $C_1$-$C_5$-Alkandiyl oder $C_4$-Alkendiyl stehen,

G für Wasserstoff (a) oder für eine der Gruppen

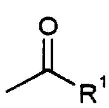 (b), 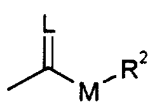 (c), 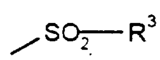 (d), 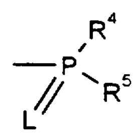 (e),

E (f)

oder

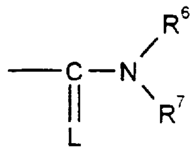

(g),

in welchen

E für ein Metallionäquivalent oder ein Ammoniumion steht,

L für Sauerstoff oder Schwefel steht,

M für Sauerstoff oder Schwefel steht,

$R^1$ für jeweils gegebenenfalls einfach bis neunfach, gleich oder verschieden durch Fluor oder Chlor substituiertes $C_1$-$C_{16}$-Alkyl, $C_2$-$C_8$-Alkenyl, $C_1$-$C_6$-Alkoxy-$C_1$-$C_6$-alkyl, $C_1$-$C_6$-Alkylthio-$C_1$-$C_6$-alkyl, Poly-$C_1$-$C_6$-alkoxy-$C_1$-$C_6$-alkyl oder für gegebenenfalls einfach bis dreifach, gleich oder verschieden durch Fluor, Chlor, $C_1$-$C_4$-Alkyl oder $C_1$-$C_4$-Alkoxy substituiertes Cycloalkyl mit 3 bis 7 Ringatomen, in welchem eine oder zwei Methylengruppen durch Sauerstoff- und/oder Schwefelatome ersetzt sein können,
für gegebenenfalls einfach bis dreifach, gleich oder verschieden durch Fluor, Chlor, Brom, Cyano, Nitro, $C_1$-$C_4$-Alkyl, $C_1$-$C_4$-Alkoxy, $C_1$-$C_3$-Halogenalkyl oder $C_1$-$C_3$-Halogenalkoxy substituiertes Phenyl,
für gegebenenfalls einfach bis dreifach, gleich oder verschieden durch Fluor, Chlor, Brom, $C_1$-$C_4$-Alkyl, $C_1$-$C_4$-Alkoxy, $C_1$-$C_3$-Halogenalkyl oder $C_1$-$C_3$-Halogenalkoxy substituiertes Phenyl-$C_1$-$C_4$-alkyl,
für jeweils gegebenenfalls einfach oder zweifach, gleich oder verschieden durch Fluor, Chlor, Brom oder $C_1$-$C_4$-Alkyl substituiertes Furanyl, Thienyl, Pyridyl, Pyrimidyl, Thiazolyl oder Pyrazolyl,
für gegebenenfalls einfach bis dreifach, gleich oder verschieden durch Fluor, Chlor oder $C_1$-$C_4$-Alkyl substituiertes Phenoxy-$C_1$-$C_5$-alkyl oder
für jeweils gegebenenfalls einfach oder zweifach, gleich oder verschieden durch Fluor, Chlor. Amino oder $C_1$-$C_4$-Alkyl substituiertes Pyridyloxy-$C_1$-$C_6$-alkyl, Pyrimidinyloxy-$C_1$-$C_6$-alkyl oder Thiazolyloxy-$C_1$-$C_6$-alkyl steht,

$R^2$ für jeweils gegebenenfalls einfach bis neunfach, gleich oder verschieden durch Fluor oder Chlor substituiertes $C_1$-$C_{16}$-Alkyl, $C_2$-$C_8$-Alkenyl, $C_1$-$C_6$-Alkoxy-$C_2$-$C_6$-alkyl, Poly-$C_1$-$C_6$-alkoxy-$C_2$-$C_6$-alkyl,
für gegebenenfalls einfach bis fünffach, gleich oder verschieden durch Fluor, Chlor, $C_1$-$C_4$-Alkyl oder $C_1$-$C_4$-Alkoxy substituiertes $C_3$-$C_6$-Cycloalkyl oder
für jeweils gegebenenfalls einfach bis dreifach, gleich oder verschieden durch Fluor, Chlor, Brom, Nitro, Cyano, $C_1$-$C_4$-Alkyl, $C_1$-$C_4$-Alkoxy, $C_1$-$C_2$-Halogenalkoxy oder $C_1$-$C_3$-Halogenalkyl substituiertes Phenyl oder Benzyl steht,

$R^3$ für gegebenenfalls einfach bis fünffach, gleich oder verschieden durch Fluor oder Chlor substituiertes $C_1$-$C_9$-Alkyl oder für jeweils gegebenenfalls einfach bis dreifach, gleich oder verschieden durch Fluor, Chlor, Brom, $C_1$-$C_4$-Alkyl, $C_1$-$C_4$-Alkoxy, $C_1$-$C_2$-Halogenalkyl, $C_1$-$C_2$-Halogenalkoxy, Cyano oder Nitro substituiertes Phenyl oder Phenyl-$C_1$-$C_2$-alkyl steht,

$R^4$ und $R^5$unabhängig voneinander für gegebenenfalls einfach bis fünffach, gleich oder verschieden durch Fluor oder Chlor substituiertes $C_1$-$C_6$-Alkyl, $C_1$-$C_6$-Alkoxy, $C_1$-$C_6$-Alkylamino, Di-($C_1$-$C_6$-alkyl)-amino, $C_1$-$C_6$-Alkylthio, $C_3$-$C_4$-Alkenylthio, $C_3$-$C_6$-Cycloalkylthio oder für jeweils gegebenenfalls einfach bis dreifach, gleich oder verschieden durch Fluor, Chlor, Brom, Nitro, Cyano. $C_1$-$C_3$-Alkoxy, $C_1$-$C_3$-Halogenalkoxy, $C_1$-$C_3$-Alkylthio, $C_1$-$C_3$-Halogenalkylthio, $C_1$-$C_3$-Alkyl oder $C_1$-$C_3$-Halogenalkyl substituiertes Phenyl, Phenoxy oder Phenylthio stehen,

$R^6$ für Wasserstoff, jeweils gegebenenfalls einfach bis fünffach, gleich oder verschieden durch Fluor oder Chlor substituiertes $C_1$-$C_8$-Alkyl, $C_3$-$C_6$-Alkenyl, $C_1$-$C_6$-Alkoxy-$C_2$-$C_6$-alkyl, für jeweils gegebenenfalls einfach bis dreifach, gleich oder verschieden durch Fluor, Chlor, $C_1$-$C_4$-Alkyl, $C_1$-$C_4$-Alkoxy, $C_1$-$C_2$-Halogenalkyl oder $C_1$-$C_2$-Halogenalkoxy substituiertes $C_3$-$C_8$-Cycloalkyl, für gegebenenfalls einfach bis dreifach, gleich oder verschieden durch Fluor, Chlor, Brom, $C_1$-$C_2$-Halogenalkyl, $C_1$-$C_5$-Alkyl, $C_1$-$C_2$-Halogenalkoxy oder $C_1$-$C_5$-Alkoxy substituiertes Phenyl oder für gegebenenfalls einfach bis dreifach, gleich oder verschieden durch Fluor, Chlor,

Brom, $C_1$-$C_5$-Alkyl, $C_1$-$C_2$-Halogenalkyl, $C_1$-$C_2$-Halogenalkoxy oder $C_1$-$C_5$-Alkoxy substituiertes Benzyl steht,

$R^7$ für Wasserstoff, jeweils gegebenenfalls einfach bis fünffach, gleich oder verschieden durch Fluor oder Chlor substituiertes $C_1$-$C_8$-Alkyl oder $C_3$-$C_8$-Alkenyl steht oder

$R^6$ und $R^7$ gemeinsam mit dem N-Atom, an das sie gebunden sind, für einen gegebenenfalls Sauerstoff oder Schwefel enthaltenden 4- bis 7-gliedrigen Ring stehen,

$R^8$ und $R^9$ unabhängig voneinander für Wasserstoff, gegebenenfalls einfach bis fünffach, gleich oder verschieden durch Fluor oder Chlor substituiertes $C_1$-$C_4$-Alkyl oder für gegebenenfalls einfach bis dreifach, gleich oder verschieden durch Fluor, Chlor, $C_1$-$C_4$-Alkyl, $C_1$-$C_4$-Alkoxy, $C_1$-$C_2$-Halogenalkyl, $C_1$-$C_2$-Halogenalkoxy, Nitro oder Cyano substituiertes Phenyl
oder zusammen für gegebenenfalls einfach bis dreifach, gleich oder verschieden durch Fluor, Chlor, $C_1$-$C_4$-Alkyl, $C_1$-$C_4$-Alkoxy oder $C_1$-$C_2$-Halogenalkyl substituiertes $C_2$-$C_5$-Alkandiyl stehen und

$R^{10}$ und $R^{11}$ unabhängig voneinander für jeweils gegebenenfalls einfach bis fünffach, gleich oder verschieden durch Fluor oder Chlor substituiertes $C_1$-$C_8$-Alkyl, $C_2$-$C_8$-Alkenyl, $C_1$-$C_8$-Alkoxy, $C_1$-$C_8$-Alkylamino, $C_3$-$C_8$-Alkenylamino, Di-($C_1$-$C_8$-alkyl)-amino oder Di-($C_3$-$C_8$-alkenyl)-amino stehen.

**5.** Verbindungen der Formel (I) gemäß Anspruch 1,
in welcher

A und Q gemeinsam für jeweils gegebenenfalls einfach oder zweifach, gleich oder verschieden durch Fluor. Chlor, Hydroxy, Mercapto, durch jeweils gegebenenfalls einfach bis dreifach, gleich oder verschieden durch Fluor oder Chlor substituiertes $C_1$-$C_6$-Alkyl, $C_1$-$C_2$-Alkoxy, $C_1$-$C_2$-Alkylthio, $C_5$-$C_6$-Cycloalkyl oder Phenyl substituiertes $C_1$-$C_4$-Alkandiyl oder $C_2$-$C_4$-Alkendiyl stehen, welches außerdem gegebenenfalls eine der nachstehenden Gruppen

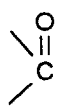 ; 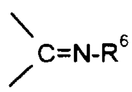 ; 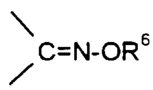 ; 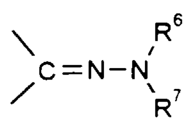 ;

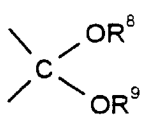 ; 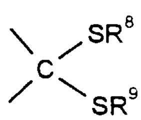 ; 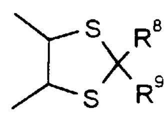 ;

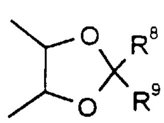 ; 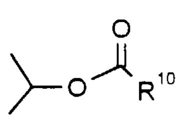 oder 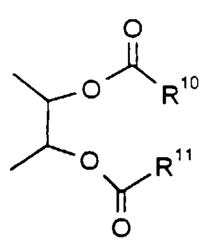

enthält oder durch eine $C_1$-$C_2$-Alkandiylgruppe überbrückt ist,

B und B' unabhängig voneinander für Wasserstoff, Fluor, Chlor, Methyl, Ethyl oder gemeinsam für jeweils gegebenenfalls durch Methyl oder Ethyl substituiertes $C_1$-$C_4$-Alkandiyl oder $C_4$-Alkendiyl stehen,

G für Wasserstoff (a) oder für eine der Gruppen

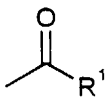 (b), 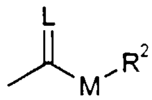 (c), 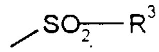 (d), 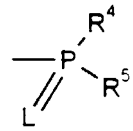 (e),

E (f)

oder

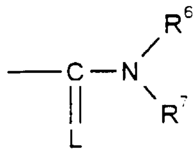 (g),

in welchen

E für ein Metallionäquivalent oder ein Ammoniumion steht,

L für Sauerstoff oder Schwefel steht,

M für Sauerstoff oder Schwefel steht.

$R^1$ für jeweils gegebenenfalls einfach bis fünffach, gleich oder verschieden durch Fluor oder Chlor substituiertes $C_1$-$C_{14}$-Alkyl, $C_2$-$C_6$-Alkenyl, $C_1$-$C_4$-Alkoxy-$C_1$-$C_6$-alkyl, $C_1$-$C_4$-Alkylthio-$C_1$-$C_6$-alkyl, Poly-$C_1$-$C_4$-alkoxy-$C_1$-$C_4$-alkyl oder gegebenenfalls einfach oder zweifach, gleich oder verschieden durch Fluor, Chlor, Methyl, Ethyl, Methoxy oder Ethoxy substituiertes Cycloalkyl mit 3 bis 6 Ringatomen, in welchem eine oder zwei Methylengruppen durch Sauerstoff- und/oder Schwefelatome ersetzt sein können,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl, Trifluormethoxy, Cyano oder Nitro substituiertes Phenyl,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl oder Trifluormethoxy substituiertes Benzyl,
für jeweils gegebenenfalls einfach oder zweifach, gleich oder verschieden durch Fluor, Chlor, Brom, Methyl oder Ethyl substituiertes Thienyl, Furanyl oder Pyridyl,
für gegebenenfalls einfach oder zweifach, gleich oder verschieden durch Fluor, Chlor, Methyl oder Ethyl substituiertes Phenoxy-$C_1$-$C_4$-alkyl oder
für jeweils gegebenenfalls einfach oder zweifach, gleich oder verschieden durch Fluor. Chlor. Amino, Methyl oder Ethyl substituiertes Pyridyloxy-$C_1$-$C_4$-alkyl, Pyrimidyloxy-$C_1$-$C_4$-alkyl oder Thiazolyloxy-$C_1$-$C_4$-alkyl steht,

$R^2$ für jeweils gegebenenfalls einfach bis fünffach, gleich oder verschieden durch Fluor oder Chlor substituiertes $C_1$-$C_{14}$-Alkyl, $C_2$-$C_6$-Alkenyl, $C_1$-$C_4$-Alkoxy-$C_2$-$C_6$-alkyl, Poly-$C_1$-$C_4$-alkoxy-$C_2$-$C_6$-alkyl, für gegebenenfalls einfach bis dreifach, gleich oder verschieden durch Fluor, Chlor, Methyl, Ethyl, Methoxy oder Ethoxy substituiertes $C_3$-$C_6$-Cycloalkyl
oder für jeweils gegebenenfalls einfach oder zweifach, gleich oder verschieden durch Fluor, Chlor, Nitro, Cyano, Methyl, Ethyl, Propyl, i-Propyl, Methoxy, Ethoxy, Trifluormethoxy oder Trifluormethyl substituiertes Phenyl oder Benzyl steht,

$R^3$ für gegebenenfalls einfach bis dreifach, gleich oder verschieden durch Fluor oder Chlor substituiertes $C_1$-$C_6$-Alkyl oder für jeweils gegebenenfalls einfach oder zweifach durch Fluor, Chlor, Brom, Methyl, Ethyl, Methoxy, Ethoxy, Trifluormethyl, Trifluormethoxy, Cyano oder Nitro substituiertes Phenyl oder Benzyl steht,

$R^4$ und $R^5$unabhängig voneinander für jeweils gegebenenfalls einfach bis dreifach, gleich oder verschieden durch

Fluor oder Chlor substituiertes $C_1$-$C_4$-Alkyl, $C_1$-$C_4$-Alkoxy, $C_1$-$C_4$-Alkylamino, Di-($C_1$-$C_4$-alkyl)-amino, $C_1$-$C_4$-Alkylthio oder für jeweils gegebenenfalls einfach oder zweifach, gleich oder verschieden durch Fluor, Chlor, Brom, Nitro, Cyano, $C_1$-$C_2$-Alkoxy, Trifluormethoxy, $C_1$-$C_2$-Alkylthio, Trifluormethyl oder $C_1$-$C_3$-Alkyl substituiertes Phenyl, Phenoxy oder Phenylthio stehen,

$R^6$ für Wasserstoff, jeweils gegebenenfalls einfach bis dreifach, gleich oder verschieden durch Fluor oder Chlor substituiertes $C_1$-$C_6$-Alkyl, $C_1$-$C_6$-Alkoxy-$C_2$-$C_4$-alkyl, für gegebenenfalls einfach oder zweifach, gleich oder verschieden durch Fluor, Chlor, Methyl, Methoxy, Trifluormethyl oder Trifluormethoxy substituiertes $C_3$-$C_6$-Cycloalkyl, für gegebenenfalls einfach oder zweifach, gleich oder verschieden durch Fluor, Chlor, Trifluormethyl, $C_1$-$C_4$-Alkyl, Trifluormethoxy oder $C_1$-$C_4$-Alkoxy substituiertes Phenyl oder für gegebenenfalls einfach oder zweifach, gleich oder verschieden durch Fluor, Chlor, $C_1$-$C_4$-Alkyl, Trifluormethyl, Trifluormethoxy oder $C_1$-$C_4$-Alkoxy substituiertes Benzyl steht,

$R^7$ für Wasserstoff, jeweils gegebenenfalls einfach bis dreifach, gleich oder verschieden durch Fluor oder Chlor substituiertes $C_1$-$C_6$-Alkyl oder $C_3$-$C_6$-Alkenyl steht oder

$R^6$ und $R^7$ gemeinsam mit dem N-Atom, an das sie gebunden sind, für einen gegebenenfalls Sauerstoff oder Schwefel enthaltenden 5- bis 7-gliedrigen Ring stehen,

$R^8$ und $R^9$ unabhängig voneinander für Wasserstoff, gegebenenfalls einfach bis dreifach, gleich oder verschieden durch Fluor oder Chlor substituiertes $C_1$-$C_4$-Alkyl oder für gegebenenfalls einfach oder zweifach, gleich oder verschieden durch Fluor, Chlor, Methyl, Ethyl, Methoxy, Ethoxy, Trifluormethyl, Trifluormethoxy, Nitro oder Cyano substituiertes Phenyl
oder zusammen für gegebenenfalls einfach oder zweifach, gleich oder verschieden durch Fluor, Chlor, Methyl, Ethyl, Methoxy, Ethoxy oder Trifluormethyl substituiertes $C_2$-$C_5$-Alkandiyl stehen und

$R^{10}$ und $R^{11}$ unabhängig voneinander für $C_1$-$C_6$-Alkyl oder $C_2$-$C_6$-Alkenyl stehen.

**6.** Verfahren zur Herstellung von Verbindungen der Formel (I) gemäß Anspruch 1, dadurch gekennzeichnet, daß man zur Herstellung von

(A) Verbindungen der Formel (Ia)

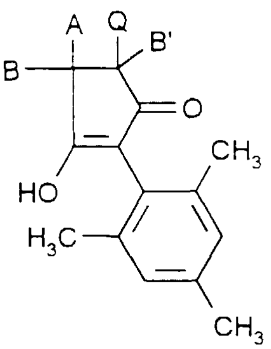

(Ia)

in welcher
A, B, B' und Q die in Anspruch 1 angegebene Bedeutung haben,
Verbindungen der Formel (II)

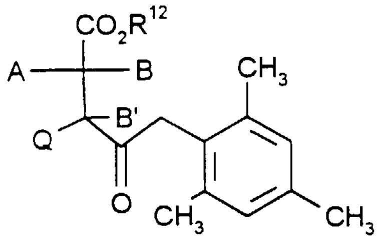

(II)

in welcher
A, B, B' und Q die oben angegebene Bedeutung haben, und
$R^{12}$ für Alkyl steht,
in Gegenwart eines Verdünnungsmittels und in Gegenwart einer Base intramolekular kondensiert;
oder
(B) der Formel (Ib)

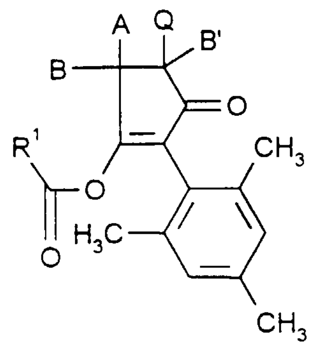

(Ib)

in welcher
A, B, B', Q und $R^1$ die in Anspruch 1 angegebene Bedeutung haben,
Verbindungen der Formel (Ia),

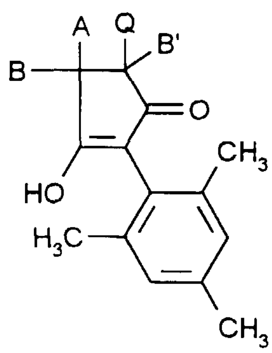

(Ia)

in welcher
A, B, B' und Q die oben angegebene Bedeutung haben,

α) mit Säurehalogeniden der Formel (III)

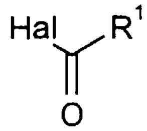

(III)

in welcher

$R^1$ die oben angegebene Bedeutung hat und
Hal für Halogen steht,

gegebenenfalls in Gegenwart eines Verdünnungsmittels und gegebenenfalls in Gegenwart eines Säurebindemittels umsetzt
oder
β) mit Carbonsäureanhydriden der Formel (IV)

$$R^1\text{-CO-O-CO-}R^1 \quad \text{(IV)}$$

in welcher

$R^1$ die oben angegebene Bedeutung hat,

gegebenenfalls in Gegenwart eines Verdünnungsmittels und gegebenenfalls in Gegenwart eines Säurebindemittels umsetzt;

oder
(C) der Formel (Ic-1)

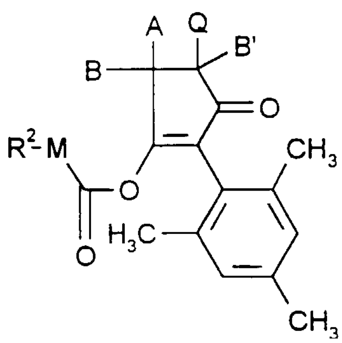

(Ic-1)

in welcher
A, B, B', Q und $R^2$ die in Anspruch 1 angegebene Bedeutung haben,
und

M für Sauerstoff oder Schwefel steht.

Verbindungen der Formel (Ia)

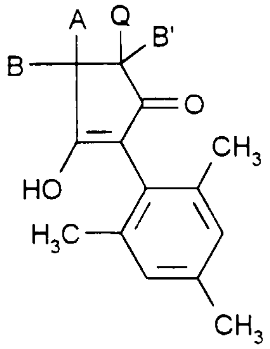

(Ia)

in welcher

A, B, B' und Q die oben angegebene Bedeutung haben,

mit Chlorameisensäureester oder Chlorameisensäurethiolester der Formel (V)

$$R^2\text{-M-CO-Cl} \quad (V)$$

in welcher
$R^2$ und M die oben angegebene Bedeutung haben,
gegebenenfalls in Gegenwart eines Verdünnungsmittels und gegebenenfalls in Gegenwart eines Säurebindemittels umsetzt;
oder
(D) der Formel (Ic-2)

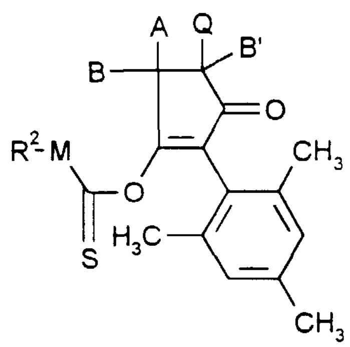

(Ic-2)

in welcher
A, B, B', Q und $R^2$ die oben angegebene Bedeutung haben
und

M für Sauerstoff oder Schwefel steht.

Verbindungen der Formel (Ia)

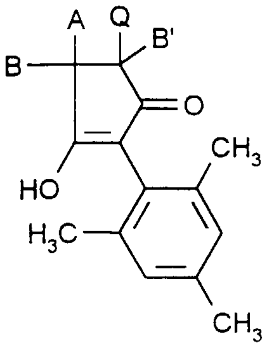

(Ia)

in welcher

A, B, B' und Q die oben angegebene Bedeutung haben,

α) mit Chlormonothioameisensäureestern oder Chlordithioameisensäureestern der Formel (VI)

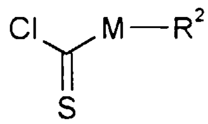

(VI)

in welcher

M und $R^2$ die oben angegebene Bedeutung haben,

gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der allgemeinen Formel (VII)

$$R^2\text{-Hal} \quad \text{(VII)}$$

in welcher

$R^2$ die oben angegebene Bedeutung hat

und

Hal für Chlor, Brom oder Iod steht,

gegebenenfalls in Gegenwart eines Verdünnungsmittels und gegebenenfalls in Gegenwart einer Base umsetzt;

oder
(E) der Formel (Id)

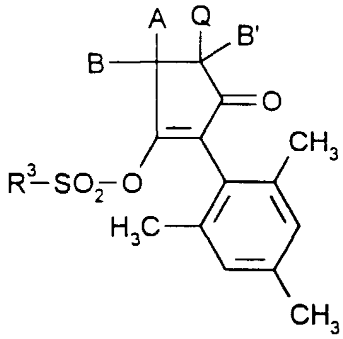

. (Id)

in welcher
A, B, B', Q und $R^3$ die in Anspruch 1 angegebene Bedeutung haben,
Verbindungen der Formel (Ia)

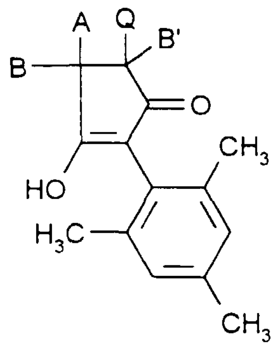

(Ia)

in welcher
A, B, B' und Q die oben angegebene Bedeutung haben,
mit Sulfonsäurechloriden der Formel (VIII)

$$R^3\text{-}SO_2\text{-}Cl \qquad \text{(VIII)}$$

in welcher

$R^3$ die oben angegebene Bedeutung hat,

gegebenenfalls in Gegenwart eines Verdünnungsmittels und gegebenenfalls in Gegenwart eines Säurebindemittels umsetzt;
oder
(F) der Formel (Ie)

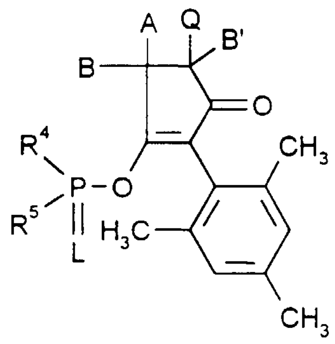

(Ie)

in welcher
A, B, L, B', Q, $R^4$ und $R^5$ die in Anspruch 1 angegebene Bedeutung haben,
Verbindungen der Formel (Ia) bzw, deren Enole

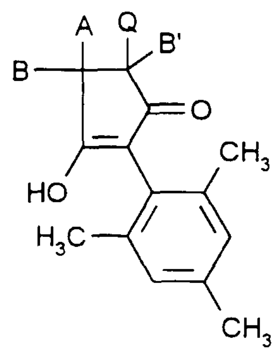

(Ia)

in welcher
A, B, B' und Q die oben angegebene Bedeutung haben,
mit Phosphorverbindungen der Formel (IX)

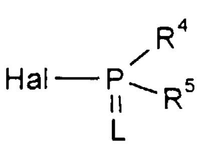

(IX)

in welcher
L, $R^4$ und $R^5$ die oben angegebene Bedeutung haben
und

Hal für Halogen steht,

gegebenenfalls in Gegenwart eines Verdünnungsmittels und gegebenenfalls in Gegenwart eines Säurebindemittels umsetzt;
oder
(G) der Formel (If)

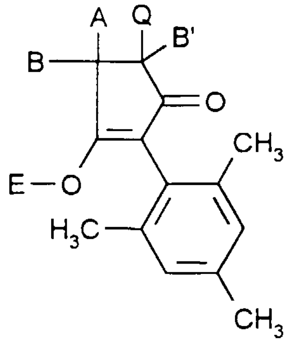

(If)

in welcher
A, B, B' und Q die oben angegebene Bedeutung haben,
und

E für ein Metallionäquivalent oder für ein Ammoniumion steht,

Verbindungen der Formel (Ia)

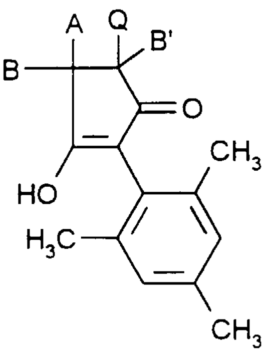

(Ia)

in welcher
A, B, B' und Q die oben angegebene Bedeutung haben,
mit Metall-Verbindungen oder Aminen der Formeln (X) und (XI)

$$Me\ R_t^{16} \quad (X)$$

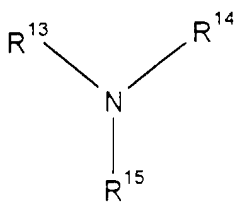

(XI)

in welchen

| | |
|---|---|
| Me | für ein- oder zweiwertige Metallionen steht, |
| t | für die Zahl 1 oder 2 steht, |
| $R^{13}$, $R^{14}$ und $R^{15}$ | unabhängig voneinander für Wasserstoff oder Alkyl stehen und |

$R^{16}$ für Wasserstoff, Hydroxy oder $C_1$-$C_4$-Alkoxy steht,

gegebenenfalls in Gegenwart eines Verdünnungsmittels umsetzt,
oder
(H) der Formel (Ig)

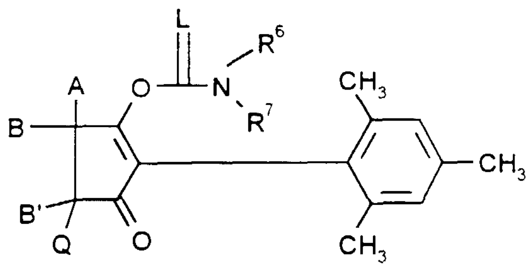

(Ig)

in welcher
A, B, L, B', Q, $R^6$ und $R^7$ die in Anspruch 1 angegebene Bedeutung haben,
Verbindungen der Formel (Ia)

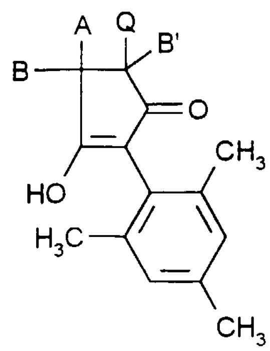

(Ia)

in welcher
A, B, B' und Q die oben angegebene Bedeutung haben,

α) mit Verbindungen der Formel (XII)

$$R^6\text{-N=C=L} \quad \text{(XII)}$$

in welcher
L und $R^6$ die oben angegebene Bedeutung haben,
gegebenenfalls in Gegenwart eines Verdünnungsmittels und gegebenenfalls in Gegenwart eines Katalysators
oder
β) mit Carbamidsäurechloriden oder Thiocarbamidsäurechloriden der Formel (XIII)

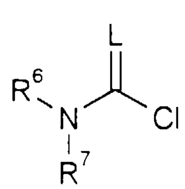

(XIII)

in welcher
L, $R^6$ und $R^7$ die oben angegebene Bedeutung haben,
gegebenenfalls in Gegenwart eines Verdünnungsmittels und gegebenenfalls in Gegenwart eines Säurebindemittels umsetzt.

**7.** Verbindungen der Formel (II)

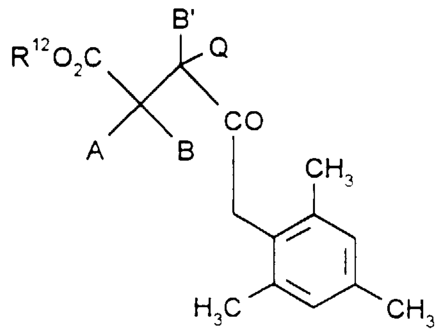

(II)

in welcher

A, B, B' und Q die in Anspruch 1 angegebene Bedeutung haben und
$R^{12}$ für Alkyl steht.

**8.** Verfahren zur Herstellung der Verbindungen der Formel (II) gemäß Anspruch 7, dadurch gekennzeichnet, daß man Verbindungen der Formel (XIV)

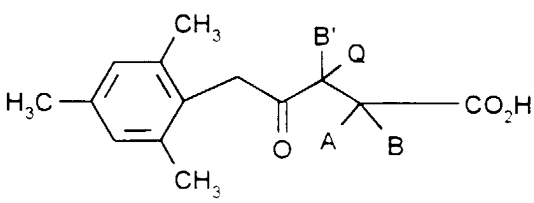

(XIV)

in welcher
A, B, B' und Q die in Anspruch 1 angegebene Bedeutung haben,
verestert

**9.** Verbindungen der Formel (XIV)

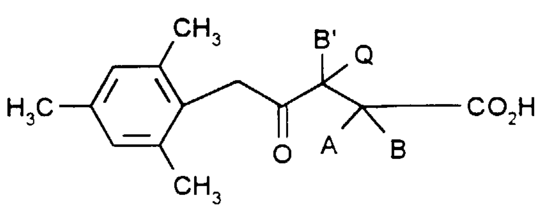

(XIV)

in welcher
A, B, B' und Q die in Anspruch 1 angegebene Bedeutung haben.

**10.** Verfahren zur Herstellung von Verbindungen der Formel (XIV), dadurch gekennzeichnet, daß man Carbonsäureanhydride der Formel (XV)

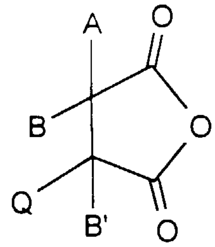

(XV)

in welcher
A, B, B' und Q die in Anspruch 1 angegebene Bedeutung haben,
mit metallorganischen Verbindungen der Formel (XVI)

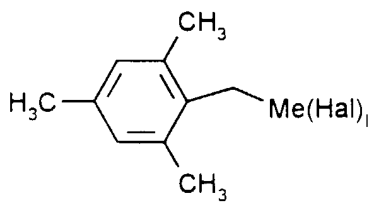

(XVI)

in welcher

Me für ein- oder zweiwertige Metallionen,
Hal für Chlor oder Brom

und

l für eine Zahl 0 oder 1 steht,

in Gegenwart eines Verdünnungsmittels umsetzt,
oder daß man Verbindungen der Formel (XVII)

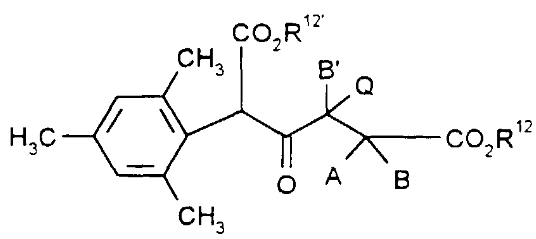

(XVII)

in welcher
A, B, B' und Q die oben angegebene Bedeutung haben und
$R^{12}$ und $R^{12'}$ für Alkyl stehen,
gegebenenfalls in Gegenwart eines Verdünnungsmittels und gegebenenfalls in Gegenwart einer Base oder Säure decarboxyliert

**11.** Verbindungen der Formel (XVII)

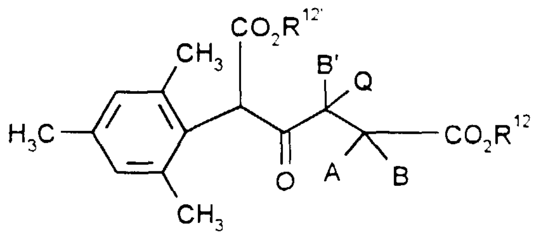

(XVII)

in welcher
A, B, B', Q, $R^{12}$ und $R^{12'}$ die in Anspruch 10 angegebene Bedeutung haben.

**12.** Verfahren zur Herstellung von Verbindungen der Formel (XVII) gemäß Anspruch 11, dadurch gekennzeichnet, daß man Dicarbonsaurehalbesterchloride der Formel (XVIII)

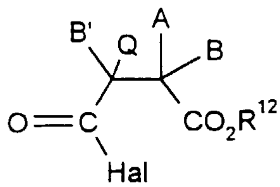

(XVIII)

in welcher

| | |
|---|---|
| A, B, B', Q und $R^{12}$ | die oben angegebene Bedeutung haben und |
| Hal | für Chlor oder Brom steht, |

mit einem substituierten Phenylessigsäureester der Formel (XIX)

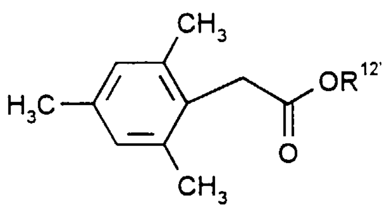

(XIX)

in welcher

$R^{12'}$ für Alkyl steht,

in Gegenwart eines Verdünnungsmittels und in Gegenwart einer Base acyliert.

**13.** Schädlingsbekämpfungsmittel und Herbizide. gekennzeichnet durch einen Gehalt an mindestens einer Verbindung der Formel (I) gemäß Anspruch 1

**14.** Verwendung von Verbindungen der Formel (I) gemäß Anspruch 1 zur Bekämpfung von tierischen Schädlingen und unerwünschtem Pflanzenbewuchs.

**15.** Verfahren zur Bekämpfung von tierischen Schädlingen und unerwünschtem Pflanzenbewuchs, dadurch gekennzeichnet, daß man Verbindungen der Formel (I) gemäß Anspruch 1 auf die Schädlinge, Pflanzen und/oder deren Lebensraum ausbringt.

**16.** Verfahren zur Herstellung von Schädlingsbekämpfungsmitteln und Herbiziden, dadurch gekennzeichnet, daß man

Verbindungen der Formel (I) gemäß Anspruch 1 mit Streckmitteln und/oder oberflächenaktiven Stoffen vermischt.

**17.** Verwendung von Verbindungen der Formel (I) gemäß Anspruch 1 zur Herstellung von Schädlingsbekämpfungsmitteln und Herbiziden.

## Claims

**1.** Compounds of the formula (I)

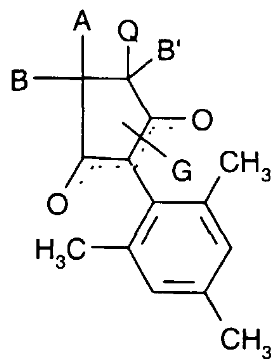

(I)

in which

A and Q together represent alkanediyl or alkenediyl, which is in each case optionally substituted by halogen, hydroxyl, mercapto or in each case optionally substituted alkyl, alkoxy, alkylthio, cycloalkyl, benzyloxy or aryl, and which furthermore optionally contains one of the following groups

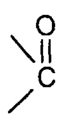 ; 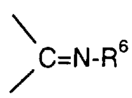 ; 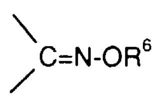 ; 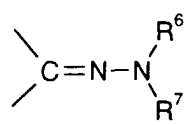 ;

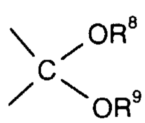 ; 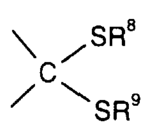 ; 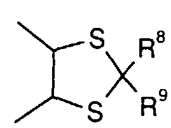 ;

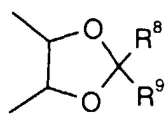 ; 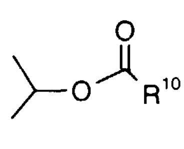 or 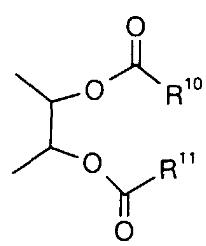

or is bridged by an alkanediyl group,

B and B' independently of one another represent hydrogen, halogen or alkyl, or together represent in each case optionally substituted alkanediyl or alkenediyl,

G represents hydrogen (a), or represents one of the groups

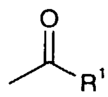 (b), 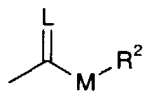 (c), 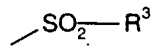 (d), 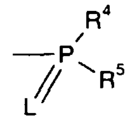 (e),

E (f)

or

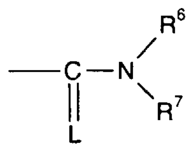 (g),

in which

E represents a metal ion equivalent or an ammonium ion,

L represents oxygen or sulphur and

M represents oxygen or sulphur,

$R^1$ represents in each case optionally substituted alkyl, alkenyl. alkoxyalkyl, alkylthioalkyl, polyalkoxyalkyl or cycloalkyl, which can contain at least one heteroatom, or in each case optionally substituted phenyl, phenylalkyl, hetaryl, phenoxyalkyl or hetaryloxyalkyl,

$R^2$ represents in each case optionally substituted alkyl, cycloalkyl, alkenyl, alkoxyalkyl or polyalkoxyalkyl, or in each case optionally substituted phenyl or benzyl,

$R^3$ represents in each case optionally substituted alkyl, phenyl or phenylalkyl,

$R^4$ and $R^5$ independently of one another represent in each case optionally halogen-substituted alkyl, alkoxy, alkylamino, dialkylamino, alkylthio, alkenylthio, cycloalkylthio, or represent in each case optionally substituted phenyl, phenoxy or phenylthio,

$R^6$ represents hydrogen or in each case optionally halogen-substituted alkyl, alkenyl or alkoxyalkyl, or represents in each case optionally substituted cycloalkyl or phenyl, or represents optionally substituted benzyl,

$R^7$ represents hydrogen or in each case optionally halogen-substituted alkyl or alkenyl, or

$R^6$ and $R^7$, together with the N atom to which they are bonded, represent a ring which optionally contains oxygen or sulphur,

$R^8$ and $R^9$ independently of one another represent hydrogen or in each case optionally substituted alkyl, phenyl or phenylalkyl, or together represent an optionally substituted alkanediyl radical and

$R^{10}$ and $R^{11}$ independently of one another represent in each case optionally halogen-substituted alkyl, alkenyl, alkoxy, alkylamino, dialkylamino, alkenylamino or dialkenylamino or in each case optionally substituted phenyl

or benzyl.

2. Compounds of the formula (I) according to Claim 1 which, incorporating the various meanings (a), (b), (c), (d), (e), (f) and (g) of the group G, have the following structures (Ia) to (Ig):

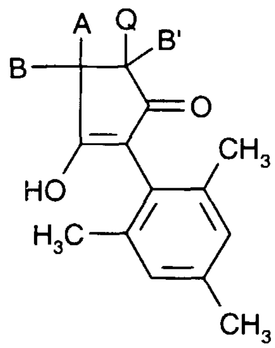

(Ia)

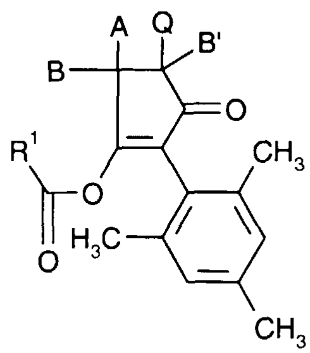

(Ib)

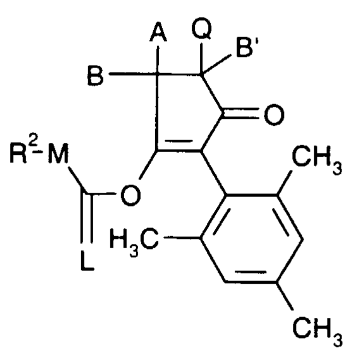

(Ic)

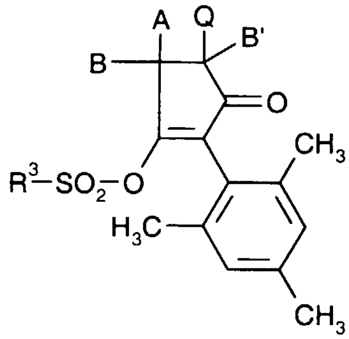

(Id)

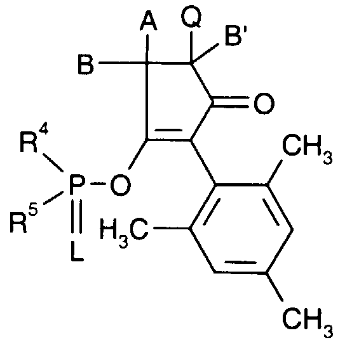

(Ie)

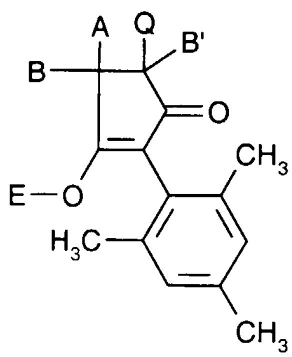

(If)

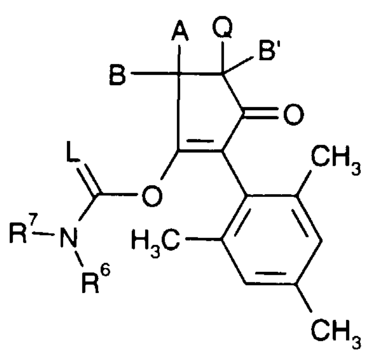

(Ig)

wherein

A, B, B', E, L, M, Q, $R^1$, $R^2$, $R^3$, $R^4$, $R^5$, $R^6$ and $R^7$ have the abovementioned meanings.

**3.** Compounds of the formula (I) according to Claim 1, in which

A and Q together represent $C_1$-$C_6$-alkanediyl or $C_2$-$C_6$-alkenediyl, which is in each case optionally substituted once to three times in an identical or different manner by halogen, hydroxyl or mercapto, or by $C_1$-$C_{10}$-alkyl, $C_1$-$C_6$-alkoxy, $C_1$-$C_6$-alkylthio or $C_3$-$C_7$-cycloalkyl, in each case optionally halogen-substituted once to nine times in an identical or different manner, or by benzyloxy or phenyl, in each case optionally substituted once to five times in an identical or different manner by halogen, $C_1$-$C_6$-alkyl or $C_1$-$C_6$-alkoxy, and which furthermore optionally contains one of the following groupings

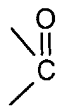 ; 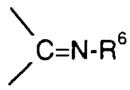 ; 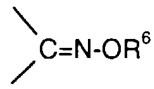 ; 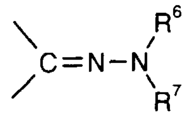 ;

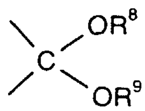 ; 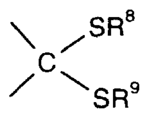 ; 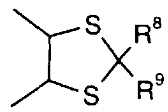 ;

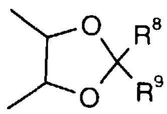 ; 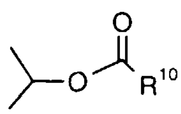 or 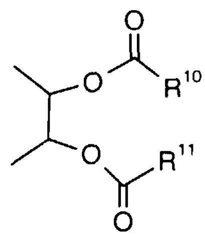

or is bridged by a $C_1$-$C_2$-alkanediyl group,

B and B' independently of one another represent hydrogen, halogen or $C_1$-$C_6$-alkyl, or together represent $C_1$-$C_6$-alkanediyl or $C_4$-alkenediyl, in each case optionally substituted by $C_1$-$C_6$-alkyl,

G represents hydrogen (a), or represents one of the groups

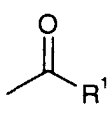 (b), 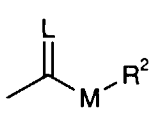 (c), 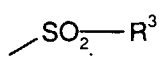 (d), 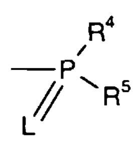 (e),

E (f)

or

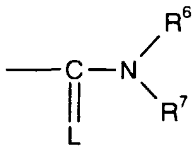

(g),

in which

E represents a metal ion equivalent or an ammonium ion,

L represents oxygen or sulphur and

M represents oxygen or sulphur,

$R^1$ represents $C_1$-$C_{20}$-alkyl, $C_2$-$C_{10}$-alkenyl, $C_1$-$C_8$-alkoxy-$C_1$-$C_8$-alkyl, $C_1$-$C_8$-alkylthio-$C_1$-$C_8$-alkyl, or poly-$C_1$-$C_8$-alkoxy-$C_1$-$C_8$-alkyl, in each case optionally substituted once or several times in an identical or different manner by halogen, or represents cycloalkyl having 3 to 8 ring atoms, which is optionally substituted once or several times in an identical or different manner by halogen, $C_1$-$C_6$-alkyl or $C_1$-$C_6$-alkoxy and in which at least one methylene group can be replaced by an oxygen and/or sulphur atom,
or represents phenyl which is optionally substituted once or several times in an identical or different manner by halogen, nitro, cyano, $C_1$-$C_6$-alkyl, $C_1$-$C_6$-alkoxy, $C_1$-$C_6$-halogenoalkyl or $C_1$-$C_6$-halogenoalkoxy,
or represents phenyl-$C_1$-$C_6$-alkyl which is optionally substituted once or several times in an identical or different manner by halogen, $C_1$-$C_6$-alkyl, $C_1$-$C_6$-alkoxy, $C_1$-$C_6$-halogenoalkyl or $C_1$-$C_6$-halogenoalkoxy,
or represents hetaryl which has 5 or 6 ring atoms and is optionally substituted once or several times in an identical or different manner by halogen or $C_1$-$C_6$-alkyl,
or represents phenoxy-$C_1$-$C_6$-alkyl which is optionally substituted once or several times in an identical or different manner by halogen or $C_1$-$C_6$-alkyl,
or represents hetaryloxy-$C_1$-$C_6$-alkyl which has 5 or 6 ring atoms and is optionally substituted once or several times in an identical or different manner by halogen, amino or $C_1$-$C_6$-alkyl,

$R^2$ represents $C_1$-$C_{20}$-a]kyl, $C_2$-$C_{10}$-alkeny], $C_1$-$C_8$-alkoxy-$C_2$-$C_8$-alkyl, or poly-$C_1$-$C_8$-alkoxy-$C_2$-$C_8$-alkyl, in each case optionally substituted once or several times in an identical or different manner by halogen,
or represents $C_3$-$C_6$-cycloalkyl which is optionally substituted once or several times in an identical or different manner by halogen, $C_1$-$C_6$-alkyl or $C_1$-$C_6$-alkoxy,
or represents phenyl or benzyl, in each case optionally substituted once or several times in an identical or different manner by halogen, nitro, cyano, $C_1$-$C_6$-alkyl, $C_1$-$C_6$-alkoxy, $C_1$-$C_3$-halogenoalkoxy or $C_1$-$C_3$-halogenoalkyl,

$R^3$ represents $C_1$-$C_{12}$-alkyl which is optionally substituted once or several times in an identical or different manner by halogen, or represents phenyl or phenyl-$C_1$-$C_4$-alkyl, in each case optionally substituted once or several times in an identical or different manner by halogen, $C_1$-$C_6$-alkyl, $C_1$-$C_6$-alkoxy, $C_1$-$C_3$-halogenoalkyl, $C_1$-$C_3$-halogenoalkoxy, cyano or nitro,

$R^4$ and $R^5$independently of one another represent $C_1$-$C_8$-alkyl, $C_1$-$C_8$-alkoxy, $C_1$-$C_8$-alkylamino, di-($C_1$-$C_8$-alkyl)-amino, $C_1$-$C_8$-alkylthio, $C_3$-$C_5$-alkenylthio, or $C_3$-$C_7$-cycloalkylthio, in each case optionally substituted once or several times in an identical or different manner by halogen, or represent phenyl, phenoxy or phenylthio, in each case optionally substituted once or several times in an identical or different manner by halogen, nitro, cyano, $C_1$-$C_4$-alkoxy, $C_1$-$C_4$-halogenoalkoxy, $C_1$-$C_4$-alkylthio, $C_1$-$C_4$-halogenoalkylthio, $C_1$-$C_4$-alkyl or $C_1$-$C_4$-halogenoalkyl,

$R^6$ represents hydrogen or $C_1$-$C_{10}$-alkyl, $C_3$-$C_8$-alkenyl or $C_1$-$C_8$-alkoxy-$C_2$-$C_8$-alkyl, in each case optionally substituted once or several times in an identical or different manner by halogen, or represents $C_3$-$C_{10}$-cycloalkyl which is optionally substituted once or several times in an identical or different manner by halogen, $C_1$-$C_6$-alkyl, $C_1$-$C_6$-alkoxy, $C_1$-$C_3$-halogenoalkyl or $C_1$-$C_3$-halogenoalkoxy, or represents phenyl which is optionally substituted once or several times in an identical or different manner by halogen, $C_1$-$C_3$-halogenoalkyl, $C_1$-$C_8$-alkyl, $C_1$-$C_3$-halogenoalkoxy or $C_1$-$C_8$-alkoxy, or represents benzyl which is optionally substituted once or several times in an identical or different manner by halogen, $C_1$-$C_8$-alkyl, $C_1$-$C_3$-halogenoalkyl, $C_1$-$C_3$-halog-

enoalkoxy or $C_1$-$C_8$-alkoxy,

$R^7$ represents hydrogen or $C_1$-$C_{10}$-alkyl or $C_3$-$C_{10}$-alkenyl, in each case optionally substituted once or several times in an identical or different manner by halogen, or

$R^6$ and $R^7$,together with the N atom to which they are bonded, represent a 3- to 7-membered ring which optionally contains oxygen or sulphur,

$R^8$ and $R^9$independently of one another represent hydrogen or $C_1$-$C_6$-alkyl which is optionally substituted once or several times in an identical or different manner by halogen, or represent phenyl or phenyl-$C_1$-$C_4$-alkyl, in each case optionally substituted once or several times in an identical or different manner by halogen, $C_1$-$C_6$-alkyl, $C_1$-$C_6$-alkoxy, $C_1$-$C_6$-halogenoalkyl, $C_1$-$C_6$-halogenoalkoxy, nitro or cyano,
or together represent $C_2$-$C_6$-alkanediyl which is optionally substituted once or several times in an identical or different manner by halogen, $C_1$-$C_6$-alkyl, $C_1$-$C_6$-alkoxy or $C_1$-$C_3$-halogenoalkyl and

$R^{10}$ and $R^{11}$independently of one another represent $C_1$-$C_{10}$-alkyl, $C_2$-$C_{10}$-alkenyl, $C_1$-$C_{10}$-alkoxy, $C_1$-$C_{10}$-alkylamino or di-($C_1$-$C_{10}$-alkyl)-amino, $C_3$-$C_{10}$-alkenylamino or di-($C_3$-$C_{10}$-alkenyl)-amino, in each case optionally substituted once or several times in an identical or different manner by halogen, or phenyl or benzyl, in each case substituted once or several times in an identical or different manner by halogen, $C_1$-$C_4$-alkyl, $C_1$-$C_4$-alkoxy, $C_1$-$C_4$-halogenoalkyl, nitro or cyano.

**4.** Compounds of the formula (I) according to Claim 1, in which

A and Q together represent $C_1$-$C_5$-alkanediyl or $C_2$-$C_5$-alkenediyl, which is in each case optionally substituted once to three times in an identical or different manner by fluorine, chlorine, bromine, hydroxyl or mercapto, or by $C_1$-$C_8$-alkyl, $C_1$-$C_4$-alkoxy, $C_1$-$C_4$-alkylthio, $C_5$-$C_7$-cycloalkyl or phenyl, in each case optionally substituted once to five times in an identical or different manner by fluorine or chlorine, and which furthermore optionally contains one of the following groupings:

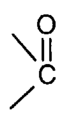 ; 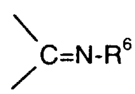 ; 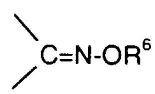 ; 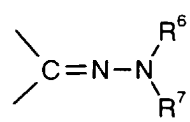 ;

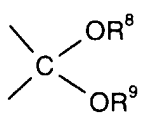 ; 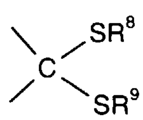 ; 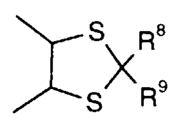 ;

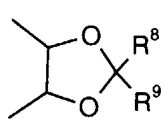 ; 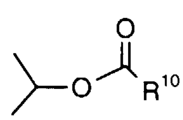 or 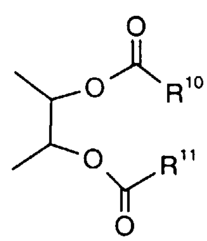

or is bridged by a $C_1$-$C_2$-alkanediyl group,

B and B' independently of one another represent hydrogen, fluorine, chlorine or $C_1$-$C_4$-alkyl, or together represent $C_1$-$C_5$-alkanediyl or $C_4$-alkenediyl, in each case optionally substituted by $C_1$-$C_4$-alkyl,

G represents hydrogen (a) or represents one of the groups

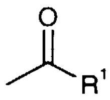 (b), 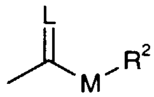 (c), 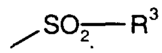 (d), 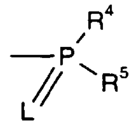 (e),

E (f)

or

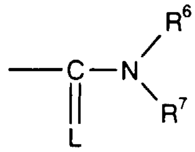 (g),

in which

E represents a metal ion equivalent or an ammonium ion,
L represents oxygen or sulphur and
M represents oxygen or sulphur,

$R^1$ represents $C_1$-$C_{16}$-alkyl, $C_2$-$C_8$-alkenyl, $C_1$-$C_6$-alkoxy-$C_1$-$C_6$-alkyl, $C_1$-$C_6$-alkylthio-$C_1$-$C_6$-alkyl, or poly-$C_1$-$C_6$-alkoxy-$C_1$-$C_6$-alkyl, in each case optionally substituted once to nine times in an identical or different manner by fluorine or chlorine, or represents cycloalkyl which has 3 to 7 ring atoms and is optionally substituted once to three times in an identical or different manner by fluorine, chlorine, $C_1$-$C_4$-alkyl or $C_1$-$C_4$-alkoxy, and in which one or two methylene groups can be replaced by oxygen and/or sulphur atoms,
or represents phenyl which is optionally substituted once to three times in an identical or different manner by fluorine, chlorine, bromine, cyano, nitro, $C_1$-$C_4$-alkyl, $C_1$-$C_4$-alkoxy, $C_1$-$C_3$-halogenoalkyl or $C_1$-$C_3$-halogenoalkoxy,
or represents phenyl-$C_1$-$C_4$-alkyl which is optionally substituted once to three times in an identical or different manner by fluorine, chlorine, bromine, $C_1$-$C_4$-alkyl, $C_1$-$C_4$-alkoxy, $C_1$-$C_3$-halogenoalkyl or $C_1$-$C_3$-halogenoalkoxy,
or represents furanyl, thienyl, pyridyl, pyrimidyl, thiazolyl or pyrazolyl, in each case optionally substituted once or twice in an identical or different manner by fluorine, chlorine, bromine or $C_1$-$C_4$-alkyl,
or represents phenoxy-$C_1$-$C_5$-alkyl which is optionally substituted once to three times in an identical or different manner by fluorine, chlorine or $C_1$-$C_4$-alkyl,
or represents pyridyloxy-$C_1$-$C_6$-alkyl, pyrimidinyloxy-$C_1$-$C_6$-alkyl or thiazolyloxy-$C_1$-$C_6$-alkyl, in each case optionally substituted once or twice in an identical or different manner by fluorine, chlorine, amino or $C_1$-$C_4$-alkyl,

$R^2$ represents $C_1$-$C_{16}$-alkyl, $C_2$-$C_8$-alkenyl, $C_1$-$C_6$-alkoxy-$C_2$-$C_6$-alkyl, or poly-$C_1$-$C_6$-alkoxy-$C_2$-$C_6$-alkyl, in each case optionally substituted once to nine times in an identical or different manner by fluorine or chlorine,
or represents $C_3$-$C_6$-cycloalkyl which is optionally substituted once to five times in an identical or different manner by fluorine, chlorine, $C_1$-$C_4$-alkyl or $C_1$-$C_4$-alkoxy,
or represents phenyl or benzyl, in each case optionally substituted once to three times in an identical or different manner by fluorine, chlorine, bromine, nitro, cyano, $C_1$-$C_4$-alkyl, $C_1$-$C_4$-alkoxy, $C_1$-$C_2$-halogenoalkoxy or $C_1$-$C_2$-halogenoalkyl,

$R^3$ represents $C_1$-$C_9$-alkyl which is optionally substituted once to five times in an identical or different manner by fluorine or chlorine, or represents phenyl or phenyl-$C_1$-$C_2$-alkyl, in each case optionally substituted once to three times in an identical or different manner by fluorine, chlorine, bromine, $C_1$-$C_4$-alkyl, $C_1$-$C_4$-alkoxy, $C_1$-$C_2$-halogenoalkyl, $C_1$-$C_2$-halogenoalkoxy, cyano or nitro,

$R^4$ and $R^5$ independently of one another represent $C_1$-$C_6$-alkyl, $C_1$-$C_6$-alkoxy, $C_1$-$C_6$-alkylamino, di-($C_1$-$C_6$-alkyl)-amino, $C_1$-$C_6$-alkylthio, $C_3$-$C_4$-alkenylthio, or $C_3$-$C_6$-cycloalkylthio, optionally substituted once to five times in an identical or different manner by fluorine or chlorine, or represent phenyl, phenoxy or phenylthio, in each case optionally substituted once to three times in an identical or different manner by fluorine, chlorine, bromine, nitro, cyano, $C_1$-$C_3$-alkoxy, $C_1$-$C_3$-halogenoalkoxy, $C_1$-$C_3$-alkylthio, $C_1$-$C_3$-halogenoalkylthio, $C_1$-$C_3$-alkyl or $C_1$-$C_3$-halogenoalkyl,

$R^6$ represents hydrogen or $C_1$-$C_8$-alkyl, $C_3$-$C_6$-alkenyl or $C_1$-$C_6$-alkoxy-$C_2$-$C_6$-alkyl, in each case optionally substituted once to five times in an identical or different manner by fluorine or chlorine, or represents $C_3$-$C_8$-cycloalkyl which is in each case optionally substituted once to three times in an identical or different manner by fluorine, chlorine, $C_1$-$C_4$-alkyl, $C_1$-$C_4$-alkoxy, $C_1$-$C_2$-halogenoalkyl or $C_1$-$C_2$-halogenoalkoxy, or represents phenyl which is optionally substituted once to three times in an identical or different manner by fluorine, chlorine, bromine, $C_1$-$C_2$-halogenoalkyl, $C_1$-$C_5$-alkyl, $C_1$-$C_2$-halogenoalkoxy or $C_1$-$C_5$-alkoxy, or represents benzyl which is optionally substituted once to three times in an identical or different manner by fluorine, chlorine, bromine, $C_1$-$C_5$-alkyl, $C_1$-$C_2$-halogenoalkyl, $C_1$-$C_2$-halogenoalkoxy or $C_1$-$C_5$-alkoxy,

$R^7$ represents hydrogen or $C_1$-$C_8$-alkyl or $C_3$-$C_8$-alkenyl, in each case optionally substituted once to five times in an identical or different manner by fluorine or chlorine, or

$R^6$ and $R^7$, together with the N atom to which they are bonded, represent a 4-to 7-membered ring which optionally contains oxygen or sulphur,

$R^8$ and $R^9$ independently of one another represent hydrogen or $C_1$-$C_4$-alkyl which is optionally substituted once to five times in an identical or different manner by fluorine or chlorine, or represent phenyl which is optionally substituted once to three times in an identical or different manner by fluorine, chlorine, $C_1$-$C_4$-alkyl, $C_1$-$C_4$-alkoxy, $C_1$-$C_2$-halogenoalkyl, $C_1$-$C_2$-halogenoalkoxy, nitro or cyano,
or together represent $C_2$-$C_5$-alkanediyl which is optionally substituted once to three times in an identical or different manner by fluorine, chlorine, $C_1$-$C_4$-alkyl, $C_1$-$C_4$-alkoxy or $C_1$-$C_2$-halogenoalkyl and

$R^{10}$ and $R^{11}$ independently of one another represent $C_1$-$C_8$-alkyl, $C_2$-$C_8$-alkenyl, $C_1$-$C_8$-alkoxy, $C_1$-$C_8$-alkylamino, $C_3$-$C_8$-alkenylamino, di-($C_1$-$C_8$-alkyl)-amino or di-($C_3$-$C_8$-alkenyl)-amino, in each case optionally substituted once to five times in an identical or different manner by fluorine or chlorine.

**5.** Compounds of the formula (I) according to Claim 1,
in which

A and Q together represent $C_1$-$C_4$-alkanediyl or $C_2$-$C_4$-alkenediyl, which is in each case optionally substituted once or twice in an identical or different manner by fluorine, chlorine, hydroxyl or mercapto, or by $C_1$-$C_6$-alkyl, $C_1$-$C_2$-alkoxy, $C_1$-$C_2$-alkylthio, $C_5$-$C_6$-cycloalkyl or phenyl, in each case optionally substituted once to three times in an identical or different manner by fluorine or chlorine, and which furthermore optionally contains one of the following groupings:

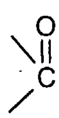 ; 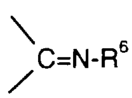 ; 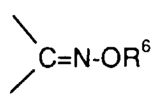 ; 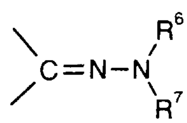 ;

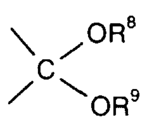 ; 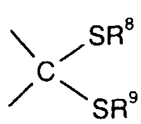 ; 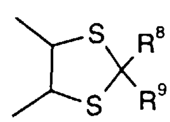 ;

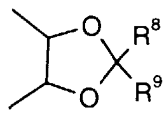 ; 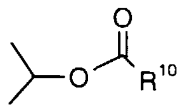 or 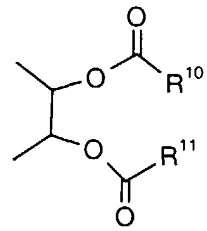

or is bridged by a $C_1$-$C_2$-alkanediyl group,

B and B' independently of one another represent hydrogen, fluorine, chlorine, methyl or ethyl, or together represent $C_1$-$C_4$-alkanediyl or $C_4$-alkenediyl, in each case optionally substituted by methyl or ethyl,

G represents hydrogen (a) or represents one of the groups

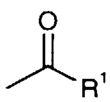 (b), 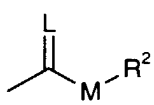 (c), 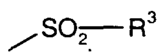 (d), 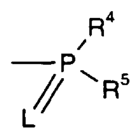 (e),

E (f)

or

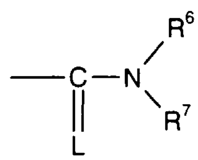 (g),

in which

E represents a metal ion equivalent or an ammonium ion,

L represents oxygen or sulphur and

M represents oxygen or sulphur,

$R^1$ represents $C_1$-$C_{14}$-alkyl, $C_2$-$C_6$-alkenyl, $C_1$-$C_4$-alkoxy-$C_1$-$C_6$-alkyl, $C_1$-$C_4$-alkylthio-$C_1$-$C_6$-alkyl, or poly-$C_1$-$C_4$-alkoxy-$C_1$-$C_4$-alkyl, in each case optionally substituted once to five times in an identical or different manner by fluorine or chlorine, or represents cycloalkyl which has 3 to 6 ring atoms and is optionally substituted once or twice in an identical or different manner by fluorine, chlorine, methyl, ethyl, methoxy or ethoxy, and in which one or two methylene groups can be replaced by oxygen and/or sulphur atoms,
or represents phenyl which is optionally substituted once or twice in an identical or different manner by fluorine, chlorine, bromine, methyl, ethyl, propyl, i-propyl, methoxy, ethoxy, trifluoromethyl, trifluoromethoxy, cyano or nitro,
or represents benzyl which is optionally substituted once or twice in an identical or different manner by fluorine, chlorine, bromine, methyl, ethyl, propyl, i-propyl, methoxy, ethoxy, trifluoromethyl or trifluoromethoxy,
or represents thienyl, furanyl or pyridyl, in each case optionally substituted once or twice in an identical or different manner by fluorine, chlorine, bromine, methyl or ethyl,
or represents phenoxy-$C_1$-$C_4$-alkyl which is optionally substituted once or twice in an identical or different manner by fluorine, chlorine, methyl or ethyl,

or represents pyridyloxy-$C_1$-$C_4$-alkyl, pyrimidyloxy-$C_1$-$C_4$-alkyl or thiazolyloxy-$C_1$-$C_4$-alkyl, in each case optionally substituted once or twice in an identical or different manner by fluorine, chlorine, amino, methyl or ethyl,

$R^2$ represents $C_1$-$C_{14}$-alkyl, $C_2$-$C_6$-alkenyl, $C_1$-$C_4$-alkoxy-$C_2$-$C_6$-alkyl, or poly-$C_1$-$C_4$-alkoxy-$C_2$-$C_6$-alkyl, in each case optionally substituted once to five times in an identical or different manner by fluorine or chlorine,
or represents $C_3$-$C_6$-cycloalkyl which is optionally substituted once to three times in an identical or different manner by fluorine, chlorine, methyl, ethyl, methoxy or ethoxy,
or represents phenyl or benzyl, in each case optionally substituted once or twice in an identical or different manner by fluorine, chlorine, nitro, cyano, methyl, ethyl, propyl, i-propyl, methoxy, ethoxy, trifluoromethoxy or trifluoromethyl,

$R^3$ represents $C_1$-$C_6$-alkyl which is optionally substituted once to three times in an identical or different manner by fluorine or chlorine, or represents phenyl or benzyl, in each case optionally substituted once or twice by fluorine, chlorine, bromine, methyl, ethyl, methoxy, ethoxy, trifluoromethyl, trifluoromethoxy, cyano or nitro,

$R^4$ and $R^5$independently of one another represent $C_1$-$C_4$-alkyl, $C_1$-$C_4$-alkoxy, $C_1$-$C_4$-alkylamino, di-($C_1$-$C_4$-alkyl)-amino, $C_1$-$C_4$-alkylthio, in each case optionally substituted once to three times in an identical or different manner by fluorine or chlorine, or represent phenyl, phenoxy or phenylthio, in each case optionally substituted once or twice in an identical or different manner by fluorine, chlorine, bromine, nitro, cyano, $C_1$-$C_2$-alkoxy, trifluoromethoxy, $C_1$-$C_2$-alkylthio, trifluoromethyl or $C_1$-$C_3$-alkyl,

$R^6$ represents hydrogen or $C_1$-$C_6$-alkyl or $C_1$-$C_6$-alkoxy-$C_2$-$C_4$-alkyl, in each case optionally substituted once to three times in an identical or different manner by fluorine or chlorine, or represents $C_3$-$C_6$-cycloalkyl which is optionally substituted once or twice in an identical or different manner by fluorine, chlorine, methyl, methoxy, trifluoromethyl or trifluoromethoxy, or represents phenyl which is optionally substituted once or twice in an identical or different manner by fluorine, chlorine, trifluoromethyl, $C_1$-$C_4$-alkyl, trifluoromethoxy or $C_1$-$C_4$-alkoxy, or represents benzyl which is optionally substituted once or twice in an identical or different manner by fluorine, chlorine, $C_1$-$C_4$-alkyl, trifluoromethyl, trifluoromethoxy or $C_1$-$C_4$-alkoxy,

$R^7$ represents hydrogen or $C_1$-$C_6$-alkyl or $C_3$-$C_6$-alkenyl, in each case optionally substituted once to three times in an identical or different manner by fluorine or chlorine, or

$R^6$ and $R^7$,together with the N atom to which they are bonded, a 5- to 7-membered ring which optionally contains oxygen or sulphur,

$R^8$ and $R^9$independently of one another represent hydrogen or $C_1$-$C_4$-alkyl which is optionally substituted once to three times in an identical or different manner by fluorine or chlorine, or represent phenyl which is optionally substituted once or twice in an identical or different manner by fluorine, chlorine, methyl, ethyl, methoxy, ethoxy, trifluoromethyl, trifluoromethoxy, nitro or cyano,
or together represent $C_2$-$C_5$-alkanediyl which is optionally substituted once or twice in an identical or different manner by fluorine, chlorine, methyl, ethyl, methoxy, ethoxy or trifluoromethyl and

$R^{10}$ and $R^{11}$independently of one another represent $C_1$-$C_6$-alkyl or $C_2$-$C_6$-alkenyl.

**6.** Process for the preparation of compounds of the formula (I) according to Claim 1, characterized in that to prepare

(A) compounds of the formula (Ia)

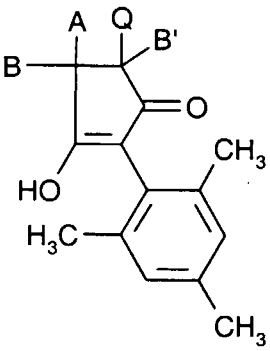

(Ia)

in which
A, B, B' and Q have the meaning given in Claim 1,
compounds of the formula (II)

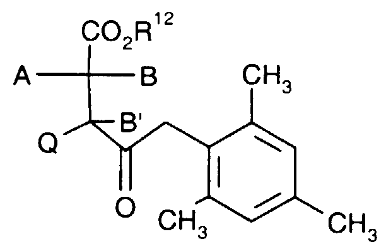

(II)

in which
A, B, B' and Q have the abovementioned meaning and
$R^{12}$ represents alkyl,
are subjected to an intramolecular condensation reaction in the presence of a diluent and in the presence of a base;
or
(B) of the formula (Ib)

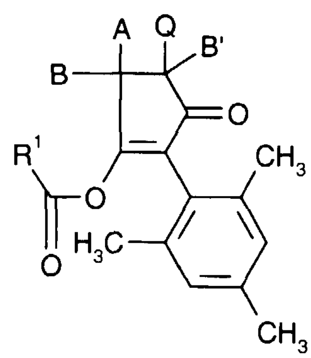

(Ib)

in which
A, B, B', Q and $R^1$ have the meaning given in Claim 1,
compounds of the formula (Ia)

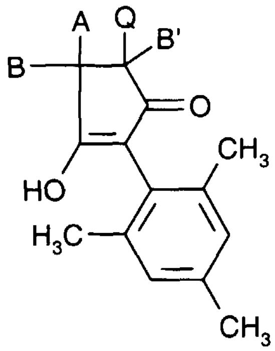

(Ia)

in which
A, B, B' and Q have the abovementioned meaning,

α) are reacted with acid halides of the formula (III)

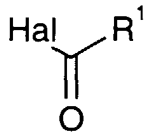

(III)

in which

$R^1$ has the abovementioned meaning and
Hal represents halogen,

if appropriate in the presence of a diluent and if appropriate in the presence of an acid-binding agent, or
β) are reacted with carboxylic acid anhydrides of the formula (IV)

$$R^1\text{-CO-O-CO-}R^1 \quad (IV)$$

in which
$R^1$ has the abovementioned meaning,
if appropriate in the presence of a diluent and if appropriate in the presence of an acid-binding agent;

or
(C) of the formula (Ic-1)

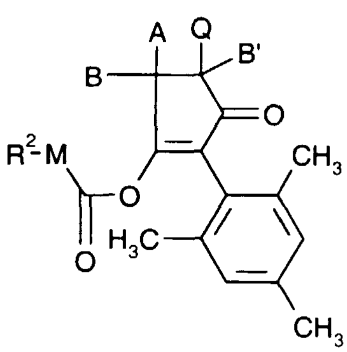

(Ic-1)

in which
A, B, B', Q and $R^2$ have the meaning given in Claim 1,
and
M represents oxygen or sulphur,
compounds of the formula (Ia)

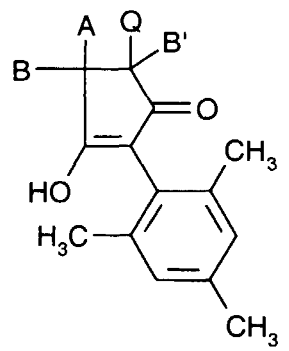

(Ia)

in which
A, B, B' and Q have the abovementioned meaning,
are reacted with chloroformic acid esters or chloroformic acid thiolesters of the formula (V)

$$R^2\text{-M-CO-Cl} \qquad (V)$$

in which
$R^2$ and M have the abovementioned meaning,
if appropriate in the presence of a diluent and if appropriate in the presence of an acid-binding agent;
or
(D) of the formula (Ic-2)

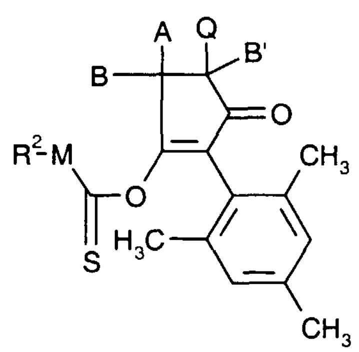

(Ic-2)

in which
A, B, B', Q and $R^2$ have the abovementioned meaning
and
M represents oxygen or sulphur,
compounds of the formula (Ia)

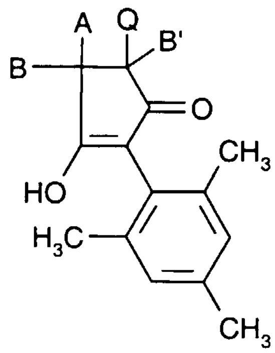

(Ia)

in which A, B, B' and Q have the abovementioned meaning,

α) are reacted with chloromonothioformic acid esters or chlorodithioformic acid esters of the formula (VI)

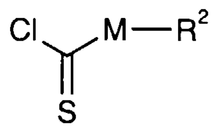

(VI)

in which
M and $R^2$ have the abovementioned meaning,
if appropriate in the presence of a diluent and if appropriate in the presence of an acid-binding agent,
or
β) are reacted with carbon disulphide and then with alkyl halides of the general formula (VII)

$$R^2\text{-Hal} \quad \text{(VII)}$$

in which
$R^2$ has the abovementioned meaning
and
Hal represents chlorine, bromine or iodine,
if appropriate in the presence of a diluent and if appropriate in the presence of a base;

or
(E) of the formula (Id)

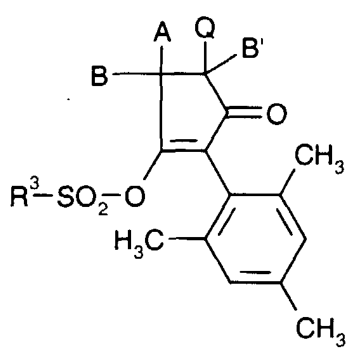

(Id)

in which
A, B, B', Q and $R^3$ have the meaning given in Claim 1,
compounds of the formula (Ia)

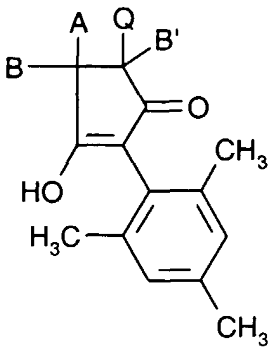

(Ia)

in which
A, B, B' and Q have the abovementioned meaning,
are reacted with sulphonic acid chlorides of the formula (VIII)

$$R^3\text{-}SO_2\text{-}Cl \qquad (VIII)$$

in which
$R^3$ has the abovementioned meaning,
if appropriate in the presence of a diluent and if appropriate in the presence of an acid-binding agent;
or
(F) of the formula (Ie)

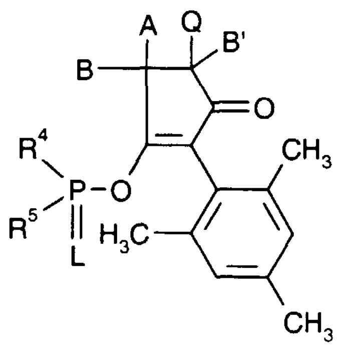

(Ie)

in which
A, B, L, B', Q, $R^4$ and $R^5$ have the meaning given in Claim 1,
compounds of the formula (Ia) or enols thereof

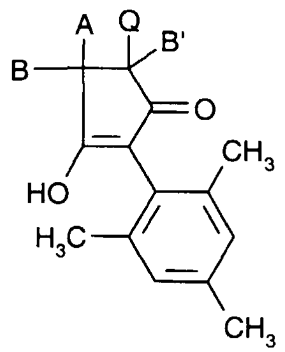

(Ia)

in which
A, B, B' and Q have the abovementioned meaning,
are reacted with phosphorus compounds of the formula (IX)

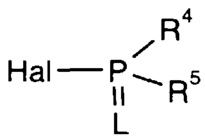

(IX)

in which
L, $R^4$ and $R^5$ have the abovementioned meaning
and
Hal represents halogen
if appropriate in the presence of a diluent and if appropriate in the presence of an acid-binding agent;
or
(G) of the formula (If)

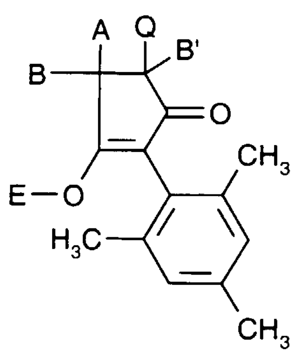

(If)

in which
A, B, B' and Q have the abovementioned meaning
and
E represents a metal ion equivalent, or represents an ammonium ion,
compounds of the formula (Ia)

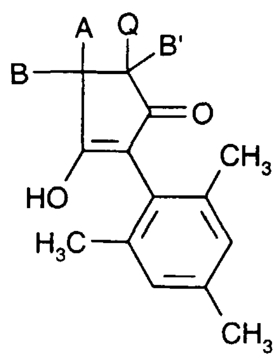

(Ia)

in which
A, B, B' and Q have the abovementioned meaning,
are reacted with metal compounds or amines of the formulae (X) and (XI)

$$Me\ R_t^{16} \qquad (X)$$

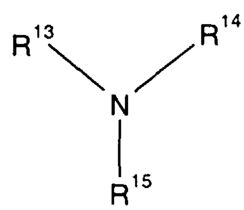

(XI)

in which

| | |
|---|---|
| Me | represents mono- or divalent metal ions, |
| t | represents the number 1 or 2, |
| $R^{13}$, $R^{14}$ and $R^{15}$, | independently of one another, represent hydrogen or alkyl and |
| $R^{16}$ | represents hydrogen, hydroxyl or $C_1$-$C_4$-alkoxy, |

if appropriate in the presence of a diluent,
or
(H) of the formula (Ig)

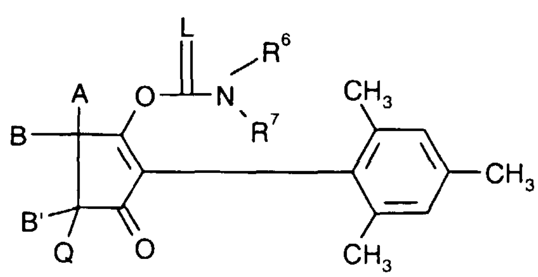

(Ig)

in which
A, B, L, B', Q, $R^6$ and $R^7$ have the meaning given in Claim 1,
compounds of the formula (Ia)

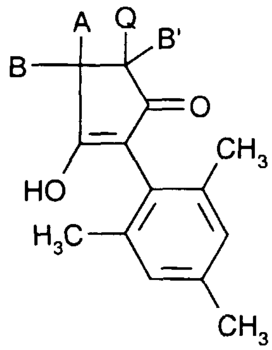

(Ia)

in which
A, B, B' and Q have the abovementioned meaning,
are reacted

α) with compounds of the formula (XII)

$$R^6\text{-N=C=L} \qquad (XII)$$

in which
L and $R^6$ have the abovementioned meaning,
if appropriate in the presence of a diluent and if appropriate in the presence of a catalyst,
or
β) with carbamic acid chlorides or thiocarbamic acid chlorides of the formula (XIII)

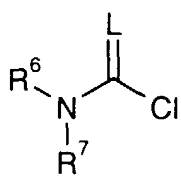

(XIII)

in which
L, $R^6$ and $R^7$ have the abovementioned meaning,
if appropriate in the presence of a diluent and if appropriate in the presence of an acid-binding agent.

**7.** Compounds of the formula (II)

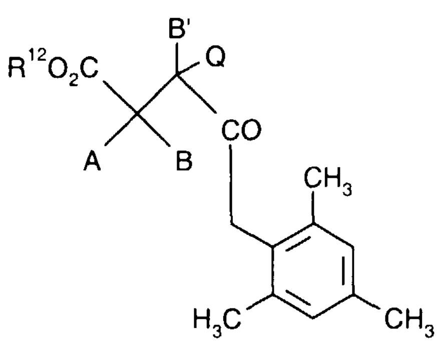

(II)

in which

A, B, B' and Q have the meaning given in Claim 1 and
$R^{12}$ represents alkyl.

**8.** Process for the preparation of the compounds of the formula (II) according to Claim 7, characterized in that compounds of the formula (XIV)

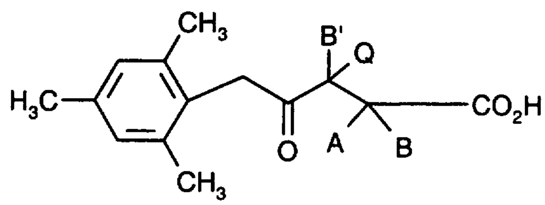

(XIV)

in which
A, B, B' and Q have the meaning given in Claim 1,
are esterified.

**9.** Compounds of the formula (XIV)

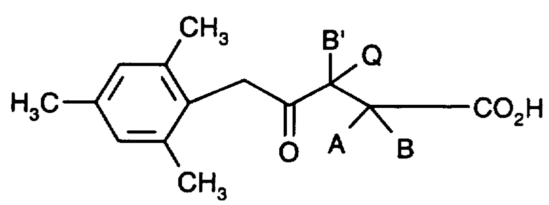

(XIV)

in which
A, B, B' and Q have the meaning given in Claim 1.

**10.** Process for the preparation of compounds of the formula (XIV), characterized in that carboxylic acid anhydrides of the formula (XV)

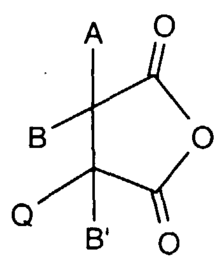

(XV)

in which
A, B, B' and Q have the meaning given in Claim 1,
are reacted with organometallic compounds of the formula (XVI)

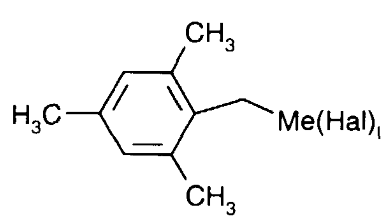

(XVI)

in which
Me represents mono- or divalent metal ions,
Hal represents chlorine or bromine
and
I represents a number 0 or 1,
in the presence of a diluent,
or in that compounds of the formula (XVII)

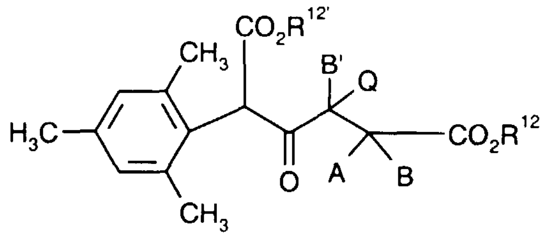

(XVII)

in which
A, B, B' and Q have the abovementioned meaning and
$R^{12}$ and $R^{12'}$ represent alkyl,
are decarboxylated, if appropriate in the presence of a diluent and if appropriate in the presence of a base or acid.

**11.** Compounds of the formula (XVII)

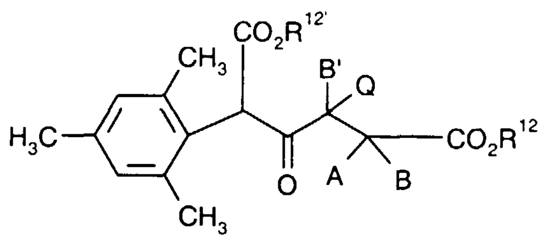

(XVII)

in which
A, B, B', Q, $R^{12}$ and $R^{12'}$ have the meaning given in Claim 10.

**12.** Process for the preparation of compounds of the formula (XVII) according to Claim 11, characterized in that dicarboxylic acid half-ester chlorides of the formula (XVIII)

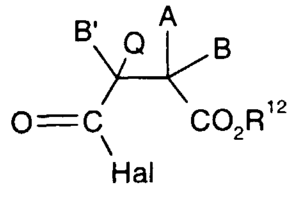

(XVIII)

in which
A, B, B', Q and $R^{12}$ have the abovementioned meaning and
Hal represents chlorine or bromine,
are acylated with a substituted phenylacetic acid ester of the formula (XIX)

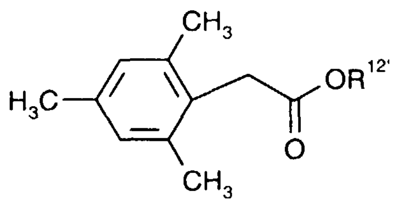

(XIX)

in which
$R^{12'}$ represents alkyl,
in the presence of a diluent and in the presence of a base.

**13.** Compositions for controlling pests, and herbicides, characterized in that they contain at least one compound of the formula (I) according to Claim 1.

**14.** Use of compounds of the formula (I) according to Claim 1 for controlling animal pests and undesirable plant growth.

**15.** Method of controlling animal pests and undesirable plant growth, characterized in that compounds of the formula (I) according to Claim 1 are applied to the pests, plants and/or their environment.

**16.** Process for the preparation of compositions for controlling pests, and herbicides, characterized in that compounds of the formula (I) according to Claim 1 are mixed with extenders and/or surface-active substances.

**17.** Use of compounds of the formula (I) according to Claim 1 for the preparation of compositions for controlling pests, and herbicides.

**Revendications**

**1.** Composés de formule (I)

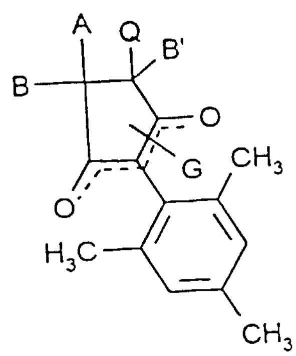

(I)

dans laquelle

A et Q représentent ensemble un groupe alcanediyle ou alcènediyle qui est éventuellement substitué dans chaque cas par un radical halogéno, hydroxy, mercapto, un radical alkyle, alkoxy, alkylthio, cycloalkyle, benzyloxy ou aryle éventuellement substitué dans chaque cas, et qui porte en outre le cas échéant l'un des groupes suivants

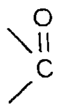 ; 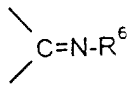 ; 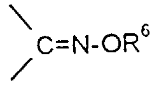 ; 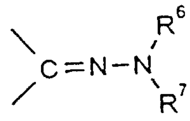 ;

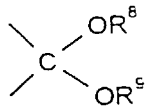 ; 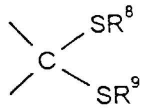 ; 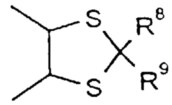 ;

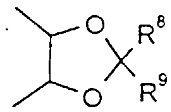 ; 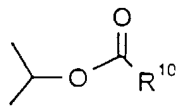 ou 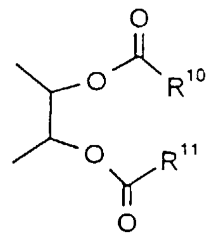

ou qui est ponté par un groupe alcanediyle,

B et B' représentent indépendamment l'un de l'autre hydrogène, un halogène ou un groupe alkyle ou forment ensemble un groupe alcanediyle ou alcènediyle dont chacun est éventuellement substitué,

G représente l'hydrogène (a) ou l'un des groupes

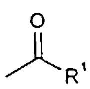 (b), 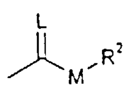 (c), 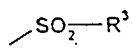 (d), 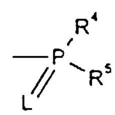 (e),

E (f)

ou

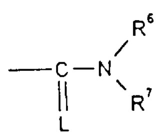 (g),

dans lesquels

E est un équivalent d'ion métallique ou un ion ammonium,

L est l'oxygène ou le soufre et

M est l'oxygène ou le soufre,

$R^1$ représente un groupe alkyle, alcényle, alkoxyalkyle, alkylthioalkyle, polyalkoxyalkyle ou cycloalkyle dont chacun est éventuellement substitué et qui peut contenir au moins un hétéroatome, un groupe phényle, phénylalkyle, hétaryle, phénoxalkyle ou hétaryloxyalkyle dont chacun est éventuellement substitué,

$R^2$ est un groupe alkyle, cycloalkyle, alcényle, alkoxyalkyle, polyalkoxyalkyle éventuellement substitué dans chaque cas ou un groupe phényle ou benzyle dont chacun est

éventuellement substitué,

$R^3$ est un groupe alkyle, phényle ou phénylalkyle éventuellement substitué dans chaque cas,

$R^4$ et $R^5$ représentent indépendamment l'un de l'autre un groupe alkyle, alkoxy, alkylamino, dialkylamino, alkylthio, alcénylthio, cycloalkylthio dont chacun est éventuellement substitué par un halogène ou un groupe phényle, phénoxy, ou phénylthio dont chacun est éventuellement substitué,

$R^6$ est l'hydrogène, un groupe alkyle, alcényle, alkoxyalkyle éventuellement substitué dans chaque cas par un halogène, un groupe cycloalkyle ou phényle éventuellement substitué dans chaque cas ou un groupe benzyle éventuellement substitué,

$R^7$ représente l'hydrogène, un groupe alkyle ou alcényle éventuellement substitué dans chaque cas par un halogène, ou bien

$R^6$ et $R^7$ forment conjointement avec l'atome d'azote auquel ils sont liés un noyau contenant éventuellement de l'oxygène ou du soufre,

$R^8$ et $R^9$ représentent indépendamment l'un de l'autre l'hydrogène ou un groupe alkyle, phényle ou phénylalkyle dont chacun est éventuellement substitué ou forment conjointement un reste alcanediyle éventuellement substitué et

$R^{10}$ et $R^{11}$ représentent indépendamment l'un de l'autre un groupe alkyle, alcényle, alkoxy, alkylamino, dialkylamino, alcénylamino ou dialcénylamino dont chacun est éventuellement substitué par un halogène, ou un groupe phényle ou benzyle dont chacun est éventuellement substitué.

2. Composés de formule (I) suivant la revendication 1, qui possède, en incorporant les différentes définitions (a), (b), (c), (d), (e), (f) et (g) du groupe G, les structures (Ia) à (Ig) suivantes :

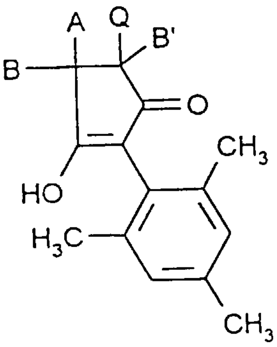

(Ia)

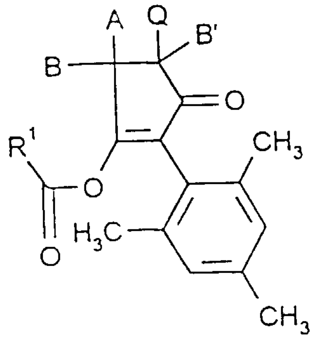

(Ib)

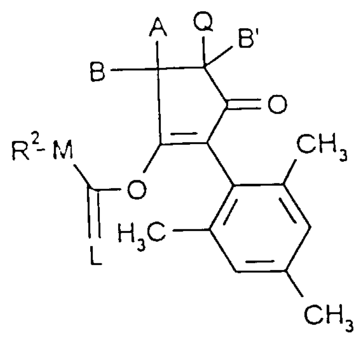

(Ic)

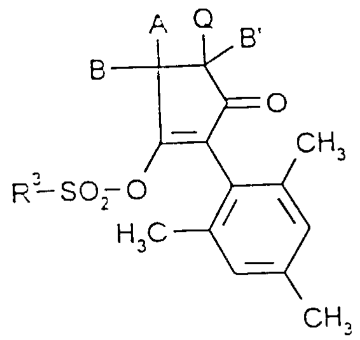

(Id)

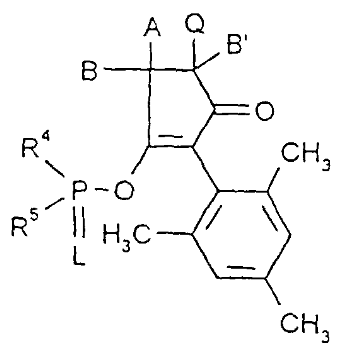

(Ie)

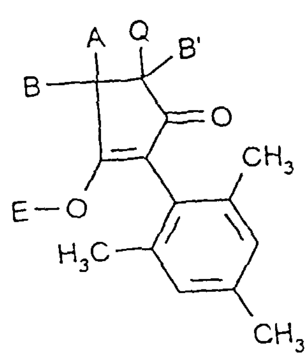

(If)

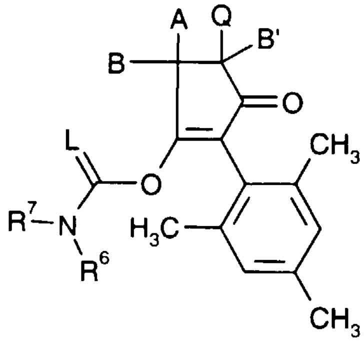

(Ig)

dans lesquelles

A, B, B', E, L, M, Q, $R^1$, $R^2$, $R^3$, $R^4$, $R^5$, $R^6$ et $R^7$ possèdent les définitions indiquées ci-dessus.

**3.** Composés de formule (I) suivant la revendication 1, dans laquelle

A et Q forment conjointement un groupe alcanediyle en $C_1$ à $C_6$ ou alcènediyle en $C_2$ à $C_6$ dont chacun est éventuellement substitué une à trois fois identiques ou différentes par un radical halogéno, hydroxy, mercapto, un radical alkyle en $C_1$ à $C_{10}$, alkoxy en $C_1$ à $C_6$, alkylthio en $C_1$ à $C_6$, cycloalkyle en $C_3$ à $C_7$ portant chacun le cas échéant 1 à 9 substituants halogéno identiques ou différents, ou un radical benzyloxy ou phényle portant chacun le cas échéant 1 à 5 substituants, identiques ou différents, halogéno, alkyle en $C_1$ à $C_6$ ou alkoxy en $C_1$ à $C_6$, qui contient en outre le cas échéant l'un des groupements suivants :

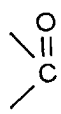 ; 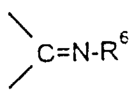 ; 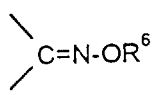 ; 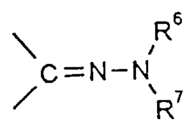 ;

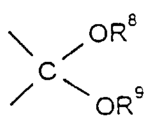 ; 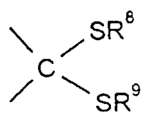 ; 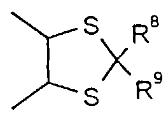 ;

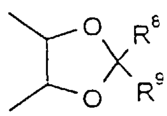 ; 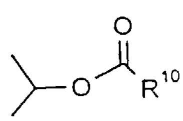 oder 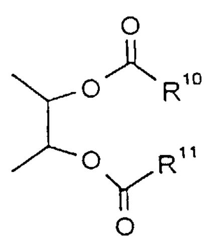

ou qui est ponté par un groupe alcanediyle en $C_1$ à $C_2$,

B et B' représentent indépendamment l'un de l'autre l'hydrogène, un halogène ou un groupe alkyle en $C_1$ à $C_6$ ou forment ensemble un groupe alcanediyle en $C_1$ à $C_6$ ou alcènediyle en $C_4$ dont chacun est substitué le cas échéant par un radical alkyle en $C_1$ à $C_6$,

G représente l'hydrogène (a) ou l'un des groupes

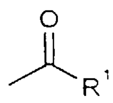 (b), 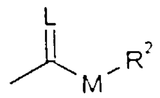 (c), 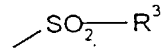 (d), 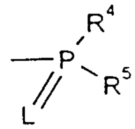 (e),

E (f)

ou

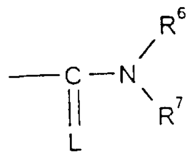 (g),

dans lesquels

E est un équivalent d'ion métallique ou un ion ammonium,
L est l'oxygène ou le soufre,
M est l'oxygène ou le soufre,

$R^1$ est un groupe alkyle en $C_1$ à $C_{20}$, alcényle en $C_2$ à $C_{10}$, (alkoxy en $C_1$ à $C_8$)-(alkyle en $C_1$ à $C_8$), (alkylthio en $C_1$ à $C_8$)-(alkyle en $C_1$ à $C_8$), poly-(alkoxy en $C_1$ à $C_8$)-(alkyle en $C_1$ à $C_8$) portant le cas échéant un ou plusieurs substituants halogéno identiques ou différents ou un groupe cycloalkyle à noyau de 3 à 8 chaînons portant le cas échéant un ou plusieurs substituants halogéno, alkyle en $C_1$ à $C_6$ ou alkoxy en $C_1$ à $C_6$ identiques ou différents, dans lequel au moins un groupe méthylène peut être remplacé par un atome d'oxygène et/ou un atome de soufre,
un groupe phényle portant éventuellement un ou plusieurs substituants, identiques ou différents, halogéno, nitro, cyano, alkyle en $C_1$ à $C_6$, alkoxy en $C_1$ à $C_6$, halogénalkyle en $C_1$ à $C_6$ ou halogénalkoxy en $C_1$ à $C_6$,
un groupe phényle-(alkyle en $C_1$ à $C_6$) portant le cas échéant un ou plusieurs substituants, identiques ou différents, halogéno, alkyle en $C_1$ à $C_6$, alkoxy en $C_1$ à $C_6$, halogénalkyle en $C_1$ à $C_6$ ou halogénalkoxy en $C_1$ à $C_6$,
un groupe hétaryle à noyau de 5 ou 6 atomes portant le cas échéant un ou plusieurs substituants halogéno, ou alkyle en $C_1$ à $C_6$, identiques ou différents,
un groupe phénoxy (alkyle en $C_1$ $C_6$) portant le cas échéant un ou plusieurs substituants, identiques ou différents, halogéno ou alkyle en $C_1$ à $C_6$, ou bien
un groupe hétaryloxy-(alkyle en $C_1$ à $C_6$) avec un noyau de 5 ou 6 atomes éventuellement substitué une ou plusieurs fois identiques ou différentes par un radical halogéno, amino ou alkyle en $C_1$ à $C_6$,

$R^2$ est un groupe alkyle en $C_1$ à $C_{20}$, alcényle en $C_2$ à $C_{10}$, (alkoxy en $C_1$ à $C_8$)-(alkyle en $C_2$ à $C_8$), poly-(alkoxy en $C_1$ à $C_8$)-(alkyle en $C_2$ à $C_8$) portant chacun le cas échéant un ou plusieurs substituants halogéno identiques ou différents,
un groupe cycloalkyle en $C_3$ à $C_6$ portant le cas échéant un ou plusieurs substituants halogéno, alkyle en $C_1$ à $C_6$, alkoxy en $C_1$ à $C_6$ identiques ou différents, ou bien
un groupe phényle ou benzyle portant chacun le cas échéant un ou plusieurs substituants, identiques ou différents, halogéno, nitro, cyano, alkyle en $C_1$ à $C_6$, alkoxy en $C_1$ à $C_6$, halogénalkoxy en $C_1$ à $C_3$ ou halogénalkyle en $C_1$ à $C_3$,

$R^3$ est un groupe alkyle en $C_1$ à $C_{12}$ portant le cas échéant un ou plusieurs substituants halogéno identiques ou différents, ou un groupe phényle ou phényl-(alkyle en $C_1$ à $C_4$) portant le cas échéant un ou plusieurs substituants, identiques ou différents, halogéno, alkyle en $C_1$ à $C_6$, alkoxy en $C_1$ à $C_6$, halogénalkyle en $C_1$ à $C_3$, halogénalkoxy en $C_1$ à $C_3$, cyano ou nitro,

$R^4$ et $R^5$représentent indépendamment l'un de l'autre un groupe alkyle en $C_1$ à $C_8$, alkoxy en $C_1$ à $C_8$, alkylamino en $C_1$ à $C_8$, di-(alkyle en $C_1$ à $C_8$)-amino, alkylthio en $C_1$ à $C_8$, alcénylthio en $C_3$ à $C_5$, cycloalkylthio en $C_3$ à $C_7$ portant chacun le cas échéant un ou plusieurs substituants halogéno identiques ou différents, un groupe phényle, phénoxy ou phénylthio portant chacun le cas échéant un ou plusieurs substituants, identiques ou différents, halogéno, nitro, cyano, alkoxy en $C_1$ à $C_4$, halogénalkoxy en $C_1$ à $C_4$, alkylthio en $C_1$ à $C_4$, halogé-

nalkylthio en $C_1$ à $C_4$, alkyle en $C_1$ à $C_4$ ou halogénalkyle en $C_1$ à $C_4$,

$R^6$ représente l'hydrogène, un groupe alkyle en $C_1$ à $C_{10}$, alcényle en $C_3$ à $C_8$, (alkoxy en $C_1$ à $C_8$)-(alkyle en $C_2$ à $C_8$) portant chacun le cas échéant un ou plusieurs substituants halogéno identiques ou différents, un groupe cycloalkyle en $C_3$ à $C_{10}$ portant le cas échéant un ou plusieurs substituants, identiques ou différents, halogéno, alkyle en $C_1$ à $C_6$, alkoxy en $C_1$ à $C_6$, halogénalkyle en $C_1$ à $C_3$ ou halogénalkoxy en $C_1$ à $C_3$, un groupe phényle éventuellement substitué par un halogène, un radical halogénalkyle en $C_1$ à $C_3$, alkyle en $C_1$ à $C_8$, halogénalkoxy en $C_1$ à $C_3$ ou alkoxy en $C_1$ à $C_8$ ou un groupe benzyle éventuellement substitué le cas échéant une ou plusieurs fois identiques ou différentes par un radical halogéno, alkyle en $C_1$ à $C_8$, halogénalkyle en $C_1$ à $C_3$, halogénalkoxy en $C_1$ à $C_3$, ou alkoxy en $C_1$ à $C_8$,

$R^7$ représente l'hydrogène, un groupe alkyle en $C_1$ à $C_{10}$ ou alcényle à $C_3$ à $C_{10}$ portant le cas échéant un ou plusieurs substituants halogéno identiques ou différents, ou bien

$R^6$ et $R^7$forment conjointement avec l'atome d'azote auquel ils sont liés un noyau de 3 à 7 chaînons contenant éventuellement de l'oxygène ou du soufre,

$R^8$ et $R^9$représentent indépendamment l'un de l'autre l'hydrogène, un groupe alkyle en $C_1$ à $C_6$ portant le cas échéant un ou plusieurs substituants halogéno identiques ou différents, ou un groupe phényle ou phényl-(alkyle en $C_1$ à $C_4$) portant le cas échéant un ou plusieurs substituants, identiques ou différents, halogéno, alkyle en $C_1$ à $C_6$, alkoxy en $C_1$ à $C_6$, halogénalkyle en $C_1$ à $C_6$, halogénalkoxy en $C_1$ à $C_6$, nitro ou cyano,
ou forment ensemble un groupe alcanediyle en $C_2$ à $C_6$ portant le cas échéant un ou plusieurs substituants, identiques ou différents, halogéno, alkyle en $C_1$ à $C_6$, alkoxy en $C_1$ à $C_6$ ou halogénalkyle en $C_1$ à $C_3$ et

$R^{10}$ et $R^{11}$représentent indépendamment l'un de l'autre un groupe alkyle en $C_1$ à $C_{10}$, alcényle en $C_2$ à $C_{10}$, alkoxy en $C_1$ à $C_{10}$, alkylamino en $C_1$ à $C_{10}$ ou di-(alkyle en $C_1$ à $C_{10}$)-amino portant chacun le cas échéant un ou plusieurs substituants halogéno identiques ou différents, un groupe alcénylamino en $C_3$ à $C_{10}$, di-(alcényle en $C_3$ à $C_{10}$)-amino ou un groupe phényle ou benzyle dont chacun est substitué une ou plusieurs fois identiques ou différentes par un radical halogéno, alkyle en $C_1$ à $C_4$, alkoxy en $C_1$ à $C_4$, halogénalkyle en $C_1$ à $C_4$, nitro ou cyano.

**4.** Composés de formule (I) suivant la revendication 1, dans laquelle

A et Q forment ensemble un groupe alcanediyle en $C_1$ à $C_5$ ou alcènediyle en $C_2$ à $C_5$ dont chacun est éventuellement substitué une à trois fois identiques ou différentes par un radical fluoro, chloro, bromo, hydroxy, mercapto, un radical alkyle en $C_1$ à $C_8$, alkoxy en $C_1$ à $C_4$, alkylthio en $C_1$ à $C_4$, cycloalkyle en $C_5$ à $C_7$ ou phényle portant chacun le cas échéant 1 à 5 substituants fluoro ou chloro identiques ou différents, qui contient en outre le cas échéant l'un des groupements suivants

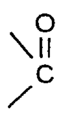 ; 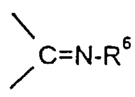 ; 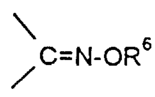 ; 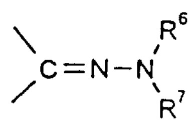 ;

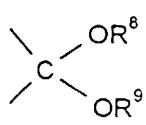 ; 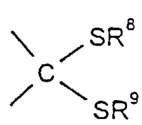 ; 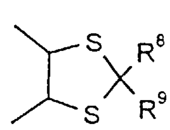 ;

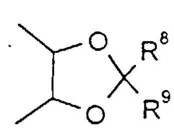 ; 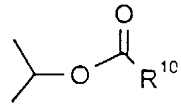 ou 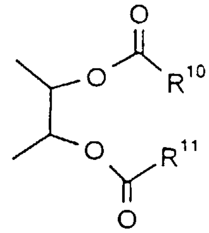

ou qui est ponté par un groupe alcanediyle en $C_1$ ou $C_2$,

B et B' représentent chacun indépendamment l'un de l'autre l'hydrogène, le fluor, le chlore ou un groupe alkyle en $C_1$ à $C_4$ ou forment ensemble un groupe alcanediyle en $C_1$ à $C_5$ ou alcènediyle en $C_4$ éventuellement substitué par un radical alkyle en $C_1$ à $C_4$,

G représente l'hydrogène (a) ou l'un des groupes

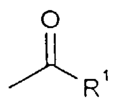 (b), 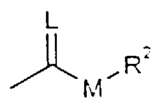 (c), 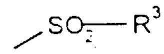 (d), 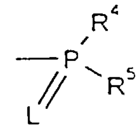 (e),

E (f)

ou

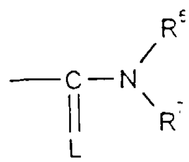 (g),

dans lesquels

E est un équivalent d'ion métallique ou un ion ammonium,
L est l'oxygène ou le soufre,
M est l'oxygène ou le soufre,

$R^1$ est un groupe alkyle en $C_1$ à $C_{16}$, alcényle en $C_2$ à $C_8$, (alkoxy en $C_1$ à $C_6$)-(alkyle en $C_1$ à $C_6$), (alkylthio en $C_1$ à $C_6$)-(alkyle en $C_1$ à $C_6$), poly-(alkoxy en $C_1$ à $C_6$)-(alkyle en $C_1$ à $C_6$) portant chacun le cas échéant 1 à 9 substituants fluoro ou chloro identiques ou différents, ou un groupe cycloalkyle à noyau de 3 à 7 atomes portant le cas échéant 1 à 3 substituants, identiques ou différentes, fluoro, chloro, alkyle en $C_1$ à $C_4$ ou alkoxy en $C_1$ à $C_4$, dans lequel un ou deux groupes méthylène peuvent être remplacés par des atomes d'oxygène et/ou des atomes de soufre,
un groupe phényle portant éventuellement un à trois substituants, identiques ou différents, fluoro, chloro, bromo, cyano, nitro, alkyle en $C_1$ à $C_4$, alkoxy en $C_1$ à $C_4$, halogénalkyle en $C_1$ à $C_3$ ou halogénalkoxy en $C_1$ à $C_3$,
un groupe phényl-(alkyle en $C_1$ à $C_4$) portant le cas échéant un à trois substituants, identiques ou différents, fluoro, chloro, bromo, alkyle en $C_1$ à $C_4$, alkoxy en $C_1$ à $C_4$, halogénalkyle en $C_1$ à $C_3$ ou halogénalkoxy en $C_1$ à $C_3$,
un groupe furannyle, thiényle, pyridyle, pyrimidyle, thiazolyle ou pyrazolyle portant chacun le cas échéant un ou deux substituants, identiques ou différents, fluoro, chloro, bromo ou alkyle en $C_1$ à $C_4$,
un groupe phénoxy-(alkyle en $C_1$ à $C_5$) portant éventuellement un à trois substituants fluoro, chloro, ou alkyle en $C_1$ à $C_4$ identiques ou différents, ou bien
un groupe pyridyloxy- (alkyle en $C_1$ à $C_6$), pyrimidinyloxy-(alkyle en $C_1$ à $C_6$) ou thiazolyloxy-(alkyle en $C_1$ à $C_6$) portant chacun le cas échéant un ou deux substituants, identiques ou différents, fluoro, chloro, amino ou alkyle en $C_1$ à $C_4$,

$R^2$ est un groupe alkyle en $C_1$ à $C_{16}$, alcényle en $C_2$ à $C_8$, (alkoxy en $C_1$ à $C_6$)-(alkyle en $C_2$ à $C_6$), poly-(alkoxy en $C_1$ à $C_6$)-(alkyle en $C_2$ à $C_6$) portant chacun le cas échéant 1 à 9 substituants, identiques ou différents, fluoro ou chloro,
un groupe cycloalkyle en $C_3$ à $C_6$ portant le cas échéant un à cinq substituants identiques ou différents, fluoro, chloro, alkyle en $C_1$ à $C_4$ ou alkoxy en $C_1$ à $C_4$, ou bien
un groupe phényle ou benzyle portant chacun le cas échéant un à trois substituants, identiques ou différents, fluoro, chloro, bromo, nitro, cyano, alkyle en $C_1$ à $C_4$, alkoxy en $C_1$ à $C_4$, halogénalkoxy en $C_1$ ou $C_2$ ou halogénalkyle en $C_1$ à $C_3$,

$R^3$ est un groupe alkyle en $C_1$ à $C_9$ portant le cas échéant 1 à 5 substituants fluoro ou chloro identiques ou

différents, ou représentent un groupe phényle ou phényl-(alkyle en $C_1$ ou $C_2$) portant chacun le cas échéant un à trois substituants, identiques ou différents, fluoro, chloro, bromo, alkyle en $C_1$ à $C_4$, alkoxy en $C_1$ à $C_4$, halogénalkyle en $C_1$ ou $C_2$, halogénalkoxy en $C_1$ ou $C_2$, cyano ou nitro,

$R^4$ et $R^5$représentent indépendamment l'un de l'autre un groupe alkyle en $C_1$ à $C_6$, alkoxy en $C_1$ à $C_6$, alkylamino en $C_1$ à $C_6$, di(alkyle en $C_1$ à $C_6$)-amino, alkylthio en $C_1$ à $C_6$, alcénylthio en $C_3$ ou $C_4$, cycloalkylthio en $C_3$ à $C_6$ portant éventuellement 1 à 5 substituants fluoro ou chloro identiques ou différents, ou un groupe phényle, phénoxy ou phénylthio portant chacun le cas échéant 1 à 3 substituants, identiques ou différents, fluoro, chloro, bromo, nitro, cyano, alkoxy en $C_1$ à $C_3$, halogénalkoxy en $C_1$ à $C_3$, alkythio en $C_1$ à $C_3$, halogénalkylthio en $C_1$ à $C_3$, alkyle en $C_1$ à $C_3$ ou halogénalkyle en $C_1$ à $C_3$,

$R^6$ représente l'hydrogène, un groupe alkyle en $C_1$ à $C_8$, alcényle en $C_3$ à $C_6$, (alkoxy en $C_1$ à $C_6$)-(alkyle en $C_2$ à $C_6$) portant chacun le cas échéant 1 à 5 substituants, identiques ou différents, fluoro ou chloro, un groupe cycloalkyle en $C_3$ à $C_8$ portant le cas échéant 1 à 3 substituants, identiques ou différents, fluoro, chloro, alkyle en $C_1$ à $C_4$, alkoxy en $C_1$ à $C_4$, halogénalkyle en $C_1$ ou $C_2$ ou halogénalkoxy en $C_1$ ou $C_2$, un groupe phényle portant éventuellement un à trois substituants, identiques ou différents, fluoro, chloro, bromo, halogénalkyle en $C_1$ ou $C_2$, alkyle en $C_1$ à $C_5$, halogénalkoxy en $C_1$ ou $C_2$ ou alkoxy en $C_1$ à $C_5$, ou un groupe benzyle portant éventuellement un à trois substituants, identiques ou différents, fluoro, chloro, bromo, alkyle en $C_1$ à $C_5$, halogénalkyle en $C_1$ à $C_2$, halogénalkoxy en $C_1$ ou $C_2$ ou alkoxy en $C_1$ à $C_5$,

$R^7$ représente l'hydrogène, un groupe alkyle en $C_1$ à $C_8$ ou alcényle en $C_3$ à $C_8$ portant chacun le cas échéant 1 à 5 substituants fluoro ou chloro identiques ou différents, ou bien

$R^6$ ou $R^7$forment conjointement avec l'atome d'azote auquel ils sont liés un noyau de 4 à 7 chaînons contenant éventuellement de l'oxygène ou du soufre,

$R^8$ et $R^9$représentent indépendamment l'un de l'autre l'hydrogène, un groupe alkyle en $C_1$ à $C_4$ éventuellement substitué 1 à 5 fois identiques ou différentes par du fluor ou du chlore, ou un groupe phényle portant éventuellement un à trois substituants, identiques ou différents, fluoro, chloro, alkyle en $C_1$ à $C_4$, alkoxy en $C_1$ à $C_4$, halogénalkyle en $C_1$ ou $C_2$, halogénalkoxy en $C_1$ ou $C_2$, nitro ou cyano,
ou forment ensemble un groupe alcanediyle en $C_2$ à $C_5$ portant le cas échéant un à trois substituants, identiques ou différents, fluoro, chloro, alkyle en $C_1$ à $C_4$, alkoxy en $C_1$ à $C_4$, ou halogénalkyle en $C_1$ ou $C_2$, et

$R^{10}$ et $R^{11}$forment indépendamment l'un de l'autre un groupe alkyle en $C_1$ à $C_8$, alcényle en $C_2$ à $C_8$, alkoxy en $C_1$ à $C_8$, alkylamino en $C_1$ à $C_8$, alcénylamino en $C_3$ à $C_8$, di(alkyle en $C_1$ à $C_8$)-amino ou di-(alcényle en $C_3$ à $C_8$)-amino portant chacun le cas échéant 1 à 5 substituants fluoro ou chloro identiques ou différents.

**5.** Composés de formule (I) suivant la revendication 1,
dans laquelle

A et Q forment ensemble un groupe alcanediyle en $C_1$ à $C_4$ ou alcènediyle en $C_2$ à $C_4$ substitué chacun le cas échéant une ou deux fois identiques ou différentes par du fluor, du chlore, un radical hydroxy, mercapto, un radical alkyle en $C_1$ à $C_6$, alkoxy en $C_1$ ou $C_2$, alkylthio en $C_1$ ou $C_2$, cycloalkyle en $C_5$ ou $C_6$ ou phényle portant chacun le cas échéant un à trois substituants fluoro ou chloro identiques ou différents, qui contient en outre le cas échéant l'un des groupes suivants :

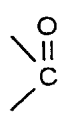 ; 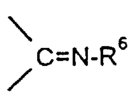 ; 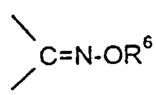 ; 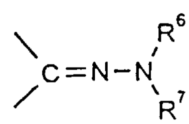 ;

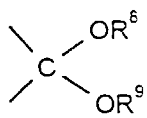 ; 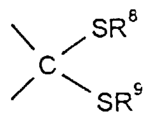 ; 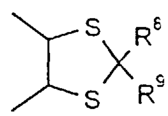 ;

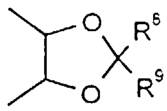 ; 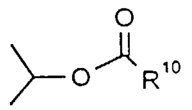 ou 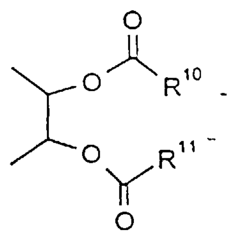

ou qui est ponté par un groupe alcanediyle en $C_1$ ou $C_2$,

B et B' représentent indépendamment l'un de l'autre l'hydrogène, le fluor, le chlore, un groupe méthyle, éthyle, ou forment conjointement un groupe alcanediyle en $C_1$ à $C_4$ ou alcènediyle en $C_4$ dont chacun est éventuellement substitué par un radical méthyle ou éthyle,

G représente l'hydrogène (a) ou l'un des groupes

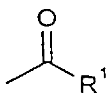 (b), 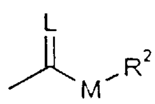 (c), 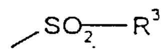 (d), 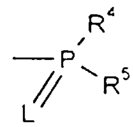 (e),

E (f)

ou

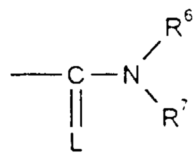 (g),

dans lesquels

E est un équivalent d'ion métallique ou un ion ammonium,
L est l'oxygène ou le soufre,
M est l'oxygène ou le soufre,

$R^1$ représente un groupe alkyle en $C_1$ à $C_{14}$, alcényle en $C_2$ à $C_6$, (alkoxy en $C_1$ à $C_4$)-(alkyle en $C_1$ à $C_6$), (alkylthio en $C_1$ à $C_4$)-(alkyle en $C_1$ à $C_6$), poly-(alkoxy en $C_1$ à $C_4$)-(alkyle en $C_1$ à $C_4$) portant chacun le cas échéant un à cinq substituants fluoro ou chloro identiques ou différents, ou un groupe cycloalkyle à noyau de 3 à 6 atomes portant éventuellement un ou deux-substituants, identiques ou différents, fluoro, chloro, méthyle, éthyle, méthoxy ou éthoxy, dans lequel un ou deux groupes méthylène peuvent être remplacés par des atomes d'oxygène et/ou des atomes de soufre,
un groupe phényle substitué le cas échéant une ou deux fois identiques ou différentes par du fluor, du chlore, du brome, un radical méthyle, éthyle, propyle, isopropyle, méthoxy, éthoxy, trifluorométhyle, trifluorométhoxy, cyano ou nitro,
un groupe benzyle portant éventuellement un ou deux substituants, identiques ou différents, fluoro, chloro, bromo, méthyle, éthyle, propyle, isopropyle, méthoxy, éthoxy, trifluorométhyle ou trifluorométhoxy,
un groupe thiényle, furannyle ou pyridyle portant chacun le cas échéant un ou deux substituants identiques ou différents, fluoro, chloro, bromo, méthyle ou éthyle,
un groupe phénoxy-(alkyle en $C_1$ à $C_4$) éventuellement substitué une ou deux fois identiques ou différentes par du fluor, du chlore, un radical méthyle ou éthyle, ou bien
un groupe pyridyloxy- (alkyle en $C_1$ à $C_4$), pyrimidyloxy-(alkyle en $C_1$ à $C_4$) ou thiazolyloxy-(alkyle en $C_1$ à $C_4$) portant chacun le cas échéant un ou deux substituants, identiques ou différents, fluoro, chloro, amino, alkyle ou éthyle,

$R^2$ est un groupe alkyle en $C_1$ à $C_{14}$, alcényle en $C_2$ à $C_6$, (alkoxy en $C_1$ à $C_4$)-(alkyle en $C_2$ à $C_6$), poly-(alkoxy en $C_1$ à $C_4$)-(alkyle en $C_2$ à $C_6$) portant chacun le cas échéant 1 à 5 substituants fluoro ou chloro identiques ou différents ou un groupe cycloalkyle en $C_3$ à $C_6$ portant éventuellement 1 à 3 substituants, identiques ou différents, fluoro, chloro, méthyle, éthyle, méthoxy ou éthoxy,
ou un groupe phényle ou benzyle portant chacun le cas échéant 1 à 2 substituants, identiques ou différents, fluoro, chloro, nitro, cyano, méthyle, éthyle, propyle, isopropyle, méthoxy, éthoxy, trifluorométhoxy ou trifluorométhyle,

$R^3$ est un groupe alkyle en $C_1$ à $C_6$ éventuellement substitué une à trois fois identiques ou différentes par du fluor ou du chlore ou un groupe phényle ou benzyle portant chacun le cas échéant un ou deux substituants fluoro, chloro, bromo, méthyle, éthyle, méthoxy, éthoxy, trifluorométhyle, trifluorométhoxy, cyano ou nitro,

$R^4$ et $R^5$représentent indépendamment l'un de l'autre un groupe alkyle en $C_1$ à $C_4$, alkoxy en $C_1$ à $C_4$, alkylamino en $C_1$ à $C_4$, di-(alkyle en $C_1$ à $C_4$)-amino, alkylthio en $C_1$ à $C_4$ portant chacun le cas échéant un à trois substituants fluoro ou chloro identiques ou différents, ou un groupe phényle, phénoxy ou phénylthio portant chacun le cas échéant un ou deux substituants, identiques ou différentes, fluoro, chloro, bromo, nitro, cyano, alkoxy en $C_1$ à $C_2$, trifluorométhoxy, alkylthio en $C_1$ ou $C_2$, trifluorométhyle ou alkyle en $C_1$ à $C_3$,

$R^6$ représente l'hydrogène, un groupe alkyle en $C_1$ à $C_6$, (alkoxy en $C_1$ à $C_6$)-(alkyle en $C_2$ à $C_4$) portant chacun le cas échéant un à trois substituants fluoro ou chloro identiques ou différents, un groupe cycloalkyle en $C_3$ à $C_6$ portant le cas échéant un ou deux substituants, identiques ou différents, fluoro, chloro, méthyle, méthoxy, trifluorométhyle ou trifluorométhoxy, un groupe phényle éventuellement substitué une ou deux fois identiques ou différentes par du fluor, du chlore, un radical trifluorométhyle, alkyle en $C_1$ à $C_4$, trifluorométhoxy ou alkoxy en $C_1$ à $C_4$ ou un groupe benzyle portant éventuellement un ou deux substituants, identiques ou différents, fluoro, chloro, alkyle en $C_1$ à $C_4$, trifluorométhyle, trifluorométhoxy ou alkoxy en $C_1$ à $C_4$,

$R^7$ représente l'hydrogène, un groupe alkyle en $C_1$ à $C_6$ ou alcényle en $C_3$ à $C_6$ portant chacun le cas échéant un à trois substituants fluoro ou chloro identiques ou différents, ou bien

$R^6$ et $R^7$forment conjointement avec l'atome d'azote auquel ils sont liés un noyau pentagonal à heptagonal contenant éventuellement de l'oxygène ou du soufre,

$R^6$ et $R^9$représentent indépendamment l'un de l'autre l'hydrogène, un groupe alkyle en $C_1$ à $C_4$ portant éventuellement un à trois substituants fluoro ou chloro identiques ou différents, ou un groupe phényle éventuellement substitué une ou deux fois identiques ou différentes par du fluor, du chlore, un radical méthyle, éthyle, méthoxy, éthoxy, trifluorométhyle, trifluorométhoxy, nitro ou cyano,
ou forment ensemble un groupe alcanediyle en $C_2$ à $C_5$ éventuellement substitué une ou deux fois identiques ou différentes par du fluor, du chlore, un radical méthyle, éthyle, méthoxy, éthoxy, ou trifluorométhyle et

$R^{10}$ et $R^{11}$représentent indépendamment l'un de l'autre un groupe alkyle en $C_1$ à $C_6$ ou alcényle en $C_2$ à $C_6$.

**6.** Procédé de production de composés de formule (I) suivant la revendication 1, caractérisé en ce que pour la préparation

(A) de composés de formule (Ia)

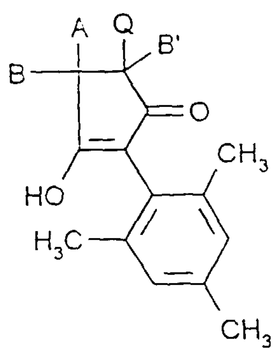

(Ia)

dans laquelle
A, B, B' et Q ont la définition indiquée dans la revendication 1,
on effectue la condensation intramoléculaire de composés de formule (II)

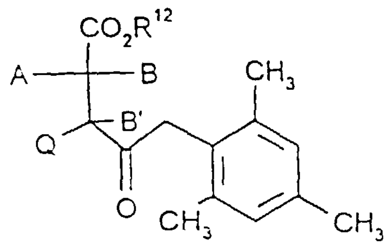

(II)

dans laquelle
A, B, B' et Q ont la définition indiquée ci-dessus, et
$R^{12}$ est un groupe alkyle,
en présence d'un diluant et en présence d'une base ;
ou bien
(B) de composés de formule (Ib)

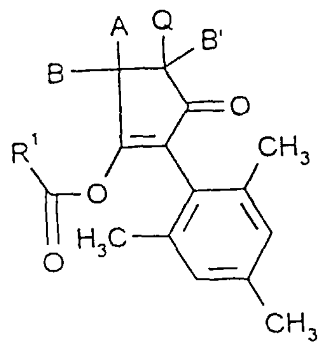

(Ib)

dans laquelle
A, B, B', Q et $R^1$ ont la définition indiquée dans la revendication 1,
on fait réagir les composés de formule (Ia)

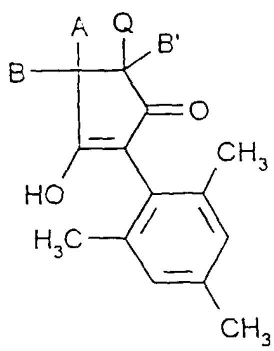

(Ia)

dans laquelle
A, B, B' et Q ont la définition indiquée ci-dessus,

α) avec des halogénures d'acides de formule (III)

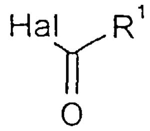

(III)

dans laquelle
$R^1$ a la définition indiquée ci-dessus et
Hal est un halogène,
éventuellement en présence d'un diluant et le cas échéant en présence d'un accepteur d'acide, ou bien
β) avec des anhydrides d'acides carboxyliques de formule générale (IV)

$$R^1\text{-CO-O-CO-}R^1 \quad \text{(IV)}$$

dans laquelle
$R^1$ a la définition indiquée ci-dessus,
le cas échéant en présence d'un diluant et en la présence éventuelle d'un accepteur d'acide ;

ou bien
(C) de formule (Ic-I)

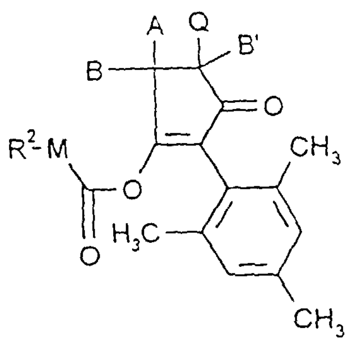

(Ic-1)

dans laquelle
A, B, B', Q et $R^2$ ont la définition indiquée dans la revendication 1
et
M est l'oxygène ou le soufre,
on fait réagir des composés de formule (Ia)

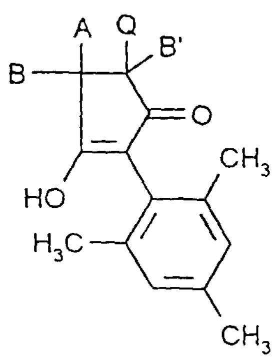

(Ia)

dans laquelle
A, B, B' et Q ont la définition indiquée ci-dessus,

avec un ester d'acide chloroformique ou un thiol-ester d'acide chloroformique de formule (V)

$$R^2\text{-M-CO-Cl} \qquad (V)$$

dans laquelle
$R^2$ et M ont la définition indiquée ci-dessus,
le cas échéant en présence d'un diluant et en la présence éventuelle d'un accepteur d'acide ;
ou bien
(D) de formule (Ic-2)

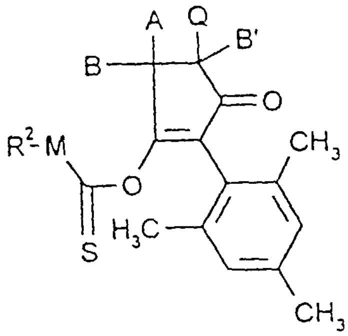

(Ic-2)

dans laquelle
A, B, B', Q et $R^2$ ont la définition indiquée ci-dessus,
et
M est l'oxygène ou le soufre,
on fait réagir des composés de formule (Ia)

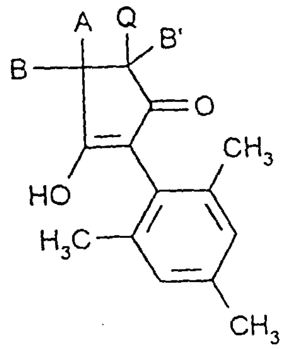

(Ia)

dans laquelle
A, B, B' et Q ont la définition indiquée ci-dessus,

α) avec des esters d'acide chloromonothioformique ou des esters d'acide chlorodithioformique de formule (VI)

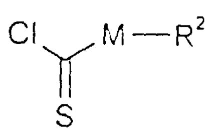

(VI)

dans laquelle
M et $R^2$ ont la définition indiquée ci-dessus, éventuellement en présence d'un diluant et le cas échéant en présence d'un accepteur d'acide,
ou bien
β) avec du chlorure de carbone puis avec des halogénures d'alkyle de formule générale (VII)

$$R^2\text{-Hal} \quad \text{(VII)}$$

dans laquelle
$R^2$ a la définition indiquée ci-dessus et
Hal représente le chlore, le brome ou l'iode, éventuellement en présence d'un diluant et en la présence éventuelle d'une base ;

ou bien
(E) de formule (Id)

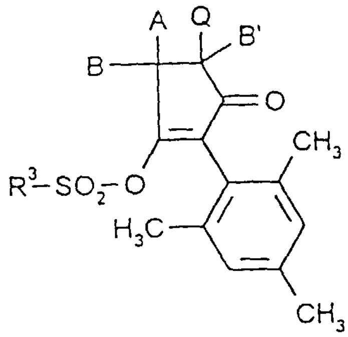

. (Id)

dans laquelle
A, B, B', Q et $R^3$ ont la définition indiquée dans la revendication 1,
on fait réagir des composés de formule (Ia)

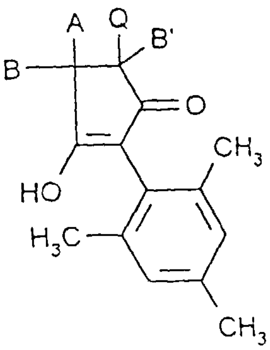

(Ia)

dans laquelle
A, B, B' et Q ont la définition indiquée ci-dessus,
avec des chlorures d'acide sulfonique de formule (VIII)

$$R^3\text{-}SO_2\text{-Cl} \quad \text{(VIII)}$$

dans laquelle
$R^3$ a la définition indiquée ci-dessus,
le cas échéant en présence d'un diluant et en la présence éventuelle d'un accepteur d'acide

ou bien
(F) de formule (Ie)

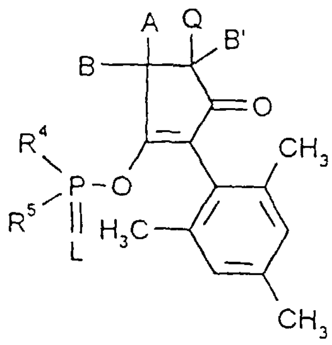

(Ie)

dans laquelle
A, B, L, B', Q, $R^4$ et $R^5$ ont la définition indiquée dans la revendication 1,
on fait réagir des composés de formule (Ia) ou leurs énoles

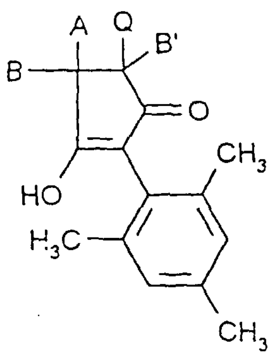

(Ia)

dans laquelle
A, B, B' et Q ont la définition indiquée ci-dessus,
avec des composés de phosphore de formule (IX)

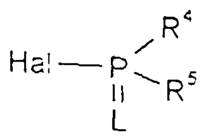

(IX)

dans laquelle
L, $R^4$ et $R^5$ ont la définition indiquée ci-dessus, et
Hal représente un halogène,
éventuellement en présence d'un diluant et en la présence éventuelle d'un accepteur d'acie,
ou bien
(G) de formule (If)

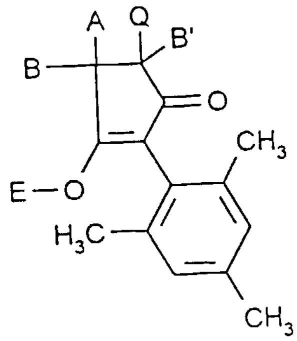

(If)

dans laquelle
A, B, B' et Q ont la définition indiquée ci-dessus,
et
E représente un équivalent d'ion métallique ou un ion ammonium,
on fait réagir des composés de formule (Ia)

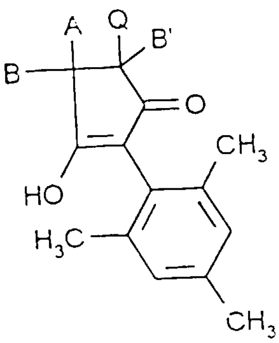

(Ia)

dans laquelle
A, B, B' et Q ont la définition indiquée ci-dessus,
avec des composés métalliques ou des amines de formules (X) et(XI)

$$Me\ R_1^{16} \qquad (X)$$

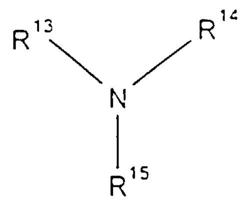

(XI)

dans lesquelles

| | |
|---|---|
| Me | représente des ions de métaux monovolents et divalents, |
| t | représente un nombre 1 ou 2, |
| $R^{13}$, $R^{14}$ et $R^{15}$ | représentent indépendamment les uns des autres l'hydrogène ou un groupe alkyle et |
| $R^{16}$ | est l'hydrogène, un groupe hydroxy ou alkoxy en $C_1$ à $C_4$, |

le cas échéant en présence d'un diluant,

ou bien
de formule (Ig)

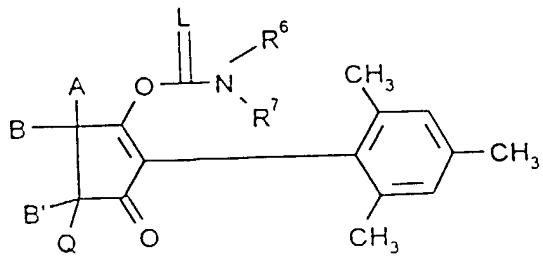

(Ig)

dans laquelle
A, B, L, B', Q, $R^6$ et $R^7$ ont la définition indiquée dans la revendication 1,
on fait réagir des composés de formule (Ia)

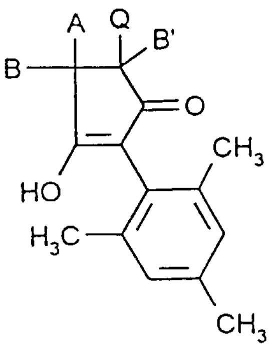

(Ia)

dans laquelle
A, B, B' et Q ont la définition indiquée ci-dessus,

α) avec des composés de formule (XII)

$$R^6\text{-N=C=L} \qquad \text{(XII)}$$

dans laquelle
L et $R^6$ ont la définition indiquée ci-dessus, le cas échéant en présence d'un diluant et en la présence éventuelle d'un catalyseur,
ou bien
β) avec des chlorures d'acide carbamique ou des chlorures d'acide thiocarbamique de formule (XIII)

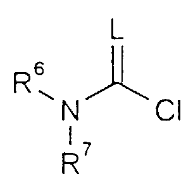

(XIII)

dans laquelle
L, $R^6$ et $R^7$ ont la définition indiquée ci-dessus,
le cas échéant en présence d'un diluant et en la présence éventuelle d'un accepteur d'acide.

**7.** Composés de formule (II)

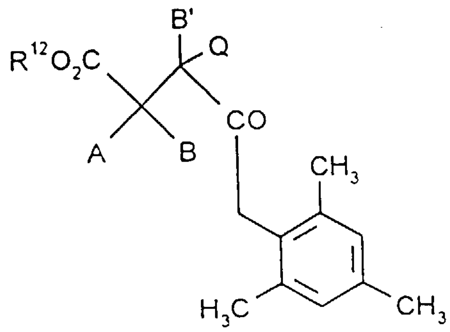

(II)

dans laquelle

| | |
|---|---|
| A, B, B' et Q | ont la définition indiquée dans la revendication 1 et |
| $R^{12}$ | est un groupe alkyle. |

**8.** Procédé de production de composés de formule (II) suivant la revendication 7, caractérisé en ce qu'on estérifie des composés de formule (XIV)

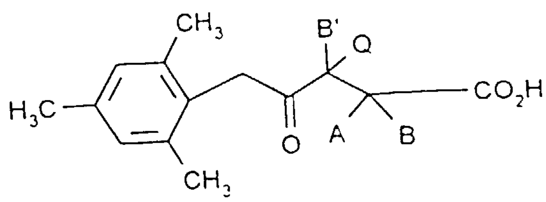

(XIV)

dans laquelle

| | |
|---|---|
| A, B, B' et Q | ont la définition indiquée dans la revendication 1. |

**9.** Composés de formule (XIV)

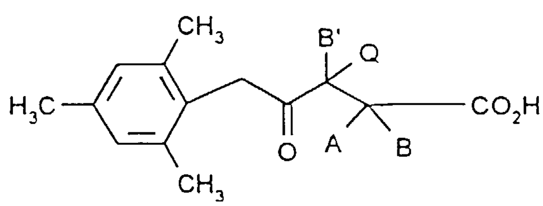

(XIV)

dans laquelle
A, B, B' et ont la définition indiquée dans la revendication

**10.** Procédé de production de composés de formule (XIV), caractérisé en ce qu'on fait réagir des anhydrides d'acides carboxyliques de formule (XV)

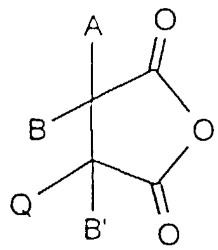

(XV)

dans laquelle
A, B, B' et Q ont la définition indiquée dans la revendication 1, avec des composés organométalliques de formule (XVI)

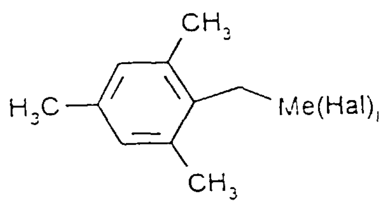

(XVI)

dans laquelle

Me représente des ions de métal monovalent ou divalent,
Hal représente le chlore ou le brome

et

1 est le nombre entier 0 ou 1,

en présence d'un diluant,
ou en ce qu'on décarboxyle des composés de formule (XVII)

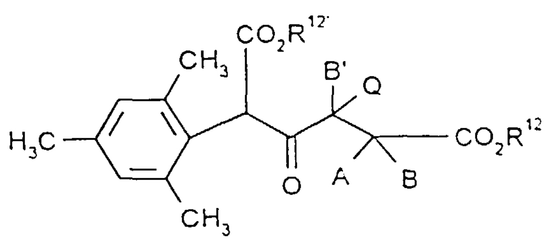

(XVII)

dans laquelle
A, B, B' et Q ont la définition indiquée ci-dessus et $R^{12}$ et $R^{12'}$ représentent un groupe alkyle,
le cas échéant en présence d'un diluant et en la présence éventuelle d'une base ou d'un acide.

**11.** Composés de formule (XVII)

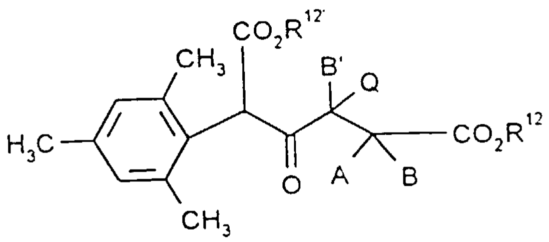

(XVII)

dans laquelle
A, B, B', Q, $R^{12}$ et $R^{12'}$ ont la définition indiquée dans la revendication 10.

**12.** Procédé de production de composés de formule (XVII) suivant la revendication 11, caractérisé en ce qu'on acyle des chlorures de semi-esters d'acides dicarboxyliques de formule (XVIII)

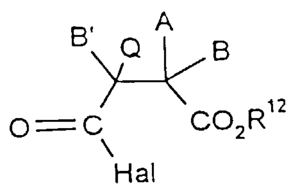

(XVIII)

dans laquelle

| | |
|---|---|
| A, B, B', Q et $R^{12}$ | ont la définition indiquée ci-dessus et |
| Hal | représente le chlore ou le brome, |

avec un ester d'acide phénylacétique substitué de formule (XIX)

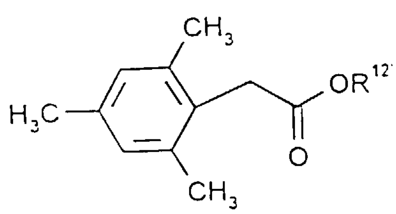

(XIX)

dans laquelle

$R^{12'}$ est un groupe alkyle,

en présence d'un diluant et en présence d'une base.

**13.** Compositions pesticides et herbicides, caractérisés par une teneur en au moins un composé de formule (I) suivant la revendication 1.

**14.** Utilisation de composés de formule (I) suivant la revendication 1 pour combattre des parasites animaux et la croissance de plantes indésirables.

**15.** Procédé pour combattre des parasites animaux et la présence de plantes indésirables, caractérisé en ce qu'on applique des composés de formule (I) suivant la revendication 1 sur les parasites, les plantes et/ou leur milieu.

**16.** Procédé de préparation de compositions pesticides et d'herbicides, caractérisé en ce qu'on mélange des composés de formule (I) suivant la revendication 1 avec des diluants et/ou des agents tensio-actifs.

**17.** Utilisation de composés de formule (I) suivant la revendication 1 pour la préparation de compositions pesticides et d'herbicides.